(19) Europäisches Patentamt
European Patent Office
Office européen des brevets

(11) EP 1 939 777 A2

(12) # EUROPEAN PATENT APPLICATION

(43) Date of publication:
**02.07.2008 Bulletin 2008/27**

(51) Int Cl.:
***G06F 19/00*** *(2006.01)*

(21) Application number: **07118387.5**

(22) Date of filing: **12.10.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **13.10.2006 US 581226**
**26.04.2007 US 914125 P**

(71) Applicant: **Soar Through Life, Ltd.**
**Nevada Incline Village NV 89451 (US)**

(72) Inventors:
- **Neville, Thomas, B.**
  **Incline Village, NV 89451 (US)**
- **Kaminski, David**
  **Cambridge, MA 02138 (US)**

(74) Representative: **Schmit Chretien Schihin & Mahler**
**Baaderstraße 3**
**80469 München (DE)**

(54) **Methods and systems of dynamic screening of disease**

(57) The present invention provides methods and systems for dynamic screening of disease in a subject. The information provided by the methods and systems can be used for many purposes including disease diagnosis and screening, disease treatment, and education the subject of life choices.

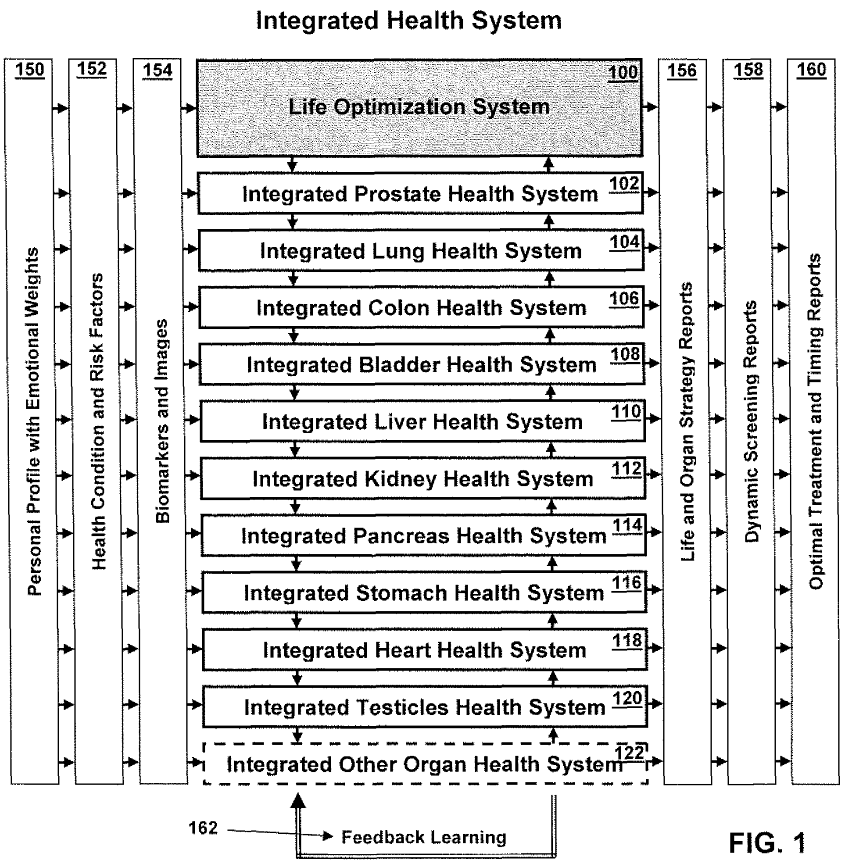


FIG. 1



Printed by Jouve, 75001 PARIS (FR)

## Description

## Background

In the field of medicine there is increasing emphasis on: health, disease prevention and early detection and treatment; avoiding unnecessary treatment; choosing the optimal timing of the best treatment based on medical evidence; and avoiding invasive and costly procedures like biopsies. The use of screening blood tests is also becoming more prevalent and cost effective. One blood draw reduces costs of screening for many conditions. New techniques reduce the cost of specific tests. The incremental cost of additional tests decreases once blood is drawn for another test. Blood can be stored for later testing if needed for specific conditions in order to reduce costs.

Another approach is periodic whole body imaging. Periodic whole body imaging is becoming more prevalent as part of a screening program and/or triggered by warning signs for one condition. Total costs are declining from $1,000, where the average cost per organ is less than $100. The Incremental cost for each additional organ is very low once imaging of other organs is initiated. Organ volume measurements and other image processing is becoming increasingly automated and is dropping in cost.

Significant investments are being made to accelerate discovery and use of biomarkers that effectively detect progressing cancer. However, many of the new biomarkers are not adequately effective based on the results of one test.

## Summary of the Invention

In an aspect of the invention, a method of assessing a disease in a subject is disclosed comprising: obtaining data from a subject corresponding to a biomarker for a disease at least two different times, wherein the data corresponding to the at least two different times form a first trend; removing data from said first trend that has a value outside a tolerance, thereby forming a second trend; and determining a relationship between said second trend and a historical trend, wherein the historical trend corresponds to the biomarker for the disease.

In another aspect of the invention, a method of assessing a disease in a subject is disclosed comprising: obtaining data from a subject corresponding to a biomarker for a disease at least two different times, wherein the data corresponding to the at least two different times form a first trend; removing data from the first trend that has a value outside a tolerance, thereby forming a second trend; and estimating future values for the biomarker using the second trend.

In an embodiment of the methods of the invention, the velocity of the second trend is used to determine the relationship between said second trend and said historical trend.

The relationship of a trend to a historical trend can determined by a probability calculation. In an embodiment, the probability calculation is a Bayesian method. In an embodiment, the data obtained from a subject comprises a ratio of two biomarkers for the disease. In another embodiment, the disease being assessed is prostate cancer and the at least one biomarker is selected from the group consisting of fPSA and PSA.

When removing data from a trend, the tolerance can be determined by a feedback iteration process.

The invention also discloses a system for assessing a disease in a subject comprising a Monte Carlo calculation engine, wherein the engine executes a method of invention.

In an aspect of the invention, a method to assess the probability of progressing disease in a subject is disclosed comprising: obtaining data from a subject corresponding to a biomarker for a disease at least two different times, wherein the data corresponding to the at least two different times form a trend; assessing a prior probability of progressing disease at more than one time point; and determining the probability of said trend relating to the prior probability of progressing disease, thereby assessing the probability of progressing disease.

In another aspect of the invention, a method for screening for progressing cancer is disclosed that comprises: estimating two trends over at least two time points corresponding to data from two biomarkers and the ratio between the two biomarkers; determining prior probabilities of progressing cancer at different times; and estimating the probability of progressing cancer by performing a Bayesian method with said trend and said prior probabilities, thereby screening for progressing cancer.

In yet another aspect of the invention, a method of assessing disease progression is disclosed that comprises: determining prior probabilities of cancer occurrence at more than one time; obtaining data from a subject corresponding to a biomarker for a disease at least two different times, wherein the data corresponding to the at least two different times form a trend; determining a probability of an observation of the trend conditional on no progressing disease; determining a probability of an observation of the trend conditional on progressing disease; and assessing disease progression by performing a Bayesian method with the prior probabilities, the probability of an observation conditional on no progressing disease, and the probability of an observation conditional on progressing disease. The probability of an observation of the trend conditional on no progressing disease or conditional on progressing disease can be calculated using Monte Carlo methods.

In an embodiment, the progressing disease is progressing prostate cancer and a biomarker is selected from the group consisting of fPSA and PSA.

In another embodiment, a method of screening or assessing progressing disease further comprises removing data that has a value outside a tolerance from the trend. In some instances, the method comprises removing data that has a value outside a tolerance from two trends.

The present invention provides a method of delivering medical treatment to a subject with a disease comprising: obtaining information provided from executing any method of assessing disease or any method of screening or assessing progressing disease of the invention; determining a course of medical action based on said information; and delivering medical treatment by following the course of medical action. The course of medical action can comprise administration of medical tests. The course of medical action can also comprise a specific time for delivering medical treatment. In another embodiment, the course of medical action comprises calculation of a Life Score.

The present invention also provides a method of diagnosing a disease in a subject comprising: obtaining information provided from executing any method of assessing disease or any method of screening or assessing progressing disease of the invention; and diagnosing the disease in the subject using said information.

In an aspect of the invention, a computer readable medium comprises computer readable instructions, wherein the computer readable instructions when executed cause a processor to execute a method of the invention. The instructions can operate in a software runtime environment.

In another aspect, the invention provides a data signal that is transmitted using a network, wherein the data signal comprises information provided from a method of the invention. The data signal can comprise packetized data that is transmitted through a carrier wave across the network.

In yet another aspect of the invention, a medical information system for assessing a disease of a subject is provided that comprises: an input device for receiving subject data corresponding to a biomarker for the disease at least two different times, wherein the data corresponding to the at least two different times form a first trend; a processor that assesses a probability of said trend relating to historical data; a storage unit in communication with the processor having a database for: (i) storing the subject data; (ii) storing historical data related to the disease; and an output device that transmits information relating to the probability of said trend relating to historical data to an end user.

In an aspect of the invention, a method is provided for assessing a disease in a subject comprising: collecting data from the subject corresponding to a biomarker for the disease at least two different times, wherein the data corresponding to the at least two different times form a trend; exporting said data for manipulation of said data by executing a method of the invention; and importing the results of said manipulation to an end user.

## BRIEF DESCRIPTION OF THE DRAWINGS

FIG. 1 is a block diagram depicting an integrated health system.
FIG. 2 is a block diagram depicting an integrated organ health system.
FIG. 3 is a graph depicting confined and penetrating progression.
FIG. 4 is a graph depicting a cure ratio graph.
FIG. 5 is flowchart depicting a cure ratio calculation method.
FIG. 6 depicts processing of Pretreatment PSA, Gleason and stage information.
FIG. 7 is a table depicting example cancer score values.
FIG. 8 depicts example cancer score tables.
FIG. 9 depicts an example for calculating an average cancer score.
FIG. 10 is a flowchart depicting a strategy system flowchart.
FIG. 11 depicts an example for estimating life scores for treatment timing.
FIGS. 12 and 13 are graphs for treatment strategies.
FIG. 14 is a flowchart depicting a treatment timing system flowchart.
FIG. 15 is a flowchart for estimating life score for treatment timing.
FIG. 16 is a life score graph.
FIG. 17 is a life score impact graph.
FIG. 18 depicts an example of a dynamic screening system.
FIG. 19 is a flowchart for a dynamic screening system.
FIG. 20 is a flowchart for trends processing.
FIG. 21 is a flowchart for calculating velocity distributions.
FIG. 22 depicts estimation for multiple volume measurements.
FIG. 23 depicts estimation for one volume measurements.
FIG. 24 depicts estimation for no volume measurement.
FIGS. 25-28 are flowcharts for prediction generation.

FIG. 29 is a flowchart depicting a method for mapping residual results.
FIG. 30 is a flowchart depicting a method for predicting PSA and free PSA.
FIG. 31 depicts hypothesis generation for progressing cancer.
FIG. 32 depicts prior probabilities processing.
FIG. 33 depicts long-term probabilities processing.
FIG. 34 is a flowchart for calculating probability of progressing cancer.
FIGS. 35 and 36 are flowcharts for estimating the probability of progressing cancer.
FIG. 37 illustrates a flow chart for utilizing personal probability distributions of progressing cancer.
FIGS. 38-40 depict flow charts demonstrating methods of calculating personal probability distributions using Monte Carlo methods.
FIGS. 41-43 show a Monte Carlo process for generating outcomes from a number of probability distributions.
FIG. 44 shows 100 possible buckets of possible results when two dimensions are divided into ten segments.
FIG. 45 depicts 10,000 possible buckets of possible results when three dimensions are divided into ten segments.
FIG. 46 depicts the number of Monte Carlo iterations required to create a reasonably stable distribution.
FIG. 47 depicts a bucket of concern defined by a range of PSA and PSAV results around observed trend results.
FIG. 48 illustrates a small cube inside a large cube depicting a hypercube bucket of concern defined by a range biomarker results
FIG. 49 depicts methods for reducing the number of calculations by focusing on a bucket of concern are disclosed below.
FIG. 50 shows a four dimensional frequency generator for a no cancer case.
FIG. 51 shows a Monte Carlo process for generating no cancer PSA outcomes from a number of probability distributions.
FIG. 52 demonstrates total calculation time can be reduced by constraining the range of values used to calculate PSA to the combinations of values that are likely to result in trend PSA values that are within range of a target value.
FIGS. 53-55 show a Monte Carlo process for generating outcomes from a number of probability distributions.
FIG. 56 shows a four dimensional frequency generator for each year of cancer plus a no cancer case.
FIGS. 57-60 depict flow charts showing a Monte Carlo process for generating outcomes from a number of probability distributions.
FIG. 61 depicts a dynamic screening custom content system.
FIGS. 62-70 depict feedback processing scenarios.
FIGS. 71 and 72 are block diagrams depicting examples of integrated health systems.

## DETAILED DESCRIPTION

### I. Integrated Health System

Integrated Health Systems, shown on **FIG. 1**, combine at least two subsystems that may include the Life Optimization System (**100**) and one or more Integrated Organ Health Systems (**102** through **122**). Inputs may include: a person's personal *Profile* with emotional weights (**150**), health conditions and risk factors (**152**), and the results of biomarker tests and analysis of images of the body and some of its organs (**154**). Outputs may include: life and organ strategy reports (**156**), Dynamic Screening reports (**158**), and optimal treatment and timing reports (**160**). Feedback learning from actual results improves the effectiveness of the systems (**162**).

### Integrated Organ Health Systems

Integrated Organ Health Systems, shown on **FIG. 2**, comprise analysis and report systems for Organ Strategy (**204** and **205**), Dynamic Screening (**208** and **210**), Treatment Timing and Treatment Type (**200** and **202**),. They may connect with the Life Optimization System (**200** and **202**), for which a patent application has been submitted. The Organ Strategy and Treatment Timing Systems build on the Treatment Type Systems (**216** and **218**), for which a patent application has been submitted. All systems will improve over time using feedback learning (**220**) from the experience of users of the systems.

### Prostates and Prostate Cancer

Integrated Organ Health Systems apply to a wide range of human organs, as suggested by the Integrated Health System chart above. The rest of the application will focus on the male prostate and prostate cancer as a concrete example that does not limit the generality of the invention.

## II. Progressing Cancer and the Cure Ratio

The Cure Ratio is an element of the Organ Strategy System and the Treatment Timing System when applied to cancer. It focuses on curable internal progressing cancer because it is the only prostate cancer that needs to be treated and can be cured.

## Four Prostate Cancer States

We consider four prostate cancer states: No Cancer, Dormant Cancer, Internal Progressing Cancer, and External Progressing Cancer. We define these states based on the probability they will occur because we are chiefly concerned with risk analysis rather than the complex biology of prostate cancer. We provide example illustrations to help you visualize the biology behind the probabilities, but caution you against reading too much into them. The risks you face, not the actual biological mechanisms, are what matter most for our analysis and your decisions.

No Cancer means absolutely no prostate cancer exists in your prostate or the surrounding areas. Cancer is neither inside your prostate nor outside it in this state.

Dormant Cancer is any prostate cancer that is not clearly progressing toward metastasis. The first Dormant Cancer cells start with a genetic mutation. These cells will remain dormant unless additional mutations are triggered. As long as cancer remains dormant, it poses no health or survival threat. Dormant Cancer can exist solely inside your prostate or outside as well as inside your prostate.

Internal Progressing Cancer is confined to the prostate. Additional mutations of Dormant Cancer cells can trigger progression. Progressing Cancer grows exponentially and will eventually lead to death unless treated. Treatment of the prostate removes or kills the growing prostate cancer and provides a full cure as long as any cancer outside the prostate remains dormant.

External Progressing Cancer is Progressing Cancer that exists outside of the prostate. Tiny amounts of Dormant Cancer that may already exist outside the prostate can start to progress, or Progressing Cancer cells in the prostate may find their way out. In either case, External Progressing Cancer is usually undetectable in its early stages. We know it exists in many men because of the significant amount of cancer recurrence after surgery that removes all of the cancer confined to the prostate. Undetectable cancer exists outside the prostate in these cases. External Progressing Cancer has a very high probability of being incurable.

## Curable Internal Progressing Cancer

In the following analysis, we focus on Internal Progressing Cancer because it can be cured. We exclude in this example both Dormant Cancer, which needs no treatment, and External Progressing Cancer, which usually cannot be cured. Internal progressing cancer is divided into two phases: the Confined Progression Phase and the Penetrating Progression Phase, as illustrated by the figure.

The Confined Progression Phase starts when the first Dormant Cancer cell mutates beyond its current state and begins exponential cancer cell multiplication. The graph on FIG. 3 begins five years before (-5) the Transition Point (at year 0) to the Penetrating Progression Phase. The tiny tumor on the left grows exponentially in total volume each year. During this phase, the cancer registers a Gleason score of 6 or less, and is labeled T1 stage cancer if detected by biopsy. The light bars suggest that the Cure Ratio is close to 100% on the left half of the graph, where the probability is high that cancer is confined to the prostate. The Cure Ratio is defined as the actual cure rate for Internal Progressing Cancer at a specific point in time divided by the maximum cure rate if treated very early in the progression process. The Cure Ratio does not apply to the case of Dormant Cancer, which is harmless, or External Progressing Cancer, which cannot be cured.

Transition Point - We define year zero in the middle of **FIG. 3** as the Transition Point when the Cure Ratio starts to decline rapidly because of the increasing chance that cancer has penetrated the prostate and is growing outside. The Transition Point marks the change in slope in the Cure Ratio rather than any specific biological state. The light bar at year zero is slightly less than 100%, which means that not all Internal Progressing Cancer can be cured. The thin dark bar above it corresponds to the small risk that cancer has penetrated the prostate and is no longer curable.

Penetrating Progression Phase - The positive numbers on the right side of the graph on **FIG. 3** show the number of years into the Penetrating Progression Phase. This phase is characterized by the increasing risk that prostate cancer has penetrated beyond the prostate, shown by the dark ovals and the corresponding decreasing Cure Ratio. The shorter light bars on the right half of the graph indicate that the Cure Ratio drops steeply each year. The increasingly tall dark bars indicate the growing probability each year that cancer has penetrated beyond the prostate and is no longer curable. The diagram shows the dark cancer penetrating beyond the prostate in order to indicate the increasing risk that penetration has occurred. For example, at year +3 there is a roughly 25% chance that cancer has penetrated far enough to become incurable and a 75% chance that cancer has not penetrated and is still curable.

### The Cure Ratio Declines with Progression

The Cure Ratio graph on **FIG. 4** shows our estimate of the Cure Ratio for Internal Progressing Cancer. Early Treatment Has a Small Cure Ratio Benefit. The curve is relatively flat during the Confined Progression Phase on the left side of the graph. Therefore, treatment a year earlier at any point in this phase provides only a small benefit in terms of increased Cure Ratio. Late Treatment Has a High Cure Ratio Cost. The curve drops steeply during the Penetrating Progression Phase on the right side of the graph. Therefore, treatment a year later at any point in this phase imposes a high cost in terms of reduced Cure Ratio.

### Cure Ratio Calculation

The Cure Ratio calculation is shown by the flow chart on **FIG. 5**.

### Cancer Score Analysis

Cancer Score Analysis is carried out in the top module (500) on **FIG. 5**. Cancer Score (CS) is our summary measure of how favorable or unfavorable a particular cancer is. We use it to compare results on a consistent basis and to quantify trends more rigorously than is possible using discrete groups (of ranges of PSA, Gleason, Stage or combinations).

Cancer Score is a single measure of prostate cancer condition. Pretreatment PSA, Gleason and Stage are transformed into a single Cancer Score, as shown in the schematic on **FIG. 6**.

We base our Cancer Score on the projected ten year Johns Hopkins results and have confirmed its validity and usefulness on results from the Cleveland Clinic. Some example Cancer Score values for representative combinations of PSA, Gleason and Stage are shown on **FIG. 7**.

Cancer Scores are available for a wide range of PSA and Gleason values for Stages T1c through T3 - just like the Partin tables. These Cancer Score tables are represented schematically on **FIG. 8 -** with one table for each Stage.

Cancer Score is useful because it allows us to compare the results of different studies on a consistent basis. Here are a few of the ways populations are grouped for analysis: Favorable and Unfavorable cancer - defined in different ways; Favorable, Intermediate and Unfavorable cancer - defined in different ways; and Grouped by cancer variable: PSA - sometimes grouped in different ways, Gleason - sometimes grouped in different ways, and Stage - sometimes grouped in different ways.

Without Cancer Score, studies using one set of groups can't be compared directly to studies using any other set of groups. Even if two studies use the same definitions of groups the results may not be comparable. To be comparable they need both the same definition and the same distribution of PSA, Gleason and Stage in each of the equivalent groups. For example, two Favorable groups defined the same way may have significantly different average Cancer Scores because one Favorable Group has a high concentration of very early stage cancer with low PSA, Gleason and Stage - while the other group has a high concentration of poorer cancer that just squeaks above the definition of Favorable. These studies are not comparable even though they use the same definitions of groups.

Cancer Score solves one, more or all these problems. We can plot results using all these groups on a consistent basis using Cancer Score. A step is to calculate the average Cancer Score for each group in a study. The process for calculating average Cancer Score may use the following steps, as suggested by **FIG. 9**:

1. Create probability distribution table for PSA, Gleason and Stage.
2. Highlight combinations that define the group in both the Cancer Score and distribution tables (for example, the green cells).
3. Multiply each individual probability by its corresponding Cancer Score.
4. Sum the highlighted probability weighted Cancer Scores.
5. Divide the total weighted Cancer Scores by the total probability for the highlighted cells to get the average Cancer Score for the group.

Fortunately, most articles report in Table 1 at least the distributions of PSA, Gleason and Stage alone. Some studies provide more details of the joint distributions. This information allows us to estimate the joint probability distribution (step 1) to complete this process.

### Surgery Analysis

The goal of surgery analysis is to estimate the time path of progression after surgery that is comparable to no treatment for the full range of Cancer Scores. Surgery is chosen as the reference treatment for two reasons: no other treatment has proven to have better cure rates and detection of recurrence is prompt and unambiguous.

Cancer Score Analysis is performed in module **500** on **FIG. 5**. Medical studies of surgery outcomes are obtained and Cancer Scores are estimated for each analysis group in the study population, including the whole population.

Cancer Free Analysis is performed in module **502** on **FIG. 5**. Medical journal articles report biochemical freedom from cancer recurrence vs years after treatment for a variety of different groups. Fortunately, there is reasonably close agreement reported among the top doctors with long time series. Response surfaces are estimated using a number of studies for freedom from cancer recurrence after surgery as a function of time after treatment and Cancer Score.

Cancer Death Analysis is performed in module **504** on **FIG. 5**. Response surfaces are estimated using the results of the cancer free analysis combined with studies of cancer death following recurrence combined with long term studies of death from cancer after surgery. Johns Hopkins has done the most extensive long-term analysis of death following recurrence after surgery. The probability of cancer death is estimated as a function of time after surgery and Cancer Score.

Progression Analysis is performed in module **508** on **FIG. 5**. Response surfaces are estimated using the results of the cancer death analysis and cancer free analysis for progression after recurrence as a function of time and Cancer Score that is consistent with progression for no treatment. After an initial transition period, detectable progression (recurrence) seems to precede cancer death by roughly fifteen years on average

**No Treatment Analysis**

The goal of no treatment analysis is to estimate the time path of detectable progression for no treatment that is comparable to surgery for the full range of Cancer Scores.

Cancer Score Analysis is performed in module **500** on **FIG. 5**. Medical studies of no treatment outcomes are obtained and Cancer Scores are estimated for each analysis group in the study population, including the whole population.

Cancer Death Analysis is performed in module **506** on **FIG. 5**. Our analysis is informed by a wide variety of medical studies (e.g., two landmark studies to define the relationship between no treatment and surgery results). We start with the surgery results because they provide the most detail over time and Cancer Score. We consider the relationship between surgery and no treatment found in an ongoing randomized trial reported in the New England Journal of Medicine. Surgery was better but not by an enormous amount. We also consider the excellent long-term outcomes for men with very favorable cancer reported in articles by Albertsen, the most recent reported in the NEJM. It appears clear that long-term no treatment outcomes converge toward surgery for very favorable cancer. Response surfaces are estimated relative to surgery using these and other results. The probability of cancer death is estimated as a function of time after diagnosis and Cancer Score.

Progression Analysis is performed in module **510** on **FIG. 5**. Response surfaces are estimated using the results of the cancer death analysis and estimates of the lag from detectable progression to cancer death as a function of time and

Cancer Score that is consistent with progression for surgery. After an initial transition period, detectable progression seems to precede cancer death by roughly fifteen years on average

**Cure Ratio vs Cancer Score**

A goal can be to estimate for progressing cancer the time path of Cure Ratio as a function of Cancer Score at diagnosis.

For Progressing Cancer Surgery Cure Rate Analysis is performed in module **512** on **FIG. 5**. The probability of progressing cancer is calculated from the results of the no treatment progression analysis. The cure rate conditional on cancer progressing is calculated using this result combined with the overall cure rate of surgery for all cancer from the surgery progression analysis step. The result is an estimate of the response surface for progressing cancer cure rate as a function of time after surgery and Cancer Score.

This probability can be expressed as the sum of the probability of no progression and the probability of cure after progression:

$$\begin{array}{lll} \text{PrNCDbp@30} & = & \text{PrNCDbp@30(nt)} + \text{PrProg@30} * \text{PrNCDap@30} \\ \text{(no cancer death)} & & \text{(no progression)} \quad + \quad \text{(cure after progression)} \end{array}$$

Where:

PrNCDbp@30: Prob of no cancer death at 30 before progression

PrNCDbp@30(nt): Prob of no cancer death at 30 before progression for for no treatment
PrProg@30: Prob of progression at 30
PrNCDap@30: Prob no cancer death at 30 after progression

No Treatment is an example of this equation:

| | | | | | | | | |
|---|---|---|---|---|---|---|---|---|
| PrNCDbp@30 | = | PrNCDbp@30(nt) | + | PrProg@30 | * | PrNCDap@30 | | |
| (no cancer death) | | (no progression) | + | (cure) | | | | |
| 73% | = | 73% | + | 27% | * | 0% | | |

If cancer progresses no treatment lets it continue to progress - there is no cure from no treatment.

Surgery Now has some chance of curing cancer even though it will progress with no treatment - that is why men choose surgery. The surgery version of the equation for a Cancer Score of 95 looks like this:

| | | | | | | | | |
|---|---|---|---|---|---|---|---|---|
| PrNCDbp@30 | = | PrNCDbp@30(nt) | + | PrProg@30 | * | PrNCDap@30 | | |
| (no cancer death) | | (no progression) | + | (cure) | | | | |
| 84% | = | 73% | + | 27% | * | 40% | | |

Cure Ratio Analysis is performed in module **518** on **FIG. 5**. The Cure Ratio is the normalized cure rate with the cure rate for very favorable cancer defined as 100%. The Cure Ratio is an increasing function of Cancer Score - on the downside lower Cancer Scores lead to lower Cure Ratios.

**Progressing Cancer Analysis**

The goal is to estimate for internal progressing cancer the time path of Cancer Score from very early stage cancer to incurable.

Progressing Timing Analysis is performed in module **516** on **FIG. 5**. The PSA path of progressing cancer versus time is estimated from medical studies of the natural history of progressing cancer. A good article is by Berger. It shows PSA trajectories vs year of detection for progressing cancer for three groups. One group was detected early with Gleason 6 - favorable cancer. The second group was detected later with Gleason 7 - intermediate cancer. The third group was detected even later with Gleason 8-10 - unfavorable cancer. The average PSA at detection was lowest for favorable cancer and highest for unfavorable cancer as you would expect. We shifted the individual curves in time to form a continuous and consistent PSA path for progressing cancer independent of what stage it was detected.

Cancer Score Analysis is performed in module **500** on **FIG. 5**. Cancer Scores are estimated for each of the three Gleason detections groups, as shown on the previous graphs.

Cancer Score Progressing Analysis is performed in module **514** on **FIG. 5**.

Cancer Scores are related to the PSA path for progressing cancer.

Cancer Score Deterioration Analysis is performed in module **520** on **FIG. 5**. Cancer Scores are plotted vs time for the three detection groups. Detection of Gleason 6 remains the reference year (0). We estimate that Cancer Score slowly approaches 100 in prior years, to the left. We project continued roughly linear decline in Cancer Score as cancer progresses.

Cure Ratio for Progressing Cancer

The goal is to estimate for internal progressing cancer the deterioration in Cure Ratio over time, relative to the Transition Point.

Cure Ratio Deterioration Analysis is performed in module **522** on **FIG. 5**. The results of the Cure Ratio analysis and the progressing cancer analysis are combined to estimate the deterioration in Cure Ratio over time for progressing cancer. We have Cure Ratio as a function of Cancer Score and Cancer Score as a function of time. Together the allow us to plot Cure Ratio as a function of time.

## III. Organ Strategy System

Male prostates are subject to a variety of conditions, such as: Infection - a temporary condition; Volume growth caused by Benign Prostatic Hyperplasia (BPH) - a long-term condition; and Progressing prostate cancer - which is distinct from dormant prostate cancer. Prostate cancer is the focus of this organ strategy system example because of its importance and the level of medical controversy surrounding it. Similar strategic analysis can be applied to other conditions of the prostate, and of course to other organs.

### Competing Prostate Cancer Strategies

Doctors strongly disagree about the best prostate cancer strategy. Urologists and other prostate cancer specialists usually recommend a Cancer Dominated Strategy that emphasizes aggressive screening and immediate treatment of prostate cancer. Other doctors, who are more focused on preventive medicine, oppose screening, or do not recommend it, because they believe it leads to unnecessary treatment and side effects. We call this a Treatment Avoidance Strategy. We encourage consideration of a third strategy - the Best Life Strategy. This strategy leads to an optimal combination of screening and treatment that offers both a long life and a high level of well-being.

The American Urology Association and the American Cancer Society recommend PSA screening and, implicitly, the aggressive detection and immediate treatment of prostate cancer. Our analysis shows that over their lifetime men have a very high probability of dormant cancer that will not harm them and a low probability of progressing cancer that might. The harder doctors look for prostate cancer the more likely they are to find the dormant cancer that probably exists in the prostate. When focused on prostate cancer the best and most well intentioned medical care increases the chance of finding and unnecessarily treating dormant cancer. The result is an excessive risk of impotence, incontinence, and other side effects of unnecessary primary treatment - surgery and different types of radiation. As screening methods improve, many doctors are moving closer to finding and treating all detectable prostate cancer, whether dormant or not. Ironically, the risk of unnecessary side effects increases as screening and detection methods improve. Therefore, the best cancer dominated medical care may not be best.

The American College of Physicians, the American College of Preventive Medicine and the U.S. Preventative Services Task Force either oppose PSA screening, or do not recommend it, because it is associated with a high risk of over-treatment and excessive side effects. They believe men will be better off avoiding unnecessary treatment even if it leads to an increased risk of progression and death from prostate cancer. Without screening men have the choice of treatment when symptoms appear or avoiding all surgery and radiation treatment. No primary treatment has been common in some Scandinavian countries and is often recommended by U.S. doctors for men with short life expectancies. Excessive risk of suffering and death from prostate cancer are the disadvantages of treatment avoidance strategies. No screening seems like an overreaction to the excessive treatment and side effects of cancer dominated strategies. Many men don't like choosing between these strategies and are looking for a better way to deal with prostate cancer.

We recommend the Best Life Strategy. It offers excellent cure rates coupled with avoidance of unnecessary side effects. Optimal screening provides early warning of progression and allows treatment of only progressing cancer while the cure rate is still high. The Best Life Strategy is compelling and practical. In one approach, men could shift the focus of their screening to progressing cancer only rather than all cancer that is usually dormant. We expect many doctors to recommend this strategy once they understand its power and practical advantages. Later in this disclosure we will evaluate all the strategies for a typical man.

### Strategy System

The methods and systems are introduced briefly below along with a high level flow chart on **FIG. 10.** Entering personal *Profile* information starts the analysis process at step **1000.** Treatment is selected for analysis at step **1002** by the user or many treatments are analyzed in iterative fashion by the system. Opposing groups of doctors offer cancer dominated strategies that lead to unnecessary treatment and side effects or treatment avoidance strategies that lead to excessive risk of progression and death. The Best Life Strategy is optimal. The system analyzes a range of strategies in iterative fashion that are selected at step **1004**. The annual probability of treatment for each future year is projected at step **1006** based on the probability of the detection of progressing cancer based on the man's risk profile and the amount of early or late warning implicit in the strategy. The Cancer Cure Ratio is estimated for treatment each year at step **1008** based on the amount of early or late warning implicit in the strategy. The Cure Ratio is used to project the probability of recurrence after treatment over time and subsequent progression at step **1010**. The probability of death from prostate cancer is projected from the risk of subsequent progression for each year of potential treatment and then cumulated for an overall probability projection at step **1012**. The risk of death from other causes is considered in estimating the increase in the overall risk of death for each future year. For each year of treatment the probability of treatment in that year is used to weight the subsequent risk of side effects. The risks for each year of treatment are cumulated to estimate an overall risk of side effects for each future year at step **1014**. At step **1016** changes in Life Score are calculated for the increased risk of death by year and for the risk of side effects using the Emotional Weights entered by the user the his *Personal Profile.* The man's overall Life Score is reduced by the Life Score Impacts of increased risks of death and side effects at step **1018**. Results are summarized for each strategy at step **1020**.

**Life Score Calculation**

Our life outcomes simulator, shown on **FIG. 11**, is used to calculate Life Score Impacts in module **1016** and Life Scores in module **1018** on **FIG. 10** for a range of strategy scenarios. The probability of progressing cancer from a previous module is an example input. The user may supply information on his personal *Profile.* The system may supply a standard range of strategy scenarios.

**Strategy System Output**

The Life Score Graph on **FIG. 12** shows the Life Scores for a typical man for each treatment strategy. A value of 100% represents the Life Score in the absence of prostate cancer and serves as a point of reference. The graph on **FIG. 12** shows the strategy that maximizes Life Score based on information entered in the *Profile.* Life Score summarizes well-being and lifespan. Treatments with the same Life Score may actually represent a tradeoff between different well-beings and life spans. Differences in Life Score may be interpreted in the context of a man's total life. For example, if he expects to live thirty more years, a 3% difference in Life Score would be equivalent to almost 1 year of his life.

Life Score Impact is the reduction in Life Score from side effects and death from prostate cancer. It measures the drop from 100% on the Life Score graph on **FIG. 12.** The graph on **FIG. 13** shows the Life Score Impact of the treatment strategies. The total impact bars are split into a black section that shows the Life Score impact of death from prostate cancer and the lighter (colored coded) section that shows the Life Score impact of side effects from treatment. The bar with the smallest impact represents the treatment strategy with the highest Life Score.

**IV. Treatment Timing System**

The Treatment Timing System helps men and their doctors choose time for treatment of prostate cancer that offers the highest Life Score. The Treatment Timing System builds on the results of the Dynamic Screening System.

**Competing Life Score Impacts**

The timing of treatment for prostate cancer is a balancing act. Early treatment increases the chance of cure but may increase the risk of unnecessary treatment and side effects.

**Timing System**

The methods and systems are introduced briefly below along with a high level flow chart on **FIG. 14**. The Probabilities and Early Warning results from Dynamic Screening are an input to the Treatment Timing System at step **1400**. Other relevant information including personal *Profile* information is entered at step **1402**. Treatment is selected for analysis at step **1404** by the user or treatments are analyzed in iterative fashion by the system. The system analyzes a range of years of early and late warning in iterative fashion from step **1406**. The annual probability of treatment for each future year is projected based on the current probability of progressing cancer and years of early warning from the Dynamic Screening System in step **1408**. The Cancer Cure Ratio is estimated for treatment each year at step **1410** based on the amount of early or late warning. The Cure Ratio is used to project the probability of recurrence after treatment over time and subsequent progression at step **1414**. The probability of death from prostate cancer is projected from the risk of subsequent progression for each year of potential treatment and then cumulated for an overall probability projection at step **1412**. The risk of death from other causes is considered in estimating the increase in the overall risk of death for each future year. For each year of treatment the probability of treatment in that year is used to weight the subsequent risk of side effects. The risks for each year of treatment are cumulated to estimate an overall risk of side effects for each future year at step **1416**. At step **1418** changes in Life Score are calculated for the increased risk of death by year and for the risk of side effects using the Emotional Weights entered by the user in his *Personal Profile.* The man's overall Life Score is reduced by the Life Score Impacts of increased risks of death and side effects at step **1420**. Results are summarized for each strategy at step **1422**. A man, his doctor and his family can use Life Score simulations to help them choose the best timing for biopsy and treatment of progressing cancer (**1424**). For a biopsy, a doctor uses a device to inject thin hollow needles into the prostate to extract tissue. A pathologist exams the tissue and provides a diagnosis of prostate cancer if it exists. Primary treatment is intended to cure prostate cancer. It includes surgery to remove the prostate and various types of radiation to kill the cancer. A pathology report after surgery can provide useful information about the progress of cancer.

### Life Score Calculation

A life outcomes simulator, shown on **FIG. 15,** is used to calculate Life Score Impacts in module **1418** and Life Scores in module **1420** on **FIG. 14** for a range of treatment timing scenarios. The probability of progressing cancer from a previous module is an example input. The user may supply information on his personal *Profile.* The system may supply a standard range of treatment timing scenarios.

### Maximum Life Score

Life Score is a measure of well-being and length of life, based on the information entered in the *Profile.* The Life Score graph on **FIG. 16** shows how Life Score varies for a range of treatment timing. A value of 100% represents Life Score in the absence of prostate cancer and serves as a point of reference. The Life Score curve is relatively flat because timing of prostate cancer treatment causes relatively small changes in well-being and length of life. Timing is measured in years before and after the Transition Point of progressing cancer (year 0). Before the Transition Point the Cure Ratio declines relatively slowly. After the Transition Point the Cure Ratio drops more steeply as the risk increases that cancer has spread outside of the prostate.

The color of the line and treatment diamond (**1600**) on the graph on **FIG. 16** may depend on the primary treatment selected in the *Profile* - purple for surgery, red for dual radiation, light orange for seed radiation and dark red for external radiation. The treatment diamond (**1600**) on each graph shows the treatment timing that maximizes Life Score and minimizes Life Score impact. For Life Scores that are different, one way to interpret the difference is in the context of a total life. For example, if someone expects to live thirty more years, a 3% difference in Life Score would be equivalent to almost 1 year of life.

The green diamond (**1602**) on each graph shows a rough estimate of biopsy timing that corresponds with the treatment timing that maximizes Life Score. A first biopsy should occur roughly six months to a year before the optimal time for treatment, so the biopsy timing diamond will show up on the graphs approximately six months to a year or more before the treatment timing diamond. The actual size of the biopsy lead time depends on a variety of factors.

### Minimum Life Score Impact

Life Score Impact is the reduction in Life Score by side effects and death from prostate cancer. It measures the drop from 100% on the Life Score graph of the previous section and allows us to magnify the changes shown. The graph on FIG. 17 shows the Life Score Impact for the range of treatment timing.

The bottom colored curve (**1704**) shows the total Life Score Impact for the treatment you chose. It is the sum of reduction in Life Score from side effects and death from prostate cancer. The curve is more pronounced than on the previous graph because the scale has been expanded. It does not span the full range of possible impacts from 0% to 100%.

The treatment diamond (**1700**) on each graph shows the treatment timing that maximizes Life Score and minimizes Life Score impact. The green diamond (**1702**) on each graph shows a rough estimate of biopsy timing that corresponds with the treatment timing that maximizes Life Score.

The gray curve (**1708**) shows the Life Score Impact of all side effects. The impact is greatest on the left when the risk of unnecessary treatment is greatest.

The black curve (**1706**) shows the Life Score Impact of death from prostate cancer. The impact is greatest on the right when late treatment leads to a decrease in cure rate and an increased risk of cancer death.

### V. Dynamic Screening System

The Dynamic Screening System helps men and their doctors screen for progressing cancer, long-term conditions and short-term conditions. It provides early warning of progressing cancer while reducing the probability of unnecessary treatment and side effects. The results are useful inputs to the Optimal Treatment Timing System. The prostate is the organ of the body used in the examples. Conditions used as examples are progressing prostate cancer, prostate volume growth caused by Benign Prostatic Hyperplasia (BPH) and infections of the prostate. Both PSA and Free PSA tests can be used for screening. Other tests may supplement them or replace them.

The flow chart on FIG. **18** provides a high level overview of the Dynamic Screening System. For one person, biomarker and image results are input on the left (**1804**). For the prostate, they are PSA and Free PSA test results and ultrasound measurements of prostate volume. The experience of other men is input from the top (**1806**). A diagnosis of temporary conditions comes out the bottom (**1808**). For the prostate, an infection is the most common and serious temporary condition. Diagnoses of progressing cancer and long-term conditions (volume growth due to BPH for the prostate) are output on the right (**1810**). All output becomes part of all screening history (**1802**) and is fed back as the experience of other men to increase the power of Dynamic Screening (**1806**).

The flow chart on FIG. **19** shows some of the modules of the Dynamic Screening System.

A man or his doctor registers him as a new user and completes the Profile for him. The man analyzes his strategy alternatives using the Prostate Strategy System and chooses the Best Life Strategy.

Using the Dynamic Screening System, the man follows suggestions about the type and timing of primary and secondary screening tests. Typically the system will recommend a baseline prostate volume study and annual PSA and Free PSA tests. Free PSA tests are currently recommended; however, other tests may be recommended in the future in conjunction with Free PSA or to substitute for it. Tests results will be entered into the system for analysis and guidance. Steadily increasing PSA due to prostate enlargement from BPH, if rapid enough, will lead the system to suggest periodic prostate volume measurements to define the rate of growth. Tests results will be entered into the system for analysis and guidance.

The Dynamic Screening System will recognize the false alarms caused by infection and other temporary conditions, provide calming perspective, suggest new PSA and Free PSA tests after the infection or condition has passed, and analyze the results of new tests.

The Dynamic Screening System will recognize early warning of possible cancer progression and suggest additional confirmation tests. Confirmation tests may include other components of PSA such as Pro PSA and any other useful new markers developed in the future. In addition, a new prostate volume study may be suggested, perhaps using more expensive technology if rapid prostate enlargement is a factor. A second round of confirmation tests will be suggested - perhaps six months after the first. Additional confirmation tests will be suggested until progression has been confirmed or rejected.

The Dynamic Screening System will confirm a high probability of progressing cancer when its calculation shows the probability is high enough to warrant consideration of biopsy and treatment

The Optimal Treatment Timing System will calculate the optimal schedule for biopsy and treatment based on ongoing screening tests and the information entered in the *Profile.* The man and his advisors will use the results to schedule a first biopsy and subsequent treatment.

In our feedback learning process, the man or his doctor will provide follow up information for the system to analyze and incorporate for use by other men.

**Control System and Decisions**

The Control System and Decisions module (**1900**) and related modules (**1922, 1924, 1926**) on FIG. **19** help control the processing in the system and help men and their doctors makes decisions about testing.

**Control Systems and Decisions Module**

The Control System and Decisions module (**1900**) helps guide the user and control the system. Annual blood tests should start at age 50 and perhaps earlier. Additional blood tests may be advised in the cases of temporary infection and increased probability of progressing cancer. A baseline prostate volume study should be performed in conjunction with the first blood tests. Additional tests may be needed if the probability of progression increases and/or prostate volume appears to be increasing rapidly. Currently both PSA and Free PSA tests are advised for early warning. Other types of tests are recommended to confirm or reject early warning. Ultrasound measurement of prostate volume and perhaps other secondary tests are recommended to help predict the consequences of prostate enlargement from BPH and other ongoing conditions that affect primary test results.

**Screening Decision Examples**

There can be four decision points embedded in the ongoing screening process: Reject False Alarms, Escalate at Early Warning, Confirm Early Warning, and Rely on Backstop Warning.

Infections and other transitory events can cause jumps in PSA and raise false fears of prostate cancer for men. These PSA scares are common and troubling. One or more of our approaches can be configured to clearly identify these false alarms for what they are and avoid unnecessary fear and concern. They also allow rejection of the tests that caused the false alarms and their replacement by new tests after the infection has been eliminated or transitory event has passed.

Significant drops in the ratios of Free PSA to Total PSA can provide early warning of progressing cancer. Average Free PSA % drops very slowly, and Free PSA velocity % drops somewhat faster. Residual Free PSA velocity % is based on predictions for no cancer progression and provides much clearer early warning. We suggest adding additional blood tests as soon as early warning is noted. Over time the additional tests can confirm progression or reject it as a false warning.

Other markers for prostate cancer are under development. Some are being tested and may soon be available commercially, if they are not already. For example, Pro PSA has shown promise in studies reported in medical journals and others are in the pipeline. Our system will suggest additional tests be considered to confirm, or refute, early warning. The initial drop in Free PSA % ratios may be an accident, but a continued drop accompanied by shifts in ratios for other tests for can confirm a high probability of progressing cancer. Recall the adage that once may be an accident, twice a coincidence but three times is enemy action - with prostate cancer as the enemy in this case. Residual Free PSA velocity % may drop and give early warning four years before (-4) the cancer Transition Point. Additional confirming blood tests are administered soon after followed by more tests in each subsequent year. Residual velocity %s for XPSA and YPSA can be calculated using the second test in year -3. A drop in all residual velocity %s will help confirm progressing cancer. The ratios for actual tests may start higher or lower than for Free PSA and may move more or less for progressing cancer - or even in the opposite direction. There can be a comparison of how the ratios actually move compared to the expected movement for both progressing cancer and its absence.

Confirmation tests can increase the probability of progressing cancer enough to suggest a biopsy followed by optimal timing for treatment or only enough to intensify the screening process. A high but not sufficiently high probability can lead to suggestions for more frequent blood testing and additional prostate volume studies, perhaps using more accurate MRI imaging, in order to more accurately estimate the probability that cancer is progressing.

For most men, early warning and confirmation tests will allow appropriately early treatment of progressing cancer, but there is a small chance that they will not provide adequate confirmation. Fortunately, residual PSA velocity provides extremely strong backstop warning of progressing cancer and makes it very hard to miss optimal timing by very much. It is hard to miss the steep increase in residual PSA velocity that typically occurs several years before the Transition Point when the Cure Ratio begins to drop steeply.

**Information Value of Test Timing Module**

The Information Value of Test Timing module (**1922**) on **FIG. 19** assesses the value of additional tests based on the existing output of the system and possible simulations of the impact of additional tests. For example, it may create a dummy pair of PSA and Free PSA tests, run the system with and without them included and observe the reduction in trend uncertainty from the additional test pair.

**Test Timing Recommendations Module**

Test decision recommendations about the type and timing of primary and secondary tests are created in the Test Timing Recommendations module (**1924**) on FIG. **19**. Examples of recommendations are presented below.

The system may suggest base tests starting at age 50 or earlier based on risk factors and personal preference. They might consist of: Primary Tests - Two annual blood tests: Total PSA and Free PSA; and Secondary Tests - One baseline prostate volume study.

The system may suggest next tests based on evaluation of the most recent tests: Base tests, Prostate enlargement tests, Retests after false alarms, and Confirmation tests after early warning.

The system may suggest periodic prostate volume studies for men with rapidly growing prostates due to BPH. For some men with extremely high growth rates the system will suggest more accurate volume studies using MRI or other high accuracy imaging.

In the Test Validity Test step the system may recognize the false alarms raised by PSA scares from infections and other temporary conditions. New PSA tests will be suggested after the condition has passed.

The Probability Estimate step may provide early warning of the possibility of progressing cancer. Additional primary tests will be suggested when early warning of progressing cancer is recognized. Additional and perhaps more accurate volume studies may be suggested for men with prostates growing from BPH.

**Test Timing Decisions Module**

The user can explore the implications of the number and timing of additional tests in the Test Timing Decisions module (**1926**) on FIG. **19**. Among other capabilities, the user can submit hypothetical results to discover the relative value of different combinations of tests and timing.

**Trends and Temporary Conditions**

Three related modules of the Dynamic Screening System (**FIG. 19**) are described in this section: the Trends module (**1902**), the Temporary Conditions Module (**1903**) and the Bayesian Probabilities module for temporary conditions (**1904**).

## Trends Module

The Trends module (**1902**) on **FIG. 19** estimates trends in PSA and Free PSA after excluding results that are outside of a reasonable tolerance range. Details of the Trends module are shown on **FIG. 20** and described in the following sections.

## Probability Leverage Data Management Module

The Probability Leverage Data Management module (**2000**) starts the trends process and controls its outer loop of iterations. Inputs may include: PSA and Free PSA dates and test results as entered by the user and feedback of the pair of highest leverage test results to remove from next the iteration. Outputs may include: PSA and Free PSA dates and test results to be used for each trend - the Red Stop trend, the Yellow Caution trend and the Green trend. These trends offer different amounts of sensitivity to anomalous tests and early warning. The module controls the outer iterative loop to find the pair of test results that creates the largest increase in the probability of progressing cancer. The first pair removed drops the trend from the red stop trend to the yellow caution trend. The second pair removed drops the trend from the yellow caution trend to the green trend.

## Related Changes Module

The Related Changes module (**2002**) may adjust test results for related changes with known impacts. Some treatments and changes in medication and life style can affect the level of PSA and the results of other screening tests. For example, treatments to reduce the size of the prostate, like a TURP, can cause a sharp drop in the level of PSA and other markers. If entered into a man's *Profile*, related changes can be used to adjust the test results, trends and predictions of PSA and other variables after the change.
Some treatments for prostate cancer conditions can significantly alter the production of PSA and other screening variables. The results of the change can be predicted based on the experience of other men. Some medications can alter the production of PSA and other screening variables. The results of the change can be predicted based on the experience of other men. Some changes in lifestyle can alter the production of PSA and other screening variables. The results of the change can be predicted based on the experience of other men for changes in: Diet, Exercise, Supplements, Recreational Drugs and also the brand and method the tests.

## Tolerance Data Management Module

The Tolerance Data Management module (**2004**) controls the inner loop that excludes test results until all included test results are within the tolerance region of the trends. Inputs may include: PSA and Free PSA dates and test results to be used for each trend: the Red Stop trend, the Yellow Caution trend and the Green trend; and Feedback about the test Pair farthest from the tolerance test region to remove from next iteration. Output may included: PSA and Free PSA dates and test results to be used for each tolerance test trend.
The module controls the inner loop of trend calculations to find trends that fit the data with all test results within the tolerance region. Pairs of tests farthest from tolerance by ratio are removed during each iteration until all remaining test results are within the tolerance region.

## Functional Form for PSA and Free PSA Modules

The Functional Form PSA and Functional Form fPSA modules (**2008**) may change the functional forms used to fit the trends based on the number and duration of test results, the characteristics of the trends and other factors that may be relevant. Inputs may include: PSA and Free PSA dates and test results to be used for each tolerance test trend; and feedback about PSA Velocity and Free PSA Velocity from the estimated trends. Output may include: Functional forms used to estimate PSA and Free PSA trends and constraints on function parameters.
The module may select functional forms used to estimate trends for PSA and Free PSA and constrain the parameters used. Example selection rules might include:

- One data point

    o No trend is estimated.

- Two data points
    - o Linear trend is estimated (two parameters)
        - ■ PSA(t) = a + b * t
        - ■ fPSA(t) = a + b * t
- Three or more data points through five years
    - o Power-law trend is estimated (three parameters)
        - ■ PSA(t) = a + b * (t - to) ^ c
        - ■ fPSA(t) = a + b * (t -to) ^ c
    - o Power-law parameter (c) is constrained to limit curvature
        - ■ c increases with number of test results
        - ■ c increases with duration of test period up to five years
        - ■ c depends on feedback of velocity from estimated trend
- Six or more data points and five to seven years
    - o Power-law trend from above is used for the most recent five years.
    - o Linear trend is used from the start of tests to five years before the last test.
        - ■ Linear trend equals power-law trend at year five.
            - PSA(t) = a + b * t
            - fPSA(t) = a + b * t
        - ■ Slope (b) is estimated to fit data (one new parameter)
        - ■ Level (a) is adjusted so linear trend equals power-law at year five.
- Eight or more data points and more than seven years
    - o Power-law trend from above is used for the most recent five years.
    - o Linear trend is used from half way between the start of tests and five years before the last test to five years before the last test, as above.
    - o Linear trend is used from the start of tests to the halfway point.
        - ■ Linear trend equals next linear trend at halfway point.
            - PSA(t) = a + b * t
            - fPSA(t) = a + b * t
        - ■ Slope (b) is estimated to fit data (one new parameter)
        - ■ Level (a) is adjusted so first linear trend equals second linear trend at halfway point.

The rationale for some functional forms is presented as an example. A line is the only trend we can fit when we have only two data points. Dynamic Screening may fit a curved trend to curved data, including accelerating PSA. The power-law function has advantages over the quadratic and other three parameter curved functions because it offers the most power for progressing cancer with little significant compromise for no progression conditions. For these reasons, the power-law function seems the dominant choice because it is the functional form of progressing cancer, our focus. Moreover, it has an intuitive, one parameter way of constraining curvature.

The power-law function can be constrained to a linear function by setting (c) = 1.0. Curvature can be limited by constraining the range of values allowed for (c).

- Power-law trend is estimated (three parameters)

o PSA(t) = a + b * (t - to) ^ c
o fPSA(t) = a + b * (t - to) ^ c

- Power-law parameter (c) is constrained to limit curvature

o c increases with number of test results
o c increases with duration of test period up to five years
o c depends on feedback of velocity from estimated trend

Decisions about limitation on curvature are a balancing act. Tight limits prevent a few data points leading to an estimated trend with far more curvature than is likely but may cause some underestimation of unusually large curvature.

**Estimate Trends for PSA and Free PSA Modules**

The Estimate Trends for PSA and Free PSA modules (**2008**) may use a variety of methods to estimate trends for trends included in the iteration controlled by the Tolerance Data Management module (**2004**). Inputs may include: PSA and Free PSA dates and test results to be used for each tolerance test trend; and the functional forms used to estimate PSA and Free PSA trends. Output may include: PSA and Free PSA trends; and Feedback about PSA Velocity and Free PSA Velocity from estimated trends. Least squares methods may be used to estimate the parameters for the chosen functional form that best fits the test results. If the unconstrained estimate of (c) for curvature is within the constraints then it is used. If the unconstrained estimate of (c) is beyond a constraint then the constraint is used for (c) and the trend is re-estimated. The trend equations may be used to define trend values at each test date, including projections to the most recent test dates.

**Identify Results Farthest from Tolerance Module**

The Identify Results Farthest from Tolerance module (**2010**) determines which test pairs are candidates for exclusion from trend estimation on the next iteration controlled by the Tolerance Data Management module (**2004**). Inputs may include: PSA and Free PSA trends; and PSA and Free PSA dates and test results to be used for each tolerance test trend. Outputs may include: Feedback about the Pair of PSA and Free PSA tests farthest from tolerance (for removal); and PSA and Free PSA trends that have all test results within tolerance and the PSA and Free PSA dates and test results within tolerance of trends.

The tolerance region for each test date may be a two dimensional region around the trends defined by two variables like PSA and Free PSA or one variable like PSA and the ratio of one variable to the other like Free PSA divided by PSA. Most of the test results within the tolerance region may be explained by random variation. Most of the test results outside of the tolerance region may be explained by short-term conditions. The shape of the tolerance region may approach a rectangle for highly correlated dimensions, may approach an oval for relatively uncorrelated dimensions or be something in between. The shape of tolerance region may be adjusted to produce unbiased trends that are not distorted by temporary conditions. For example, infections are the most prevalent temporary condition and cause PSA to increase and the Free PSA % to decrease. Some of the area of the tolerance region in that direction may be reduced in order to reduce the bias caused by infections.

For each tolerance iteration, pairs of PSA and Free PSA tests are compared to the estimated trends. Beyond-tolerance pairs that are farthest by ratio from the trend at the test date are identified for removal from the next tolerance iteration through the feedback process. The tolerance iteration process stops the first time all pairs are within tolerance. The trends and remaining pairs are output to the next process step.

An oval or ellipse shape may be appropriate for relatively uncorrelated variables such as PSA and Free PSA %. An elliptical tolerance region may be calculated in the following way. With FreePSA on the Y axis and PSA on the X axis of a coordinate plane, the upper half of an ellipse is defined by

$$y = f + \sqrt{b^2\left(1 - \frac{(x-p)^2}{a^2}\right)}$$

where *f* = FreePSAtrend, *p* = PSAtrend, *b* = (some tolerance value * FreePSAtrend), and *a* = (some tolerance value * PSAtrend). If the FreePSA point is less than the FreePSAtrend value, FreePSA' is defined as (FreePSAtrend+(FreePSAtrend-FreePSA)), otherwise FreePSA' is FreePSA. If the point (PSA, FreePSA') is above the ellipse then both tests are excluded, otherwise both tests are included.

**Calculate Velocity Uncertainties Module**

The Calculate Velocity Uncertainties module (**2012**) may calculate velocity uncertainties for each trend for use by the Bayesian calculation of the probability of progressing cancer. Inputs may include: PSA and Free PSA trends from the removal of each possible high leverage pair; and PSA and Free PSA dates and test results with each possible high leverage pair. Outputs may include: Feedback: PSA and Free PSA velocity uncertainties to the rest of the system; PSA and Free PSA trends with the highest leverage pair removed; and PSA and Free PSA dates and test results with the highest leverage pair removed.

A variety of methods can be used to calculate uncertainties in the velocities, like PSA Velocity, and velocity ratios, like Free PSA Velocity %, which is the ratio of Free PSA Velocity to PSA Velocity. Monte Carlo methods may be used to calculate the velocity distributions for each variable around the trends, as shown on **FIG. 21**. Probability distributions for the variables may come from studies of other men or the experience of the man under consideration. In the case of Free PSA, variation in Free PSA is correlated with variation in Free PSA so this relationship is considered by the method used to estimate the distribution of Free PSA % and Free PSA in light of the randomly drawn corresponding PSA result.

**Identify Results with Highest Probability Leverage Module**

The Identify Results with Highest Probability Leverage module (**2014**) may determine which test pairs seem to be most anomalous and are the best candidates for elimination to create the yellow Caution trend and the Green trend. Test pairs may be tested for impact on the probability of progressing cancer in real time using the rest of the system (**2018**) or using rules of thumb or reduced-form results based on off-line simulations using the rest of the system. Inputs may include: PSA and Free PSA trends that have all test results within tolerance; PSA and Free PSA dates and test results within tolerance of trends; and Probability of progressing cancer from the rest of the system. Outputs may include: Feedback about the Pair of highest leverage PSA and Free PSA tests results (for removal); PSA and Free PSA trends for Stop, Caution and Green trends; and PSA and Free PSA dates and test results for Stop, Caution and Green trends.

For the red Stop trends the first set of PSA and Free PSA trends may be passed through to the next step. The red Stop trend is most sensitive to anomalous test results but provides the earliest warning if cancer is progressing.

For the yellow Caution trends, the most likely high leverage pairs of PSA and Free PSA tests may be removed one at a time. Trends may be calculated and the results may be sent to the rest of the system for calculation of the probability of progressing cancer. The pair that causes the largest change in the probability of progressing cancer may be identified and removed. The trends estimated without that pair may be passed through to the next step as the yellow Caution trends. The removed pair may feed back to the probability leverage data management step for exclusion from the start of the Green trend iteration.

For the Green trends, the most likely high leverage pairs of PSA and Free PSA tests may be removed one at a time. Trends may be calculated and the results may be sent to the rest of the system for calculation of the probability of progressing cancer. The pair that causes the largest change in the probability of progressing cancer may be identified and removed. The trends estimated without that pair may be passed through to the next step as the Green trends.

In many cases significant probabilities of progression may not be calculated by the rest of the Dynamic Screening system. Velocities may be very low, or trend uncertainty may be high. In these cases, fall back methods may be used to estimate which pairs of results will tend to increase the probability the most. Pairs that increase PSA Velocity trends the most and decrease Free PSA Velocity % trends the most are likely to increase the probability the most. The relative impact of the two velocities may be calculated from the specific conditions or estimated from off line calculations for many men.

**Progression Probabilities from Rest of the System**

The trends and their uncertainties may be sent to the rest of the system (2018) for analysis and calculation of the probability of progressing cancer. This function may be performed in real time or as off-line simulations where the results are used as rules of thumb or reduced form models. Inputs may include: PSA and

Free PSA trends from the removal of each possible high leverage pair; and PSA and Free PSA velocity uncertainties. Outputs may include: Probability of progressing cancer from the removal of each possible high leverage pair.

**Create Stop, Caution and Green Trends Module**

The Create Stop, Caution and Green Trends module (**2016**) may collect, label and output the three trends for use by the rest of the system and for display, as well as pass them through to the next step. Inputs may include: PSA and Free PSA trends with the highest leverage pair removed; and PSA and Free PSA dates and test results with the highest leverage pair removed. Outputs may include:
PSA and Free PSA trends for Stop, Caution and Green trends; and PSA and Free PSA dates and test results for Stop, Caution and Green trends.

**Temporary Conditions Module**

The Temporary Conditions module (**1904**) shown on **FIG. 19** may assess the possibility of temporary conditions and their severity. Inputs may include trends and pairs of test results. Outputs may include estimates of the severity of the temporary condition. Infection of the prostate is an example. Severity of prostate infections may be indicated by how much a PSA test result exceeds the value for that date predicted by the trend and by how much the corresponding test Free PSA % is below the value for that date predicted by the trends

**Temporary Probabilities Module**

The Temporary Probabilities Module (**1906**) shown on **FIG. 19** may estimate the probability of temporary conditions, like infection of the prostate. A variety of methods may be used to calculate the estimate of the probability, including Bayesian methods. In broad terms a prior probability of an infection is augmented by estimates of the probability of the observed test results, such as PSA and Free PSA %, given that the prostate is infected and given it is not infected.

**Long-Term Conditions Severity**

The Long-Term Conditions Severity module (**1920**) on **FIG. 19** estimates the severity of long-term conditions assuming cancer is not progressing. Growth in prostate volume is the long-term condition we are considering as an example. Prostate volume growth is caused by Benign Prostatic Hyperplasia and causes bothersome symptoms like frequent urination and difficulty urinating. The severity of the condition is measured by prostate volume measured in cubic centimeters and volume velocity measured by the increase in cubic centimeters per year.

**Volume Measurement**

Prostate volume can be measured from images of the prostate. Ultrasound is the most common and cost effective imaging technique for measuring prostate volume, but MRI and other techniques can be used effectively. Multiple volume measurements over time are needed to fit a trend and estimate volume velocity. Every man should consider a baseline study done when he reaches age 50 or earlier for men with higher risk of cancer or a history of prostate enlargement. Volume studies can be as infrequent as every five years for men with no evidence of prostate enlargement and no indications of progressing cancer. More frequent studies are suggested for men with enlarging prostates due to BPH and with increasing probability of progressing cancer.
PSA trends can be used to estimate volume and volume velocity if no volume measurements are available and can be used in conjunction with one or more volume measurements to improve the estimates of volume velocity. Progressing prostate cancer increases PSA without increasing prostate volume significantly. Therefore, we explicitly assume that cancer is not progressing when we use PSA trends to estimate prostate volume trends. Progressing cancer is a competing hypothesis to explain increasing PSA.

**Multiple Volume Measurements**

Multiple volume measurements are the best way to estimate a volume trend. The trend defines volume and volume velocity at each point between the first and last measurement and can be used to project them beyond the last measurement to the present. However, only two volume measurements over a short period of time can lead to unreasonably high or negative velocity estimates because of variation in volume measurements. Therefore, the Volume Estimation System uses the PSA Trend to constrain the range of reasonable volume velocities. The PSA trend values divided by volume trend values provides a good estimate of PSA density. PSA Velocities from the PSA trend divided by PSA density provide a good estimate of Volume Velocities, which can be used to constrain the overall estimate of Volume Velocities to reasonable values. **FIG. 22** shows the Volume Estimation System for multiple volume measurements.

**One Volume Measurement**

One volume measurement alone does not allow estimation of a volume trend, but it substantially improves it compared to estimates with no volume measurement. The Volume Estimation System uses the volume measurement and the PSA Trend to estimate the volume trend. The PSA trend value at the time of the volume measurement divided by the volume measurement provides a good estimate of PSA density. The PSA trend divided by PSA density provides a good estimate of the Volume trend, which can be used to project current Volume and
Volume Velocity. **FIG. 23** shows the Volume Estimation System for one volume measurement.

**Volume Estimates from PSA Trend - No Volume Measurement**

Many men have no volume measurement. The Volume Estimation System uses the PSA Trend and three typical PSA densities to estimate the volume trend.
The densities are chosen from population data based on the man's age: average, high (upper $10^{th}$ percentile) and low (lower $10^{th}$ percentile). The PSA trend divided by one or more of the PSA densities provides one or more estimates of the Volume trend, which can be used to project current Volume and Volume Velocity. **FIG. 24** shows the Volume Estimation System for no volume measurement.

**Long-Term Conditions**

The Long-Term Conditions module (**1914**) on **FIG. 19** predicts non-cancer primary test results based on past experience, recent secondary test results and the experience of other men. Prostate volume growth is the long-term condition of concern for the prostate. Volume growth is caused by Benign Prostatic Hyperplasia. PSA and Free PSA increase as the prostate grows.
Predicted PSA Velocity, Free PSA Velocity and Free PSA Velocity % provide a reference against which actual results can be compared and analyzed for progressing cancer. These predicted velocities are subtracted from trend velocities in order to calculate residual velocities. Free PSA is the preferred second screening test and is described below; however, the results of other screening tests can be predicted in the same way either as a substitute or complementary confirming test.
The prediction methods vary depending on the amount of data available. Prostate volume can be measured cost-effectively using ultrasound images and can be measured using MRI and other images. The method of predicting trends in PSA and Free PSA Velocities depends on the number of volume measurements available. Example methods are shown for three cases: multiple volume measurements, one volume measurement and no volume measurement. Multiple volume measurements are used to estimate a volume trend and calculate the corresponding volume velocity trend.
Predictions of velocities for no progressing cancer improve as length of the PSA and Free PSA testing history increases and the number of tests increases. Example methods are shown for two cases: long testing history and Short testing history. Not all combinations of volume measurements and testing history are shown, but they can be inferred from the examples shown.
Finally, the system estimates uncertainty in the predicted results measured by standard deviation in the predicted velocities. These standard deviations are inputs to the calculation of the probability of progressing cancer and the years of early warning for progressing cancer.

**Multiple Volume Measurements and Long Screening History**

This method may apply when two or more volume measurements are available along with a long screening history, as shown on FIG. 25. A volume trend is estimated. Volume velocity is compared with population velocities for the same volumes and may be constrained for reasonableness.
In module (**2500**) PSA and fPSA blood test results are input to the method. The Volume trend is used to estimate a past volume (**2514**) in order to estimate past densities. Past PSA and fPSA trend results are divided (**2504** and **2524**) by past trend volume or volumes to calculate densities. Average values are calculated for fPSA% (**2516**), Free PSA divided by PSA, but they play a secondary role when a long screening history is available. Please see the short screening history example to learn about its stronger role. Velocities are calculated as annual changes - dPSA/dt (**2506**) and dfPSA/dt (**2526**). fPSA Vel % (**2518**) is calculated as Free PSA Velocity divided by PSA Velocity using a combination of projected PSA Density Velocity and projected fPSA Density Velocity. However, fPSA Vel % may play a secondary role in projecting fPSA Density Velocity when a long screening history is available. PSA Density Velocity (**2508**) is projected from past trends. fPSA Density Velocity (**2528**) is calculated using primarily the projection of Free PSA Density Velocity (2526) and secondarily the estimate of fPSA Vel % (**2518**). Current Volume Velocity (**2520**) is estimated from the Volume trend. A Volume Velocity trend is calculated from the Volume trend. PSA and Free PSA Velocities are calculated by multiplying (**2510** and **2530**) the current volume velocity (**2520**) times the projected density velocities (**2508** and **2528**). Predicted values for PSA and Free PSA with no progressing cancer are calculated by integrating (2512 and 2532) the PSA and Free PSA velocities and adding them to the PSA and Free PSA trend values at the start of the integration period.

**One Volume Measurement and Long Screening History**

This method may apply when one volume measurement is available along with a long screening history, as shown on **FIG. 26**. A reduced form of this method may be used that predicts current PSA Velocity based on the volume measurement and PSA trend value at the time of the measurement and Free PSA velocity based on current predicted PSA Velocity and the Free PSA Velocity % trend.

In module (**2600**) PSA and fPSA blood test results are input to the method. The one Volume measurement (**2614**) is used to estimate past densities and to predict current volume velocity (**2620**). Past PSA and fPSA trend results are divided (**2604** and **2624**) by past volume to calculate past densities. Average values are calculated for fPSA% (**2616**), Free PSA divided by PSA, but they play a secondary role when a long screening history is available. Please see the short screening history example to learn about its stronger role. Velocities are calculated as annual changes - dPSA/dt (**2606**) and dfPSA/dt (**2626**). fPSA Vel % (**2618**) is calculated as Free PSA Velocity divided by PSA Velocity using a combination of projected PSA Density Velocity and projected fPSA Density Velocity. However, fPSA Vel % may play a secondary role in projecting fPSA Density Velocity when a long screening history is available. PSA Density Velocity (**2608**) is projected from past trends. fPSA Density Velocity (**2628**) is calculated using primarily the projection of Free PSA Density Velocity (**2626**) and secondarily the estimate of fPSA Vel % (**2618**). Current Volume Velocity (**2620**) is estimated from the one volume test (**2614**). PSA and Free PSA Velocities are calculated by multiplying (**2610** and **2630**) the current volume velocity (**2620**) times the projected density velocities (**2608** and **2628**). Predicted values for PSA and Free PSA with no progressing cancer are calculated by integrating (**2612** and **2632**) the

PSA and Free PSA velocities and adding them to the PSA and Free PSA trend values at the start of the integration period.

**No Volume Measurement and Long Screening History**

This method may apply when no volume measurement is available along with a long screening history, as shown on **FIG. 27**. A reduced form of this method may be used that predicts current PSA Velocity based on past PSA levels and

Free PSA velocity based on current predicted PSA Velocity and the Free PSA Velocity % trend.

In module (**2700**) PSA and fPSA blood test results are input to the method. The PSA trend is divided (**2704**) by age specific population PSA densities to estimate prostate volumes (**2714**). This volume estimate is used to predict current volume velocity (**2720**). fPSA trend results are divided (**2724**) by volume estimates to calculate Free PSA densities. Average values are calculated for fPSA% (**2716**), Free PSA divided by PSA, but they play a secondary role when a long screening history is available. Please see the short screening history example to learn about its stronger role. Velocities are calculated as annual changes - dPSA/dt (**2706**) and dfPSA/dt (**2726**). fPSA Vel % (**2718**) is calculated as Free PSA Velocity divided by PSA Velocity using a combination of projected PSA Density Velocity based on population PSA densities and projected fPSA Density Velocity based on population PSA densities and fPSA Vel %. However, fPSA Vel % may play a secondary role in projecting fPSA Density Velocity when a long screening history is available. PSA Density Velocity (**2708**) is projected from past trends. fPSA Density Velocity (**2728**) is calculated using primarily the projection of Free PSA Density Velocity (**2726**) and secondarily the estimate of fPSA Vel % (**2718**). Current Volume Velocity (**2720**) is estimated from the volume estimate (**2714**). PSA and Free PSA Velocities are calculated by multiplying (**2710** and **2730**) the current volume velocity (**2720**) times the projected density velocities (**2708** and **2728**). Predicted values for PSA and Free PSA with no progressing cancer are calculated by integrating (**2712** and **2732**) the PSA and Free PSA velocities and adding them to the PSA and Free PSA trend values at the start of the integration period.

**No Volume Measurement and Short Screening History**

This method may apply when no volume measurement is available along with a short screening history, as shown on **FIG. 28**. A reduced form of this method may be used that predicts current PSA Velocity based on past PSA levels and

Free PSA velocity based on current predicted PSA Velocity and the Free PSA Velocity % trend or just the Free PSA % trend.

In module (**2800**) PSA and fPSA blood test results are input to the method. The PSA trend is divided (**2804**) by age specific population PSA densities to estimate prostate volumes (**2814**). This volume estimate is used to predict current volume velocity (**2820**). fPSA trend results are divided (**2824**) by volume estimates to calculate Free PSA densities. Average values are calculated for fPSA% (**2816**), Free PSA divided by PSA. This value is used as the estimate for fPSA Velocity % when no history of that variable is available for projection. Velocities are calculated as annual changes - dPSA/dt (**2806**) and dfPSA/dt (**2826**). dfPSA/dt may not be available. If available fPSA Vel % (**2818**) is calculated as Free PSA Velocity divided by PSA Velocity using a combination of projected PSA Density Velocity based on population PSA densities and projected fPSA Density Velocity based on population PSA densities and fPSA Vel %. However, fPSA Vel % may play a stronger role in projecting fPSA Density Velocity when a short screening history is available. PSA Density Velocity (**2808**) is projected from past trends. fPSA Density Velocity (**2828**) is calculated using primarily the projection of PSA Density Velocity (**2808**) and the estimate of Free PSA Velocity % (**2818**) and secondarily the estimate of fPSA Vel (**2826**). Current Volume Velocity (**2820**) is estimated from the volume estimate (**2814**). PSA and Free PSA Velocities are calculated by multiplying (**2810** and **2830**) the current volume velocity (**2820**) times the projected density velocities (**2808** and **2828**). Predicted values for PSA and Free PSA with no progressing cancer are calculated by integrating (**2812** and **2832**) the PSA and Free PSA velocities and adding them to the PSA and Free PSA trend values at the start of the integration period.

**Uncertainty in Predicted Values for No Progressing Cancer**

The process for estimating the probability of long-term conditions like progressing cancer and volume growth depends on the total amount of uncertainty in the predicted PSA Velocity, Free PSA Velocity and Free PSA Velocity %. The system may estimate uncertainty in the predicted results using standard deviation in the predicted velocities.

Trend uncertainty and biologic uncertainty contribute to the total amount of uncertainty in PSA and Free PSA trends. Trend uncertainty is caused mostly by short-term biologic variation with some test measurement variation thrown in, module **1902** on **FIG. 19**. The other source of variation is long-term biologic uncertainty about volume growth. It reflects variation in PSA and Free PSA for men with similar types of volume growth. We use standard deviation to define and measure the amount of variation of each type.

Trend and biologic variation may move independently of each other, so we can't simply add them together to produce total variation. The table below shows total standard deviation for PSA Velocity for four trend standard deviations and one biologic standard deviation, assuming they are minimally correlated.

| | | | | |
|---|---|---|---|---|
| Trend Standard Deviation | 0.05 | 0.10 | 0.30 | 0.60 |
| Biologic Standard Deviation | 0.10 | 0.10 | 0.10 | 0.10 |
| Total Standard Deviation | 0.11 | 0.14 | 0.32 | 0.61 |

The results show that the total tends to be dominated by the larger standard deviation, which will usually be the trend standard deviation in the early stages of Dynamic Screening. Even when the component standard deviations are equal at 0.10, the total is only 0.14 rather than the simple sum of 0.20. This result reflects, in part, the independence of the two sources of variation.

Variation in Free PSA Velocity and Free PSA Velocity % trends may be handled in an analogous way to combine trend and biologic standard deviations.

**Residual Values**

Maps of residual values and velocities are used to provide early warning of progressing cancer. Residual values are calculated by subtracting predicted values from actual values. Residual velocities can be calculated in several ways. Residual velocities can be calculated by subtracting predicted velocity trends from estimated velocity trends, for example: Residual PSA Velocity (dPSA/dt) = Estimated trend PSA Velocity minus Predicted PSA Velocity. Residual velocities can be calculated by subtracting predicted trends from estimated trends and then calculating the rate of change, for example: Residual PSA Velocity = the annual rate of change in Residual PSA where Residual PSA = Estimated trend PSA minus Predicted PSA trend.

**Residual Value Maps**

Residual maps of values and velocities may be presented as plots of Free PSA % vs PSA and Free PSA Velocity % vs PSA Velocity, where data points may be determined by the dates of blood tests or spaced by year or some other unit of time. Please refer to **FIG. 29** for one way of creating residual value maps.

The residual calculators subtract the predicted value from the estimated trend value of PSA (**2900**) and Free PSA (**2920**). Residual PSA, Free PSA and Free PSA % (their ratio) are plotted vs time (**2910**). Residual Free PSA or Residual Free PSA % is plotted vs residual PSA (**2912**) for each blood test or for specified dates, perhaps one year apart. Residual PSA Velocity (2904) and Free PSA Velocity (**2924**) are calculated by differentiating the residual values. Residual PSA Velocity, Residual Free PSA Velocity and Residual Free PSA Velocity % (their ratio) are plotted vs time (**2914**). Residual Free PSA Velocity or Residual Free PSA Velocity % is plotted vs residual PSA Velocity (**2916**) for each blood test or for specified dates, perhaps one year apart.

**Alternative Method for Predicting PSA and Free PSA**

An alternative method for predicting PSA and Free to be used in calculating residual values is presented in this section. Predicted results are used as a baseline to subtract from actual results to create residual results. The prediction method is introduced below and shown on **FIG. 30**.

The screening history of all men who have provided data is combined with the screening history of the man making prostate cancer decisions. Possible time paths are generated based on the experience of men with progressing cancer. Time paths for the man without progressing cancer are predicted using a combination of concurrent and sequential methods - which are described in later sections. Predicted values are calculated as the sum of the no cancer prediction and the progressing cancer hypothesis. The error calculators subtract the predicted value from the actual value of PSA and Free PSA. PSA and Free PSA values and prediction errors are plotted vs. time. Free PSA is plotted vs. residual PSA for their values and prediction errors. The best prediction is estimated using least squares calculations and other methods to find the prediction that best matches actual results using an iterative survey of a large number of predictions.

**Progressing Cancer**

Early detection of progressing cancer is a function of Dynamic Screening.

**Progressing Cancer Trends and Distributions**

The Progressing Cancer module (**1910**) on **FIG. 19** considers known trends for progressing cancer. Population studies and other sources are analyzed to predict the time patterns for progressing cancer of PSA Velocity and PSA, Free PSA Velocity and Free PSA, and Free PSA Velocity % and Free PSA %. In addition, the biologic uncertainty in these time patterns is estimated from population studies and other sources.

**Uncertainty in Predicted Values for No Progressing Cancer**

The process for estimating the probability of long-term conditions like progressing cancer and volume growth depends on the total amount of uncertainty in the predicted PSA Velocity, Free PSA Velocity and Free PSA Velocity %. The system may estimate uncertainty in the predicted results using standard deviation in the predicted velocities.

Trend uncertainty and biologic uncertainty contribute to the total amount of uncertainty in PSA and Free PSA trends. Trend uncertainty is caused mostly by short-term biologic variation with some test measurement variation thrown in, module **1902** on **FIG. 19**. The other source of variation is long-term biologic uncertainty about progressing cancer. It reflects variation in PSA and Free PSA for men with similar types of progressing cancer. We use standard deviation to define and measure the amount of variation of each type.

Trend and biologic variation may move independently of each other, so we can't simply add them together to produce total variation. The table below shows total standard deviation for PSA Velocity for four trend standard deviations and one biologic standard deviation, assuming they are minimally correlated.

| | | | | |
|---|---|---|---|---|
| Trend Standard Deviation | 0.05 | 0.10 | 0.30 | 0.60 |
| Biologic Standard Deviation | 0.10 | 0.10 | 0.10 | 0.10 |
| Total Standard Deviation | 0.11 | 0.14 | 0.32 | 0.61 |

The results show that the total tends to be dominated by the larger standard deviation, which will usually be the trend standard deviation in the early stages of Dynamic Screening. Even when the component standard deviations are equal at 0.10, the total is only 0.14 rather than the simple sum of 0.20. This result reflects, in part, the independence of the two sources of variation.

Variation in Free PSA Velocity and Free PSA Velocity % trends may be handled in an analogous way to combine trend and biologic standard deviations.

### Alternative Hypothesis Generators for Progressing Cancer

Progressing cancer hypotheses for PSA and fPSA growth may be generated using the screening history of other men with progressing cancer, as shown in **FIG. 31.** The screening history focuses on the residual values of PSA and fPSA generated by progressing cancer alone without the contributions of non-cancerous prostate cells. PSA doubling times and fPSA Velocity % probabilities are variables used. A doubling time is selected and the exponential growth path for PSA is calculated for each hypothesis generated. A value for fPSA Velocity % is selected for each hypothesis generated. The growth path for fPSA is calculated by combining the fPSA Velocity % with the exponential growth in PSA. PSA timing and doubling times and fPSA Velocity % are varied as part of the iterative error minimization process.

### Early Warning

The Early Warning of progressing cancer module (**1918**) on **FIG. 19** estimates the number of years of early, or late, warning based on the trends for an individual man. Residual PSA Velocity is the preferred trend to use but the PSA Velocity trend or even the PSA trend may be analyzed in conjunction with related Free PSA variables and their ratios with PSA variables. Residual PSA Velocity may be compared with prostate cancer trends that relate PSA Velocity from progressing cancer to the number of years of early warning, allowing Residual PSA Velocity to be translated into an equivalent number of years of early warning. Years of early warning refers to the number of years before the Transition Point when the Cure Ratio begins to decline steeply over time.

### Prior Probabilities

The Prior Probabilities module (**1908**) on **FIG. 19** uses population data, the man's risk factors and his screening history to estimate the probability of undetected early warning. Inputs may include risk factors for the individual being screened and his individual history of screening. More detailed steps of the Prior Probabilities module are shown on **FIG. 32**.

We define the Transition Point as the year in which cancer has progressed enough to begin causing a steep decline in the Cure Ratio. Early warning is defined as detection before the Transition Point. It is measured in years before the Transition Point. Late warning is defined as detection after the Transition Point.

Risk adjusted means that the risk for an average man has been adjusted up or down by the Risk Ratio entered in the Personal Profile for a specific man. Undetected refers to the probability of cancer that has not already been detected. For cancer with eight years of early warning probability of previous detection is low after many years of Dynamic Screening, so the undetected probability of that early cancer is relatively high. In contrast, for cancer with three years of late warning the probability of previous detection is high, so the undetected probability of that late cancer is very close to zero.

### Risk Adjusted Incidence

The Risk Adjusted Incidence module (**3200**) on **FIG. 32** may estimate the probability of progressing cancer for a range of years of early (or late) warning based on individual risk factors. Men throughout the world may have higher or lower risk than the average man in the United States. Users may input in their personal *Profile* their personal Risk Factors or their estimate of their Risk Ratio. Background and guidance for choosing a Risk Ratio is provided there. Factors that appear to affect the risk of prostate cancer include: Family history; Race - Black is at risk, possibly because of lack of vitamin D; Diet - Asian is better than American with lots of beef; and Latitude of home that affects sunlight creation of vitamin D.

The Risk Ratio may scale the Average Annual Risk using the following formula:

$$\text{Risk Adjusted Annual Risk (age)} = \text{Risk Ratio x Average Annual Risk (age)}$$

Average Annual Risk is the annual risk for a man of a given age in the reference population, such as all U.S. men. The Risk Ratio may be entered by the user or estimated by the module based on risk factors entered by the user.

**Probability of Early Warning**

The Probability of Early Warning module is shown as (**3202**) on **FIG. 32**. The module may consider the probability of progressing cancer for each year of early and late warning for him at his current age. Consider a man age 60. At his current age 60, the age 59 Risk Adjusted Incidence of progressing cancer will be one year late (+1). In the same way his age 58 cancer will be two years late (+2) at his current age 60. In the opposite direction, at age 61 his annual risk at the Transition Point will be one year early at his current age 60. In the same way his age 62 cancer will be two years early (-2) at his current age 60. The table below shows the mapping.

| Age | Years Before/After |
|---|---|
| 58 | +2 |
| 59 | +1 |
| 60 | 0 |
| 61 | -1 |
| 62 | -2 |

One possible equation for the mapping is:

$$\text{Years Before/After the Transition} = \text{Current Age} - \text{Age}$$

**Probability of Past Detection**

The Probability of Past Detection module is shown as (**3204**) on **FIG. 32**. The longer a man uses Dynamic Screening the more early warning of progressing cancer he is likely to get. Past Dynamic Screening increases the chance that more advanced cancer will already have been detected; and, therefore, is no longer a likely possibility.

The probability of detection increases with later warning. In the extreme, metastasis and death unambiguously confirm the detection of prostate cancer. Symptoms typically show up at about three or four years of late warning, so much of this cancer will be detected in men who are not screened. Current PSA screening is hit or miss, but tends to detect cancer in a range around the Transition Point (year 0). These situations are taken into account by the module when it estimates the probability of past detection as a function of early warning.

Two inputs are used for the most basic estimates for past Dynamic Screening: Years of PSA Dynamic Screening and Years of Free PSA Dynamic Screening. Longer periods of testing lead to earlier warning of progressing cancer. There can be a matrix of possible past detection vectors based on these two dimensions. The probability of past detection varies greatly depending on the type and duration of screening.

Some men may continue Dynamic Screening after the apparent detection of progressing cancer in order to be sure that cancer is progressing. This situation requires special handling to reflect possible detection that has not been acted on.

**Probabilities of Undetected Early Warning**

The Probability of Undetected Early Warning module is shown as (**3206**) on **FIG. 32**. The probability of undetected early warning is a function of the probability of early warning (**3202**) and the probability of past detection (**3204**). One possible equation is:

$$\text{Probability of Undetected Early Warning (years before/after Transition Point)} = \text{Probability of Early Warning (years)} \times (1 - \text{Probability of Past Detection (years)})$$

## Long-Term Probabilities

The Long-Term Probabilities module (**1916**) on **FIG. 19** estimates the probabilities of one or more long-term conditions, such as progressing cancer or prostate volume growth. **FIG. 33** shows an example of the high level inputs and outputs for estimating the probability of progressing cancer. Prior probabilities are the starting point and come from module **1908** on **FIG. 19**. Trend residual velocities come from module **1912** on **FIG. 19**. Velocities and trends may be used in other embodiments. The Long-Term Probabilities module on **FIG. 33** adjusts the prior probabilities of progressing cancer based on how the trend residual velocities compare with patterns for progressing cancer and the predicted values for no cancer. A variety of methods can be used to estimate the probability, including Bayesian and simulation methods. The process is complicated because a variety of cancer stages are possible, characterized by years of early warning. Therefore, the module may consider a range of progressing cancer possibilities (different years of early warning) and a no-cancer (not present or not progressing) possibility defined by the no-cancer predicted values. For each of these possibilities a probability distribution may be constructed that may be characterized by a mean and by variation, which may be characterized by standard deviations. There are two sources of variation that may be considered. First, trend variation may be caused by possibly random variation in test results. Second, biologic variation may be caused by differences among men or for a specific man over time.

## Example Flow Chart for Direct Calculation of Probabilities

A high level flow chart of the direct calculation of the probability of progressing cancer is shown on **FIG. 34**. The direct calculation may be based on Bayesian methods and contrasts with iterative methods.

## Probability Estimate for Iterative Calculation of Probabilities

An iterative process may be used to calculate the probability of progressing cancer. Estimates of the probability distributions of the components that comprise actual and predicted PSA and Free PSA are used to generate probability distributions used in the method. The method is shown by the flow chart on **FIG. 35**. The process iteratively selects predictions and hypotheses, calculates their probabilities and then through a series of steps calculates the joint probability of the resulting prediction. At the end of the iterative process the probability of progressing cancer is calculated.

The screening history of all men who have provided data is combined with the screening history of the man making prostate cancer decisions. Variables considered include: No Cancer and Cancer PSA and fPSA Trends, including: Average PSA and fPSA% trends, PSA Velocity and fPSA Velocity % trends, and Residual PSA and fPSA Velocity % trends. Probabilities are associated with various combinations of PSA doubling time and fPSA Velocity % from cancer based on the experience of men with progressing cancer. The probability distributions of the components of predicted PSA and Free PSA are estimated - volume measurement error and velocity density prediction errors are example factors that can cause PSA to vary. The error distributions are run through a prediction simulator to translate the input error distributions into an overall probability distribution for predicted PSA and Free PSA for no progressing cancer. Predictions of PSA and fPSA are calculated by adding the progressing cancer hypothesis to the no cancer prediction. The joint probability for each prediction is calculated from the probabilities for each progressing cancer hypothesis and no cancer prediction. PSA and

Free PSA values and prediction errors are plotted vs time. Free PSA is plotted vs residual PSA for their values and prediction errors. The joint probability that the actual results are explained by the predictions is calculated using a variety of methods, including Bayesian inference. The probability of progressing cancer is estimated after many iterations of hypotheses and predictions based on the probabilities of scenarios with progressing cancer.

## Probability Estimate Using Confirming Tests

The system will recognize early warning of possible cancer progression and suggest additional confirmation tests. Confirmation tests may include other components of PSA such as Pro PSA and any other useful new markers developed in the future. In addition, a new prostate volume study may be suggested, perhaps using more expensive technology if rapid prostate enlargement is a factor. A second round of confirmation tests will be suggested - perhaps six months after the first. Additional confirmation tests will be suggested until progression has been confirmed or rejected. The initial drop in Free PSA % ratios may be an accident, but a continued drop accompanied by shifts in ratios for other tests for can confirm a high probability of progressing cancer. Recall the adage that once may be an accident, twice a coincidence but three times is enemy action - with prostate cancer as the enemy in this case.

A possible confirming method is shown on the flow chart on **FIG. 36**. The process iteratively selects predictions and hypotheses, calculates their probabilities and then through a series of steps calculates to joint probability of the resulting prediction. At the end the iterative process the probability of progressing cancer is calculated from the scenarios that include it.

The screening history of all men who have provided data is combined with the screening history of the man making prostate cancer decisions. Variables considered include: No Cancer and Cancer fPSA, xPSA and PSA Trends, including: Average fPSA%/xPSA% trends, fPSA/xPSA Velocity % trends, and Residual fPSA/xPSA Velocity % trends. Probabilities are associated with various combinations of PSA doubling time and fPSA and xPSA Velocity %s from cancer based on the experience of men with progressing cancer. Bayesian inference may be used to estimate the probability distributions of the components of predicted PSA, fPSA and xPSA - volume measurement error and velocity density prediction errors are example factors that can cause PSA to vary. The error distributions are run through a prediction simulator to translate the input error distributions into an overall probability distribution for predicted PSA, fPSA and xPSA for no progressing cancer. Predictions of PSA, fPSA and xPSA are calculated by adding the progressing cancer hypothesis to the no cancer prediction. The joint probability for each prediction is calculated from the probabilities for each progressing cancer hypothesis and no cancer prediction. PSA, fPSA and xPSA values and prediction errors are plotted vs time. Residual fPSA and xPSA are plotted vs residual PSA for their values and prediction errors. The joint probability that the actual results are explained by the predictions may be calculated using Bayesian methods. The probability of progressing cancer is estimated after many iterations of hypotheses and predictions based on the probabilities of scenarios with progressing cancer.

**Personalized Probabilities for Use in Integrated Health Systems and Methods**

A system to perform the Bayes calculation of the probability of progressing cancer can be configured with the following components: prior probabilities of cancer at various stages of progression; probability of the observation of various biomarker trends conditional on no progressing cancer; and probability of the observation of various biomarker trends conditional on cancer at various stages of progression.

A system can be configured for generating one or both of the last two categories of probabilities for an individual man with specific observed biomarker trends and corresponding measurement uncertainty in those trends.

Consider a man concerned about prostate cancer with a series of PSA and Free PSA biomarker results from blood tests. Trends can be estimated for each biomarker and analyzed using methods previously disclosed. The results might be:

- Trend PSA 3.0 Standard Deviation 0.4
- Trend PSA Velocity 0.40 Standard Deviation 0.20
- Trend Free PSA % 17.0% Standard Deviation 2.0%
- Trend Free PSA Velocity % 6.0% Standard Deviation 3.0% where, Trend PSA Velocity is the annual rate of change in Trend PSA; Trend Free PSA % is Trend Free PSA divided by Trend PSA; and Trend Free PSA Velocity % is Trend Free PSA Velocity divided by Trend PSA Velocity.

Other information about the man may be available, including age, measurement of prostate volume in some cases, and other factors that may affect the conditional probabilities.

Typically, no highly specific conditional distributions can be estimated directly from available population data. The disclosed method calculates the needed personalized probabilities.

In an embodiment, the method starts by creating personalized biologic probability models of: (a) no cancer conditions of the prostate: healthy and volume growth; (b) cancer at various stages of progression; and (c) combined models of no cancer conditions and various stages of cancer progression.

Those models are then combined with trend uncertainty models to create an overall multi-dimensional distribution or part of the distribution relevant to the specific trend results. The distributions are multi-dimensional in that trend values and trend velocities, or annual rates of change, are considered for at least one biomarker, such as PSA. The disclosed example describes a method for creating four dimensional distributions and probabilities for two biomarkers: PSA and Free PSA. Higher dimensional distributions and probabilities will be needed when additional biomarkers are considered.

Monte Carlo methods are used to create four dimensional probability distributions for PSA, PSAV, fPSA% and fPSAV% from random draws from the probability distributions of the underlying biologic and trend uncertainty models.

The calculation process can be time consuming and slow the response for online users. The complexity and time of calculation can increase exponentially as additional biomarkers become available and are incorporated into the method. Therefore, efficient methods of calculating the probabilities can be beneficial.

The disclosed method focuses on the probabilities of the observed trend values rather than very much larger four dimensional probability distributions for PSA, PSAV, fPSA% and fPSAV% for the full range of possible outcomes. This approach reduces the amount of calculations necessary to calculate the personalized probabilities needed for the Bayes calculations. The reduction is achieved in practice using a hierarchical triage approach that aborts a Monte Carlo iteration as soon as one of the values falls outside the target range for first PSA, then PSAV, then fPSA% and finally fPSAV%.

The Dynamic Screening System helps men and their doctors screen for progressing cancer, long-term conditions and short-term conditions. It provides early warning of progressing cancer while reducing the probability of unnecessary treatment and side effects. The results are useful inputs to the optimal Treatment Timing System. The prostate is the organ of the body used in the examples. Conditions used as examples are progressing prostate cancer, prostate volume growth caused by Benign Prostatic Hyperplasia (BPH) and infections of the prostate. Both PSA and Free PSA tests can be used for screening. Other tests may supplement them or replace them.

The approaches described herein can be used as an alternative method for creating the long-term probabilities, as shown on **FIG. 37**. The long-term probabilities module is split into a personalized probability distributions module and probabilities module and a Bayes long-term probabilities module. The Bayes calculations in the second module are discussed in the above incorporated references. The first module is discussed below. The outputs of module are probabilities of the observed trend results: PSA, PSAV, fPSA% and fPSAV% conditional on no cancer and cancer for various years (X). These are created using personal information and input from biologic and trend models, as disclosed below.

The personalized probability distributions & probabilities module uses a four dimensional frequency generator, shown on **FIG. 38**, which calculates personalized probability distributions and probabilities for cancer and no cancer cases in iterative fashion. Each iteration is initiated by the Monte Carlo iteration controller (**3899**) and ended by the Monte Carlo iteration completion module (**3898**), which returns control to the controller (**3899**). For each iteration, trend values for a healthy prostate are generated from probability distributions in module **3800**. Trend values for prostate volume growth are generated from probability distributions in module **3820**. No cancer values are calculated in module **3821** as the sum of values from module **3800** and module **3820**. In module **3822** the values for each iteration are added to the appropriate four dimensional bucket defined by ranges in four dimensions. As the number of iterations increase, frequency distributions for the no cancer case are built up in module **3822** and output at the end of the process. For each iteration, trend values for each year X cancer case are generated from probability distributions in module **3840**. A range of cases are calculated for year X cancers, where X is a measure of cancer progression. Values for each year X cancer plus no cancer case are calculated in module **3841** as the sum of values from module **3840** and module **3821**. In module **3842** the values for each iteration are added to the appropriate four dimensional bucket defined by ranges in four dimensions. As the number of iterations increase, frequency distributions for each year X cancer plus no cancer case are built up in module **3842** and output at the end of the process.

At times, it is computationally more efficient to use independent Monte Carlo processes for the no cancer case and cancer plus no cancer cases. The four dimensional frequency generator, shown on **FIG. 39**, calculates personalized probability distributions and probabilities for the no cancer case in iterative fashion. Each iteration is initiated by the Monte Carlo iteration controller (**3999**) and ended by the Monte Carlo iteration completion module (3998), which returns control to the controller (**3999**). For each iteration, trend values for a healthy prostate are generated from probability distributions in module **3900**. Trend values for prostate volume growth are generated from probability distributions in module **3920**. No cancer values are calculated in module **3921** as the sum of values from module **3900** and module **3920**. In module 3922 the values for each iteration are added to the appropriate four dimensional bucket defined by ranges in four dimensions. As the number of iterations increase, frequency distributions for the no cancer case are built up in module **3922**.

The four dimensional frequency generator, shown on **FIG. 40**, calculates personalized probability distributions and probabilities for cancer plus no cancer cases in iterative fashion. Each iteration is initiated by the Monte Carlo iteration controller (**4099**) and ended by the Monte Carlo iteration completion module (**4098**), which returns control to the controller (**4099**). For each iteration, trend values for a healthy prostate are generated from probability distributions in module **4000**. Trend values for prostate volume growth are generated from probability distributions in module **4020**. No cancer values are calculated in module **4021** as the sum of values from module **4000** and module **4020**. For each iteration, trend values for each year X cancer case are generated from probability distributions in module **4040**. A range of cases are calculated for year X cancers, where X is a measure of cancer progression. Values for each year X cancer plus no cancer case are calculated in module **4041** as the sum of values from module **4040** and module **4021.**

In module **4042** the values for each iteration are added to the appropriate four dimensional bucket defined by ranges in four dimensions. As the number of iterations increase, frequency distributions for each year X cancer plus no cancer case are built up in module **4042**.

**FIG. 41** shows the Monte Carlo process in modules **3800, 3900** and **4000** for generating outcomes for a healthy prostate from a number of probability distributions. Each iteration is initiated by the Monte Carlo iteration controller (**4199**). A volume for a healthy prostate is drawn from a probability distribution in module **4100**. The nature and values of the distribution are affected by data from the personal profile. For example, age may influence the distribution and a volume measurement will strongly influence the distribution. Volume velocity for a healthy prostate is drawn from a probability distribution in module **4110**. The nature and values of the distribution are affected by the volume drawn in module **4100** and data from the personal profile. For example, the mean and standard deviation for the volume velocity distribution tend to be larger for larger volumes. PSA density for a healthy prostate is drawn from a probability distribution in module **4101**.

The nature and values of the distribution are affected by data from the personal profile. For example, age may influence the distribution; and past PSA and volume measurements may strongly influence the distribution if they are available. The flow chart implicitly assumes that the PSA density of new healthy prostate tissue has the same PSA density as old healthy prostate tissue. If future research indicates they are different then a second healthy PSAV density module would be used with number **4111**. A value for biologic PSA is calculated in module **4102** as the product of the healthy PSA density from module **4101** and the volume of a healthy prostate from module **4100**. In a similar way, a value for biologic PSAV is calculated in module **4112** as the product of the healthy PSA density from module **4101** and the volume velocity of a healthy prostate from module **4110**. Trend PSA (**4103**) is the PSA trend multiplier (**4104**) multiplied by biologic PSA (**4102**), and trend PSAV (**4113**) is the PSAV trend multiplier (**4114**) multiplied by biologic PSAV (**4112**). Trend variables add trend variation to biologic outcomes in order to simulate uncertainty in observed trend results. Trend multipliers (**4104** and **4114**) typically have a mean of 1.0 and standard deviations equal to the coefficients of variation (CV) for each of the estimated trends (where CV = SD/Mean). The CVs are obtained from analysis of PSA trends. A value for biologic Free PSA is calculated in module **4106** as the product of the healthy Free PSA % from module **4105** and biologic PSA from module **4102**. In a similar way, a value for biologic Free PSAV is calculated in module **4116** as the product of the healthy Free PSA % from module **4105** and biologic PSAV from module **4112**. The Free PSA % for a healthy prostate is drawn from a probability distribution in module **4105**. The nature and values of the distribution are affected by data from the personal profile. For example, age may influence the distribution; and past PSA and

Free PSA results will strongly influence the distribution if they are available. The flow chart implicitly assumes that the Free PSA % of new healthy prostate tissue has the same Free PSA % as old healthy prostate tissue. If future research indicates they are different then a second healthy Free PSAV % module would be used with number **4115**. A value for biologic Free PSA % is calculated in module **4107** as healthy biologic Free PSA from module **4106** divided by biologic PSA from module **4102**. In a similar way, a value for biologic Free PSAV % is calculated in module **4117** as healthy Free PSAV from module **4116** divided by biologic PSAV from module **4112**. Trend Free PSA % (**4108**) is the Free PSA % trend multiplier (**4109**) multiplied by biologic Free PSA % (**4107**), and trend Free PSAV % (**4118**) is the Free PSAV % trend multiplier (**4119**) multiplied by biologic Free PSAV % (**4117**). Trend variables add trend variation to biologic outcomes in order to simulate observed trend results. Trend multipliers (4109 and 4119) typically have a mean of 1.0 and standard deviations equal to the coefficients of variation (CV) for each of the estimated trends (where CV = SD/Mean). The CVs are obtained from analysis of Free PSA and PSA trends. There are four outputs of this module, shown by the thick black arrows: trend PSA (**4103**), trend PSAV (**4113**), trend Free PSA % (**4108**) and trend Free PSAV % (**4118**).

**FIG. 42** shows the Monte Carlo process in modules **3800, 3900** and **4000** for generating outcomes for prostate volume growth from a number of probability distributions. Each iteration is initiated by the Monte Carlo iteration controller (**4299**). A volume for volume growth is drawn from a probability distribution in module **4220**. The nature and values of the distribution are affected by data from the personal profile. For example, age may influence the distribution and a volume measurement will strongly influence the distribution. Volume velocity for volume growth is drawn from a probability distribution in module **4230**. The nature and values of the distribution are affected by the healthy volume drawn in module **4100**, the volume for volume growth drawn in module **4220** and data from the personal profile. For example, the mean and standard deviation for the volume velocity distribution tend to be larger for larger volumes. PSA density for volume growth is drawn from a probability distribution in module **4221**. The nature and values of the distribution are affected by healthy PSA density (**4101**) and data from the personal profile. For example, age may influence the distribution; and past PSA and volume measurements may strongly influence the distribution if they are available. The flow chart implicitly assumes that the PSA density of new volume growth has the same PSA density as old volume growth. If future research indicates they are different then a second volume growth PSAV density module would be used with number **4231**. A value for biologic PSA is calculated in module **4222** as the product of the volume growth PSA density from module **4221** and the volume of volume growth from module **4220**. In a similar way, a value for biologic PSAV is calculated in module **4232** as the product of volume growth PSA density from module **4221** and the volume velocity for volume growth from module **4230**. Trend PSA (**4223**) is the PSA trend multiplier (**4104**) multiplied by biologic PSA (**4222**), and trend PSAV (**4233**) is the PSAV trend multiplier (**4114**) multiplied by biologic PSAV (**4232**). A value for biologic Free PSA is calculated in module **4226** as the product of the volume growth Free PSA % from module **4225** and biologic PSA from module **4222**. In a similar way, a value for biologic Free PSAV is calculated in module **4236** as the product of the volume growth Free PSA % from module **4225** and biologic PSAV from module **4222**. The Free PSA % for volume growth is drawn from a probability distribution in module **4225**. The nature and values of the distribution are affected by the healthy Free PSA % (**4105**) and data from the personal profile. For example, age may influence the distribution; and past PSA and

Free PSA results will strongly influence the distribution if they are available. The flow chart implicitly assumes that the Free PSA % for volume growth has the same Free PSA % as old volume growth. If future research indicates they are different then a second volume growth Free PSAV % module would be used with number **4235**. A value for biologic Free PSA % is calculated in module **4227** as volume growth biologic Free PSA from module **4226** divided by biologic PSA from module **4222**. In a similar way, a value for biologic Free PSAV % is calculated in module **4236** as volume growth Free PSAV from module **4236** divided by biologic PSAV from module **4232**. Trend Free PSA % (**4228**) is the Free PSA % trend multiplier (**4109**) multiplied by biologic Free PSA % (**4227**), and trend Free PSAV % (**4238**) is the Free PSAV % trend multiplier (**4119**) multiplied by biologic Free PSAV % (**4237**). There are four outputs of this module, shown by the thick black arrows: trend PSA (**4223**), trend PSAV (**4233**), trend Free PSA % (**4228**) and trend Free PSAV % (**4238**).

**FIG. 43** shows the Monte Carlo process in modules **3800, 3900** and **4000** for generating outcomes for year X cancer from a number of probability distributions, where X is a measure of cancer progression. For example, X can be measured as the number of years before or after the Transition Point, defined as the time of progression when the cure rate begins to decline steeply. Other reference points for measuring X may work as well. In this example, fifteen year X cases might be considered:

- 2 Years After the Transition Point
- 1 Year After the Transition Point
- 0 Years = At the Transition Point
- 1 Year Before the Transition Point
- 2 Years Before the Transition Point
- 3 Years Before the Transition Point
- 4 Years Before the Transition Point
- 5 Years Before the Transition Point
- 6 Years Before the Transition Point
- 7 Years Before the Transition Point
- 8 Years Before the Transition Point
- 9 Years Before the Transition Point
- 10 Years Before the Transition Point
- 11 Years Before the Transition Point
- 12 Years Before the Transition Point

In this example, there will be fifteen parallel versions of **FIG. 43 -** one for each year X case.

Each iteration is initiated by the Monte Carlo iteration controller (**4399**). A volume for year X cancer is drawn from a probability distribution in module **4340**. The nature and values of the distribution may be affected by data from the personal profile. For example, age may influence the distribution. Volume velocity for year X cancer is drawn from a probability distribution in module **4350**. The nature and values of the distribution are affected by the volume for cancer X drawn in module **4340** and data from the personal profile. For example, the mean and standard deviation for the volume velocity distribution tend to be larger for larger volumes. PSA density for year X cancer is drawn from a probability distribution in module **4341**. The nature and values of the distribution may be affected by data from the personal profile. For example, age may influence the distribution; and past PSA and volume measurements may strongly influence the distribution if they are available. The flow chart implicitly assumes that the PSA density of new year X cancer has the same PSA density as old year X cancer. If future research indicates they are different then a second year X cancer PSAV density module would be used with number **4351**. A value for biologic PSA is calculated in module **4342** as the product of the year X cancer PSA density from module **4341** and the volume of year X cancer from module **4340**. In a similar way, a value for biologic PSAV is calculated in module **4352** as the product of year X cancer PSA density from module **4341** and the volume velocity for year X cancer from module **4350**. Trend PSA (**4343**) is the PSA trend multiplier (**4104**) multiplied by biologic PSA (**4342**), and trend PSAV (**4353**) is the PSAV trend multiplier (**4114**) multiplied by biologic PSAV (**4352**). Trend variables add trend variation to biologic outcomes in order to simulate observed trend results. Trend multipliers (**4104** and **4114**) typically have a mean of 1.0 and standard deviations equal to the coefficients of variation (CV) for each of the estimated trends (where CV = SD/Mean). The CVs are obtained from analysis of PSA trends. A value for biologic Free PSA is calculated in module **4346** as the product of the year X cancer Free PSA % from module **4345** and biologic PSA from module **4342**. In a similar way, a value for biologic Free PSAV is calculated in module **4356** as the product of the year X cancer Free PSA % from module **4345** and biologic PSAV from module **4352**. The Free PSA % for year X cancer is drawn from a probability distribution in module **4345**. The nature and values of the distribution may be affected by data from the personal profile. For example, age may influence the distribution; and past PSA and Free PSA results may strongly influence the distribution if they are available. The flow chart implicitly assumes that the Free PSA % for year X cancer has the same Free PSA % as old year X cancer. If future research indicates they are different then a second year X cancer Free PSAV % module would be used with number **4355**. A value for biologic Free PSA % is calculated in module **4347** as year X cancer biologic Free PSA from module **4346** divided by biologic PSA from module **4342**. In a similar way, a value for biologic Free PSAV % is calculated in module **4357** as year X cancer Free PSAV from module **4356** divided by biologic PSAV from module **4352.** Trend Free PSA % (**4348**) is the Free PSA % trend multiplier (**4109**) multiplied by biologic Free PSA % (**4347**), and trend Free PSAV % (**4358**) is the Free PSAV % trend multiplier (**4119**) multiplied by biologic Free PSAV % (**4357**). Trend variables add trend variation to biologic outcomes in order to simulate observed trend results. Trend multipliers (**4109** and **4119**) typically have a mean of 1.0 and standard deviations equal to the coefficients of variation (CV) for each of the estimated trends (where CV = SD/Mean). The CVs are obtained from analysis of Free PSA and PSA trends. There are four outputs of this module, shown by the thick black arrows: trend PSA (**4343**), trend PSAV (**4353**), trend Free PSA % (**4348**) and trend Free PSAV % (**4358**).

The approach described in this example generates extensive four dimensional distributions that can be used to find the probabilities needed for the Bayes calculations of the probability of progressing cancer. However, the calculations can be time consuming and cause delays in real time responses to users. The approach of focused probabilities is discussed below to address this if it is an issue for a situation at hand. The number of calculations and the time to perform them can be reduced substantially by focusing narrowly on the probabilities needed for the Bayes calculations rather than on generating extensive four dimensional distributions. Additional discussion of methods for focusing on the needed probabilities is provided below.

For one biomarker, such as PSA, we are interested in two dimensions: PSA and

PSA Velocity (PSAV). We can create a two dimensional rectangle of possible Monte Carlo results by dividing each dimension into segments. **FIG. 44** shows the 100 possible buckets of possible results when each dimension is divided into ten segments.

The segments for each of the two dimensions might be:

- Ten segments for the PSA dimension:

    o >= 0 and < 1
    o >= 1 and < 2
    o >= 2 and < 3
    o >= 3 and < 4
    o >= 4 and < 5
    o >= 5 and < 6
    o >= 6 and < 7
    o >= 7 and < 8
    o >= 8 and < 9
    o >= 9

- Ten segments for the PSAV dimension:

    o >= 0.0 and < 0.1
    o >= 0.1 and < 0.2
    o >= 0.2 and < 0.3
    o >= 0.3 and < 0.4
    o >= 0.4 and < 0.5

o >= 0.5 and < 0.6
o >= 0.6 and < 0.7
o >= 0.7 and < 0.8
o >= 0.8 and < 0.9
o >= 0.9

For two tests, such as PSA and Free PSA, we are interested in four dimensions: PSA, PSAV, fPSA% and fPSAV%. We can create a four dimensional hyper cube of possible Monte Carlo results by dividing each dimension into segments. **FIG. 45** suggests the 10,000 possible buckets of possible results when each dimension is divided into ten segments (even though we can depict only three of the four dimensions).

The number of Monte Carlo iterations required to create a reasonably stable distribution increases exponentially with the number of tests, as shown by the table of **FIG. 46**. The first column shows the number of biomarker tests considered. Some drug testing companies that a panel of tests will become standard screening practice in the future for conditions such as prostate cancer. The second column shows the corresponding number of dimensions when both trend values and velocities are considered for each test. The third column shows the ten segments we are assuming for each dimension. The fourth column shows the corresponding number of buckets for which frequencies are collected to create the overall multi-dimensional probability distributions. The fifth column shows the 100 average frequency collected in each bucket. An average frequency of this magnitude is needed to assure at least a few results collected in the buckets on the low frequency tails of the distributions.

The table of **FIG. 46** suggests that a trillion Monte Carlo iterations would be required for each case if a panel of five screening tests become the standard for a condition such as prostate cancer. This creates an enormous problem for an online service the offers real time analysis. The delays would be unacceptable using the fastest computers available now or in the near future.

The Monte Carlo calculations for one personalized case requires the frequency for only one bucket rather than the frequencies for all possible buckets.

Consider a man concerned about prostate cancer with a series of PSA biomarker results from blood tests. Trends can be estimated for each biomarker and analyzed using methods previously disclosed. The results might be:

- Trend PSA 3.0 Standard Deviation 0.4
- Trend PSA Velocity 0.40 Standard Deviation 0.20

The bucket used to collect the frequency of this outcome might be:

- PSA = 3.0 +/- 0.5 or PSA >2.5 and <3.5
- PSAV = 0.4 +/- 0.05 or PSAV >0.35 and <0.45

The gray rectangle on the table of **FIG. 47** shows conceptually the bucket of concern defined by the range of PSA and PSAV results around the observed trend results. For one case, we are not interested in any of the other buckets that are not shaded.

Now consider a man concerned about prostate cancer with a series of PSA and

Free PSA biomarker results from blood tests. Trends can be estimated for each biomarker and analyzed using methods previously disclosed. The results might be:

- Trend PSA 3.0 Standard Deviation 0.4
- Trend PSA Velocity 0.40 Standard Deviation 0.20
- Trend Free PSA % 17.0% Standard Deviation 2.0%
- Trend Free PSA Velocity % 6.0% Standard Deviation 3.0%

The bucket used to collect the frequency of this outcome might be:

- PSA = 3.0 +/- 0.5 or PSA >2.5 and <3.5
- PSAV = 0.4 +/- 0.05 or PSAV >0.35 and <0.45
- fPSA% = 17.0% +/- 2.0% or fPSA% >15.0% and <19.0%
- fPSAV% = 6.0% +/- 2.0% or fPSAV% >4.0% and <8.0%

The small cube inside the large cube shown by **FIG. 48** suggests conceptually the hypercube bucket of concern defined by the range of PSA, PSAV, fPSA% and fPSAV% results around the observed trend results (even though we can depict only three of the four dimensions). For one case, we are not interested in any of the other buckets that are outside the small cube.

In general for a single case we know trend values for PSA, PSAV, fPSA% and fPSAV%, which is a point in the 4D hyper cube. We can create a small hyper cube bucket around the point to collect Monte Carlo results that fall within the ranges. We will use the frequency of the results in the bucket to estimate the probability of the results.

- Trend PSA +/- PSA Range Delta
- Trend PSAV +/- PSAV Range Delta
- Trend fPSA% +/- fPSA% Range Delta
- Trend fPSAV% +/- fPSAV% Range Delta

Monte Carlo results that fall with the bucket, like the solid dot in the small cube of **FIG. 48**, are recorded; and results that fall outside the bucket, like the circle in the large cube of **FIG. 48**, are not recorded.

Detailed methods for reducing the number of calculations by focusing on the bucket of concern are disclosed below. They are elaborations of the approach shown on **FIG. 49**. The Monte Carlo processes disclosed above are reorganized to calculate one dimension at a time, in this case:

- PSA in module **4900.**
- PSAV in module **4902.**
- fPSA% in module **4904.**
- fPSAV% in module **4906.**

For each iteration controlled by module **4999**, PSA is calculated in module **4900** using Monte Carlo methods. As controlled by module **4901**, the process stops for this iteration if PSA falls outside of the target range of the bucket, but the process continues if PSA falls within the target range of the bucket. If the iteration continues, PSAV is calculated in module **4902** using Monte Carlo methods. As controlled by module **4903**, the process stops for this iteration if PSAV falls outside of the target range of the bucket, but the process continues if PSAV falls within the target range of the bucket. If the iteration continues, fPSA% is calculated in module **4904** using Monte Carlo methods. As controlled by module **4905**, the process stops for this iteration if fPSA% falls outside of the target range of the bucket, but the process continues if fPSA% falls within the target range of the bucket. If the iteration continues, fPSAV% is calculated in module **4906** using Monte Carlo methods. As controlled by module **4907**, the process stops for this iteration if fPSAV% falls outside of the target range of the bucket, but the process continues if fPSAV% falls within the target range of the bucket.

We use the term sequential triage to describe stopping the iteration at each stage as soon we know that the result will miss the target bucket. Many unnecessary calculations can be avoided using this method.

Methods for efficiently calculating the no cancer probabilities needed for the Bayes calculations are disclosed below.

**FIG. 50** shows the four dimensional frequency generator for the no cancer case. Each iteration is initiated by the Monte Carlo iteration controller (**5099**). For each iteration, PSA is calculated in module **5000** using Monte Carlo methods. The process stops for this iteration if PSA falls outside of the target range of the bucket, but the process continues if PSA falls within the target range of the bucket. If the iteration continues, PSAV is calculated in module **5001** using Monte Carlo methods. The process stops for this iteration if PSAV falls outside of the target range of the bucket, but the process continues if PSAV falls within the target range of the bucket. If the iteration continues, fPSA% is calculated in module **5002** using Monte Carlo methods. The process stops for this iteration if fPSA% falls outside of the target range of the bucket, but the process continues if fPSA% falls within the target range of the bucket. If the iteration continues, fPSAV% is calculated in module **5003** using Monte Carlo methods. The process stops for this iteration if fPSAV% falls outside of the target range of the bucket, but the process continues if fPSAV% falls within the target range of the bucket. The four dimensional frequency collector (**5004**) keeps track of the number of Monte Carlo iterations started and the number of outcomes that fall in the 4D bucket. Frequency is calculated by dividing the number of outcomes in the bucket by the number of iterations started. Finally, control is passed to the Monte Carlo iteration completion module (5098).

**FIG. 51** shows the Monte Carlo process for generating no cancer PSA outcomes from a number of probability distributions. Each iteration is initiated by the Monte Carlo iteration controller (**5199**).

A volume for a healthy prostate is drawn from a probability distribution in module **4100**. The nature and values of the distribution are affected by data from the personal profile. For example, age may influence the distribution and a volume measurement will strongly influence the distribution. PSA density for a healthy prostate is drawn from a probability distribution in module **4101**. The nature and values of the distribution are affected by data from the personal profile. For example, age may influence the distribution; and past PSA and volume measurements may strongly influence the distribution if they are available. A value for healthy biologic PSA is calculated in module **4102** as the product of the healthy PSA density from module **4101** and the volume of a healthy prostate from module **4100**.

A volume for volume growth is drawn from a probability distribution in module **4220**. The nature and values of the distribution are affected by data from the personal profile. For example, age may influence the distribution and a volume measurement will strongly influence the distribution. PSA density for volume growth is drawn from a probability distribution in module **4221**. The nature and values of the distribution are affected by healthy PSA density (**4101**) and data from the personal profile. For example, age may influence the distribution; and past PSA and volume measurements may strongly influence the distribution if they are available. A value for volume growth biologic PSA is calculated in module **4222** as the product of the volume growth PSA density from module **4221** and the volume of volume growth from module **4220**.

No cancer biologic PSA is calculated in module **5102** as the sum of healthy biologic PSA from module **4102** and volume growth biologic PSA from module **4222**.

No cancer trend PSA (**5103**) is the previously drawn PSA trend multiplier (**4104**) multiplied by no cancer biologic PSA (**5102**). Trend multipliers (**4104**) typically have a mean of 1.0 and standard deviations equal to the coefficients of variation (CV) for each of the estimated trends (where CV = SD/Mean). The CVs are obtained from analysis of PSA trends.

Module **5197** evaluates whether the no cancer trend PSA from module **5103** is within the target range. If the decision is yes to continue the iteration for year X cancer the system may then proceed to module **5399** on **FIG. 53** to start the process for no cancer PSAV.

Trend PSA from module **5197** is the one output of this module, shown by the thick black arrow.

As suggested by **FIG. 52**, total calculation time can be reduced by constraining the range of values used to calculate PSA to the combinations of values (the not shaded area 5200) that are likely to result in trend PSA values that are within range of the target value checked in module **5197**. Healthy PSA falls within a relatively narrow range with high probability. Therefore, most of the variation in no cancer PSA is caused by volume growth. For a given target PSA range in module **5197**, there are combinations of low volume growth and/or low volume PSA density shown by shaded area **5201** that are very likely to result in no cancer trend PSA values (5103) that are less than the target range. These combinations of values need not used in the Monte Carlo process. In a similar way for a given target PSA range in module **5197**, there are combinations of high volume growth and/or high volume PSA density shown by shaded area **5202** that are very likely to result in no cancer trend PSA values (**5103**) that are greater than the target range. These combinations of values need not used in the Monte Carlo process. Only combinations of values in the not shaded area **5200** needed to be considered in the Monte Carlo process. However, the overall number of iterations considered needs to include the number of iterations that would have been generated for the shaded areas **5201** and **5202**, as well as for the not shaded area **5200**.

**FIG. 53** shows the Monte Carlo process for generating no cancer PSAV outcomes from a number of probability distributions. Each iteration is initiated by the Monte Carlo iteration controller (**5399**).

Volume velocity for a healthy prostate is drawn from a probability distribution in module **4110**. The nature and values of the distribution are affected by the volume previously drawn in module **4100** and data from the personal profile. For example, the mean and standard deviation for the volume velocity distribution tend to be larger for larger volumes. PSA density for a healthy prostate was previously drawn from a probability distribution in module **4101**. The flow chart implicitly assumes that the PSA density of new healthy prostate tissue has the same PSA density as old healthy prostate tissue. If future research indicates they are different then a second healthy PSAV density module would be used with number **4111**. A value for healthy biologic PSAV is calculated in module **4112** as the product of the healthy PSA density from module **4101** and the volume velocity of a healthy prostate from module **4110**.

Volume velocity for volume growth is drawn from a probability distribution in module **4230**. The nature and values of the distribution are affected by the healthy volume drawn in module **4100**, the volume for volume growth drawn in module **4220** and data from the personal profile. For example, the mean and standard deviation for the volume velocity distribution tend to be larger for larger volumes. PSA density for volume growth was previously drawn from a probability distribution in module **4221**. The flow chart implicitly assumes that the PSA density of new volume growth has the same PSA density as old volume growth. If future research indicates they are different then a second volume growth PSAV density module would be used with number **4231**. A value for volume growth biologic PSAV is calculated in module **4232** as the product of volume growth PSA density from module **4221** and the volume velocity for volume growth from module **4230**.

No cancer biologic PSAV is calculated in module **5312** as the sum of healthy biologic PSAV from module **4112** and volume growth biologic PSA from module **4232**.

No cancer trend PSAV (**5313**) is the previously drawn PSAV trend multiplier (**4114**) multiplied by no cancer biologic PSAV (**5312**). Trend multipliers (**4114**) typically have a mean of 1.0 and standard deviations equal to the coefficients of variation (CV) for each of the estimated trends (where CV = SD/Mean). The CVs are obtained from analysis of PSA trends.

Module 5397 evaluates whether the no cancer trend PSAV from module **5313** is within the target range. If the decision is yes to continue the iteration for year X cancer the system may then proceed to module **5499** on **FIG. 54** to start the process for no cancer Free PSA.

Trend PSAV from module **5397** is the one output of this module, shown by the thick black arrow.

**FIG. 54** shows the Monte Carlo process for generating no cancer Free PSA outcomes from a number of probability distributions. Each iteration is initiated by the Monte Carlo iteration controller (**5499**).

A value for healthy biologic Free PSA is calculated in module **4106** as the product of the healthy Free PSA % from module **4105** and biologic PSA from module **4102**. The Free PSA % for a healthy prostate is drawn from a probability distribution in module **4105**. The nature and values of the distribution are affected by data from the personal profile. For example, age may influence the distribution; and past PSA and Free PSA results will strongly influence the distribution if they are available.

A value for volume growth biologic Free PSA is calculated in module **4226** as the product of the volume growth Free PSA % from module **4225** and biologic PSA from module **4222**. The Free PSA % for volume growth is drawn from a probability distribution in module **4225**. The nature and values of the distribution are affected by the healthy Free PSA % (**4105**) and data from the personal profile.

For example, age may influence the distribution; and past PSA and Free PSA results will strongly influence the distribution if they are available.

No cancer biologic Free PSA is calculated in module **5406** as the sum of healthy biologic fPSA from module **4106** and volume growth biologic fPSA from module **4226**.

A value for no cancer biologic Free PSA % is calculated in module **5407** as biologic Free PSA from module **5406** divided by biologic PSA from module **5302**. Trend Free PSA % (**5408**) is the Free PSA % trend multiplier (**4109**) multiplied by biologic Free PSA % (**5407**). Trend variables add trend variation to biologic outcomes in order to simulate observed trend results. Trend multipliers (4109) typically have a mean of 1.0 and standard deviations equal to the coefficients of variation (CV) for each of the estimated trends (where CV = SD/Mean). The CVs are obtained from analysis of Free PSA and PSA trends.

Module **5497** evaluates whether the no cancer trend fPSA% from module **5408** is within the target range. If the decision is yes to continue the iteration the system may calculate trend fPSA in module **5490** and then proceed to module **5599** on **FIG. 55** to start the process for no cancer Free PSAV. Trend fPSA is calculated in module **5490** as trend fPSA% (**5497**) multiplied by trend PSA (**5103**).

Trend fPSA% (**5497**) and trend fPSA (**5490**) are the two outputs of this module, shown by the thick black arrows.

**FIG. 55** shows the Monte Carlo process for generating Free PSAV outcomes from a number of probability distributions. Each iteration is initiated by the Monte Carlo iteration controller (**5599**).

A value for healthy biologic Free PSAV is calculated in module **4116** as the product of the healthy Free PSA % previously drawn from module **4105** and previously calculated biologic PSAV from module **4112**. The flow chart implicitly assumes that the Free PSA % of new healthy prostate tissue has the same Free PSA % as old healthy prostate tissue. If future research indicates they are different then a second healthy Free PSAV % module would be used with number **4115** instead of module **4105**.

A value for volume growth biologic Free PSAV is calculated in module **4236** as the product of the volume growth Free PSA % previously drawn from module **4225** and previously calculated biologic PSAV from module **4232**. The flow chart implicitly assumes that the Free PSA % for volume growth has the same Free PSA % as old volume growth. If future research indicates they are different then a second volume growth Free PSAV % module would be used with number **4235** instead of module **4225**.

No cancer biologic Free PSAV is calculated in module **5516** as the sum of healthy biologic fPSAV from module **4116** and volume growth biologic fPSAV from module **4236**.

A value for no cancer biologic Free PSAV% is calculated in module **5557** as biologic Free PSAV from module **5516** divided by previously calculated biologic PSAV from module **5312**.

Trend Free PSA V% (**5558**) is the Free PSAV % trend multiplier (**4119**) multiplied by biologic Free PSAV % (**5557**). Trend variables add trend variation to biologic outcomes in order to simulate observed trend results. Trend multipliers (**4119**) typically have a mean of 1.0 and standard deviations equal to the coefficients of variation (CV) for each of the estimated trends (where CV = SD/Mean). The CVs are obtained from analysis of Free PSA and PSA trends.

Module **5597** evaluates whether the no cancer trend fPSAV% from module **5558** is within the target range. If the decision is yes to continue the iteration the system may calculate trend fPSAV in module **5590** and then return control to the iteration controller. Trend fPSAV is calculated in module **5590** as trend fPSAV% (**5597**) multiplied by previously calculated trend PSAV (**5313**).

Trend fPSA% (**5597**) and trend fPSA (**5590**) are the two value outputs of this module, shown by the thick black arrows.

Methods for efficiently calculating year X cancer probabilities needed for the Bayes calculations are disclosed below.

**FIG. 56** shows the four dimensional frequency generator for each year X cancer plus no cancer case. Each iteration is initiated by the Monte Carlo iteration controller (**5699**). For each iteration, PSA is calculated in module **5600** using Monte Carlo methods. The process stops for this iteration if PSA falls outside of the target range of the bucket, but the process continues if PSA falls within the target range of the bucket. If the iteration continues, PSAV is calculated in module **5601** using Monte Carlo methods. The process stops for this iteration if PSAV falls outside of the target range of the bucket, but the process continues if PSAV falls within the target range of the bucket. If the iteration continues, fPSA% is calculated in module **5602** using Monte Carlo methods. The process stops for this iteration if fPSA% falls outside of the target range of the bucket, but the process continues if fPSA% falls within the target range of the bucket. If the iteration continues, fPSAV% is calculated in module **5603** using Monte Carlo methods. The process stops for this iteration if fPSAV% falls outside of the target range of the bucket, but the process continues if fPSAV% falls within the target range of the bucket. The four dimensional frequency collector (**5604**) keeps track of the number of Monte Carlo iterations started and the number of outcomes that fall in the 4D bucket. Frequency is calculated by dividing the number of outcomes in the bucket by the number of iterations started. Finally, control is returned to the Monte Carlo iteration completion module (**5698**).

**FIG. 56** shows the Monte Carlo process for generating outcomes for year X cancer from a number of probability distributions, where X is a measure of cancer progression. For example, X can be measured as the number of years before or after the Transition Point, defined as the time of progression when the cure rate begins to decline steeply. Other reference points for measuring X may work as well. In this example, fifteen year X cases might be considered:

- 2 Years After the Transition Point
- 1 Year After the Transition Point
- 0 Years = At the Transition Point
- 1 Year Before the Transition Point
- 2 Years Before the Transition Point
- 3 Years Before the Transition Point
- 4 Years Before the Transition Point
- 5 Years Before the Transition Point
- 6 Years Before the Transition Point
- 7 Years Before the Transition Point
- 8 Years Before the Transition Point
- 9 Years Before the Transition Point
- 10 Years Before the Transition Point
- 11 Years Before the Transition Point
- 12 Years Before the Transition Point

In this example, there will be fifteen parallel versions of **FIG. 43 -** one for each year X case.

**FIG. 57** shows the Monte Carlo process for generating year X cancer plus no cancer PSA outcomes from a number of probability distributions. Each iteration is initiated by the Monte Carlo iteration controller (**5799**).

A volume for year X cancer is drawn from a probability distribution in module **4340**. The nature and values of the distribution may be affected by data from the personal profile. For example, age may influence the distribution. A value for biologic PSA is calculated in module **4342** as the product of the year X cancer PSA density from module **4341** and the volume of year X cancer from module **4340**. Trend PSA (**4343**) is the PSA trend multiplier (**4104**) multiplied by biologic PSA (**4342**), Trend variables add trend variation to biologic outcomes in order to simulate observed trend results. Trend multipliers (**4104**) typically have a mean of 1.0 and standard deviations equal to the coefficients of variation (CV) for each of the estimated trends (where CV = SD/Mean). The CVs are obtained from analysis of PSA trends.

Module **5796** evaluates whether each year X trend PSA from module **4343** is low enough compared to the observed trend PSA to warrant continuation of the iteration. If a year X trend (**1043**) exceeds the target range then it is clear the combined trend PSA (**5790**) will exceed the target range and the iteration should be stopped for the year X cancer case. More complex decision rules may be used to eliminate iterations that have a low probability of meeting the final test in module **5797** and avoid unnecessary calculations. If the decision is yes to continue the iteration for year X cancer the system may then proceed to module **5890** on **FIG. 58** to check and see if year X trend PSAV from module **5853** is low enough compared to the observed trend PSAV to warrant continuation of the iteration.

A volume for a healthy prostate is drawn from a probability distribution in module **4100**. The nature and values of the distribution are affected by data from the personal profile. For example, age may influence the distribution and a volume measurement will strongly influence the distribution. PSA density for a healthy prostate is drawn from a probability distribution in module **4101**. The nature and values of the distribution are affected by data from the personal profile. For example, age may influence the distribution; and past PSA and volume measurements may strongly influence the distribution if they are available. A value for biologic PSA is calculated in module **4102** as the product of the healthy PSA density from module **4101** and the volume of a healthy prostate from module **4100**.

A volume for volume growth is drawn from a probability distribution in module **4220**. The nature and values of the distribution are affected by data from the personal profile. For example, age may influence the distribution and a volume measurement will strongly influence the distribution. PSA density for volume growth is drawn from a probability distribution in module **4221**. The nature and values of the distribution are affected by healthy PSA density (**4101**) and data from the personal profile. For example, age may influence the distribution; and past PSA and volume measurements may strongly influence the distribution if they are available. A value for biologic PSA is calculated in module **4222** as the product of the volume growth PSA density from module **4221** and the volume of volume growth from module **4220**.

No cancer biologic PSA is calculated in module **5702** as the sum of healthy biologic PSA from module **4102** and volume growth biologic PSA from module **4222**.

No cancer trend PSA (**5703**) is the previously drawn PSA trend multiplier (**4104**) multiplied by no cancer biologic PSA (**5702**).

Year X cancer plus no cancer trend PSA is calculated in module **5790** as the sum of year X cancer trend PSA from module **5796** and no cancer trend PSA from module **5703**.

Module **5797** evaluates whether each year X cancer plus no cancer trend PSA from module **5790** is within the target range. If the decision is yes to continue the iteration for year X cancer the system may then proceed to module **5899** on **FIG. 58** to start the process for year X trend PSAV.

Trend PSA (**5797**) is the one output of this module, shown by the thick black arrow.

**FIG. 58** shows the Monte Carlo process for generating year X cancer plus no cancer PSAV outcomes from a number of probability distributions. Each iteration is initiated by the Monte Carlo iteration controller (**5899**).

A volume velocity for year X cancer is drawn from a probability distribution in module **4350**. The nature and values of the distribution are affected by the volume for cancer X drawn in module **4340** and data from the personal profile. For example, the mean and standard deviation for the volume velocity distribution tend to be larger for larger volumes. A value for biologic PSAV is calculated in module **4352** as the product of the year X cancer PSA density from module **4341** and the volume velocity of year X cancer from module **4350**. Trend PSAV (**4353**) is the PSAV trend multiplier (**4114**) multiplied by biologic PSAV (**4352**), Trend variables add trend variation to biologic outcomes in order to simulate observed trend results. Trend multipliers (**4114**) typically have a mean of 1.0 and standard deviations equal to the coefficients of variation (CV) for each of the estimated trends (where CV = SD/Mean). The CVs are obtained from analysis of PSA trends.

Module **5896** evaluates whether each year X trend PSAV from module **4353** is low enough compared to the observed trend PSAV to warrant continuation of the iteration. If a year X trend (**4353**) exceeds the target range then it is clear the combined trend PSA (**5890**) will exceed the target range and the iteration should be stopped for the year X cancer case. More complex decision rules may be used to eliminate iterations that have a low probability of meeting the final test in module **5897** and avoid unnecessary calculations.

Volume velocity for a healthy prostate is drawn from a probability distribution in module **4110.** The nature and values of the distribution are affected by the volume previously drawn in module **4100** and data from the personal profile. For example, the mean and standard deviation for the volume velocity distribution tend to be larger for larger volumes. PSA density for a healthy prostate was previously drawn from a probability distribution in module **4101**. The flow chart implicitly assumes that the PSA density of new healthy prostate tissue has the same PSA density as old healthy prostate tissue. If future research indicates they are different then a second healthy PSAV density module would be used with number **4111**. A value for biologic PSAV is calculated in module **4112** as the product of the healthy PSA density from module **4101** and the volume velocity of a healthy prostate from module **4110**.

Volume velocity for volume growth is drawn from a probability distribution in module **4230**. The nature and values of the distribution are affected by the healthy volume drawn in module **4100**, the volume for volume growth drawn in module **4220** and data from the personal profile. For example, the mean and standard deviation for the volume velocity distribution tend to be larger for larger volumes. PSA density for volume growth was previously drawn from a probability distribution in module **4221**. The flow chart implicitly assumes that the PSA density of new volume growth has the same PSA density as old volume growth. If future research indicates they are different then a second volume growth PSAV density module would be used with number **4231**. A value for biologic PSAV is calculated in module **4232** as the product of volume growth PSA density from module **4221** and the volume velocity for volume growth from module **4230**.

No cancer biologic PSAV is calculated in module **5812** as the sum of healthy biologic PSAV from module **4112** and volume growth biologic PSA from module **4232**.

No cancer trend PSAV (**5813**) is the previously drawn PSAV trend multiplier (**4114**) multiplied by no cancer biologic PSAV (**5812**).

Year X cancer plus no cancer trend PSAV is calculated in module **5890** as the sum of year X cancer trend PSAV from module **5896** and no cancer trend PSAV from module **5813.**

Module **5897** evaluates whether each year X cancer plus no cancer trend PSAV from module **5890** is within the target range. If the decision is yes to continue the iteration for year X cancer the system may then proceed to module **5999** on **FIG. 59** to start the process for year X trend Free PSA.

Trend PSAV (**5897**) is the one value output of this module, shown by the thick black arrow.

**FIG. 59** shows the Monte Carlo process for generating year X cancer plus no cancer Free PSA outcomes from a number of probability distributions. Each iteration is initiated by the Monte Carlo iteration controller (**5999**).

A value for healthy biologic Free PSA is calculated in module **4106** as the product of the healthy Free PSA % from module **4105** and biologic PSA from module **4102**. The Free PSA % for a healthy prostate is drawn from a probability distribution in module **4105**. The nature and values of the distribution are affected by data from the personal profile. For example, age may influence the distribution; and past PSA and Free PSA results will strongly influence the distribution if they are available.

A value for volume growth biologic Free PSA is calculated in module **4226** as the product of the volume growth Free PSA % from module **4225** and biologic PSA from module **4222**. The Free PSA % for volume growth is drawn from a probability distribution in module **4225**. The nature and values of the distribution are affected by the healthy Free PSA % (**4105**) and data from the personal profile.

For example, age may influence the distribution; and past PSA and Free PSA results will strongly influence the distribution if they are available.

No cancer biologic Free PSA is calculated in module **5906** as the sum of healthy biologic fSAV from module **4106** and volume growth biologic PSA from module **4226.**

A value for year X cancer biologic Free PSA is calculated in module **4346** as the product of the year X cancer Free PSA % from module **4345** and biologic PSA from module **4342**. The Free PSA % for year X cancer is drawn from a probability distribution in module **4345**. The nature and values of the distribution may be affected by data from the personal profile. For example, age may influence the distribution; and past PSA and Free PSA results may strongly influence the distribution if they are available.

Year X cancer plus no cancer biologic fPSA is calculated in module **5946** as the sum of year X cancer fPSA from module **4346** and no cancer fPSA from module **5906**.

A value for year X cancer plus no cancer biologic Free PSA % is calculated in module **5947** as biologic Free PSA from module **5946** divided by biologic PSA from module **5942**. Biologic PSA in module **5942** is calculated as the sum of healthy, volume growth and year X cancer biologic PSAs from modules **4102**, **4222** and **4342** respectively.

Trend Free PSA % (**5948**) is the Free PSA % trend multiplier (4109) multiplied by biologic Free PSA % (**5947**). Trend variables add trend variation to biologic outcomes in order to simulate observed trend results. Trend multipliers (4109) typically have a mean of 1.0 and standard deviations equal to the coefficients of variation (CV) for each of the estimated trends (where CV = SD/Mean). The CVs are obtained from analysis of Free PSA and PSA trends.

Module **5997** evaluates whether each year X cancer plus no cancer trend fPSA% from module **5948** is within the target range. If the decision is yes to continue the iteration for year X cancer the system may calculate trend fPSA in module **5990** and then proceed to module **6099** on **FIG. 60** to start the process for year X trend Free PSAV. Trend fPSA is calculated in module **5990** as trend fPSA% (**5997**) multiplied by previously calculated trend PSA (**5790**).

Trend fPSA% (**5997**) and trend fPSA (**5990**) are the two outputs of this module, shown by the thick black arrows.

**FIG. 60** shows the Monte Carlo process for generating year X cancer plus no cancer Free PSAV outcomes from a number of probability distributions. Each iteration is initiated by the Monte Carlo iteration controller (**6099**).

A value for healthy biologic Free PSAV is calculated in module **4116** as the product of the healthy Free PSA % previously drawn from module **4105** and biologic PSAV from module **4112.** The flow chart implicitly assumes that the Free PSA % of new healthy prostate tissue has the same Free PSA % as old healthy prostate tissue. If future research indicates they are different then a second healthy Free PSAV % module would be used with number **4115** instead of module **4105**.

A value for volume growth biologic Free PSAV is calculated in module **4236** as the product of the volume growth Free PSA % previously drawn from module **4225** and biologic PSAV from module **4232**. The flow chart implicitly assumes that the Free PSA % for volume growth has the same Free PSA % as old volume growth. If future research indicates they are different then a second volume growth Free PSAV % module would be used with number **4235** instead of module **4225**.

No cancer biologic Free PSAV is calculated in module **6016** as the sum of healthy biologic fPSAV from module **4116** and volume growth biologic fPSAV from module **4236**.

A value for year X cancer biologic Free PSAV is calculated in module **4356** as the product of the year X cancer Free PSA % from module **4345** and biologic PSA from module **4352**. The Free PSA % for year X cancer was previously drawn from a probability distribution in module **4345**. The flow chart implicitly assumes that the Free PSA % for year X cancer has the same Free PSA % as old year X cancer. If future research indicates they are different then a second year X cancer Free PSAV % module would be used with number **4355** instead of module **4345**. Year X cancer plus no cancer biologic fPSAV is calculated in module 6056 as the sum of year X cancer fPSAV from module **4356** and no cancer fPSAV from module **5916**.

A value for year X cancer plus no cancer biologic Free PSAV% is calculated in module **6057** as biologic Free PSAV from module **6056** divided by biologic PSAV from module **6052**. Biologic PSAV in module **6052** is calculated as the sum of healthy, volume growth and year X cancer biologic PSAVs from modules **4112**, **4232** and **4352** respectively.

Trend Free PSA V% (**6058**) is the Free PSAV % trend multiplier (**4119**) multiplied by biologic Free PSAV % (**6057**). Trend variables add trend variation to biologic outcomes in order to simulate observed trend results. Trend multipliers (**4119**) typically have a mean of 1.0 and standard deviations equal to the coefficients of variation (CV) for each of the estimated trends (where CV = SD/Mean). The CVs are obtained from analysis of Free PSA and PSA trends.

Module **6097** evaluates whether each year X cancer plus no cancer trend fPSAV% from module **6058** is within the target range. If the decision is yes to continue the iteration for year X cancer the system may calculate trend fPSAV in module **6090** and then return control to the iteration controller. Trend fPSAV is calculated in module **6090** as trend fPSAV% (**6097**) multiplied by previously calculated trend PSAV (5890).

Trend fPSA% (**5997**) and trend fPSA (**5990**) are the two outputs of this module, shown by the thick black arrows.

### Warnings and Alerts

Cancer warnings and alerts may be triggered by variables in the Dynamic Screening Analysis System and may determine choices of custom content. Cancer warnings may be triggered when a combination of the probability of progressing cancer and the years of early warning reach predetermined levels. Cancer alerts may be triggered when a combination of residual velocities and strength of evidence reach predetermined levels.

### Green, Caution and Stop Cases

One or two anomalous tests can skew trends and temporarily push results into an Alert status or even a Warning status - especially if the user is just starting Dynamic Screening or is testing infrequently. The Dynamic Screening System helps assess the impact of potentially anomalous tests by presenting results for additional cases where one or two of the most anomalous results are excluded - the Yellow Caution and Green cases. The Green Case excludes the two Test Pairs that most increase concern about progressing cancer. It provides the least early warning with the least overstatement of risk. The Yellow Caution Case urges caution before drawing any conclusions from this case. It excludes the test pair that causes most concern about progressing cancer. It provides earlier warning with more potential overstatement of risk than the Green case. The Red Stop

Case urges users to stop and pause before drawing any conclusions from this case. It provides the earliest warning but may overstate the risk based on only one or two tests. The Red Stop case doesn't exclude any tests, other than tests excluded because they are outside the tolerance area. False Alerts from minor infections of the prostate are most likely for this case.

### Cancer Warnings

Cancer warning status may determine custom content in reports to users. Warning levels may be triggered when specified variables reach predetermined levels, either individually or in combination. Variables that may trigger cancer warnings include the probability of progressing cancer and the number of years of early warning.

### Cancer Alerts

Cancer Alert status signals some concern about test trends and raises the question: How much sooner than one year should the next Test Pair (PSA + Free PSA) be scheduled? The Alert is likely to be caused by random variation or a mild infection of your prostate. In rare cases, it may be a very early hint of progressing cancer. Alert levels may be triggered when specified variables reach predetermined levels, either individually or in combination. Variables that may trigger Alerts include residual values such as residual PSA Velocity and residual Free PSA Velocity % and the strength of evidence based on variables such as the length of screening history and the number of screening tests of each type, for example PSA and Free PSA.

The Alert status has been triggered by an increase in the Alert level for at least one case on the graph at the bottom left. Alert levels are based on dynamic analysis of PSA and Free PSA trends, as shown later. Alert levels increase on a scale of 1 to 10 as trends look more like progressing cancer and less like volume growth. The seriousness of the Alert increases as the strength of test evidence increases, shown on the graph at the bottom right. Strength of Evidence increases with more tests over a longer period of time, as explained later. The Residual Velocity Map captures this information to create a picture of prostate cancer if it is progressing. Velocities are the annual change in each variable. Residual velocities are the annual changes from progressing cancer (in theory) after estimates of velocities caused by benign volume growth are subtracted. Residual PSA Velocity is the horizontal axis. Residual Free PSA Velocity % is the vertical axis - calculated as residual Free PSA Velocity divided by residual PSA Velocity. It is an attempt to measure the Free PSA % from new progressing cancer or unexpected prostate volume growth. Predetermined curves on the residual velocity map may be used to determine Alert levels either on their own or in combination with other variables, such as strength of evidence.

**Custom Content System**

A high level block diagram of how the custom content system might function is shown on **FIG. 61**. Custom content includes words, paragraphs, numbers, tables, graphs and other content used in custom reports produced by the system and suggested by the list of outputs on the right of **FIG. 61**. Custom content can depend on one input variable or combinations of two or more variables suggested by the list of input variables on the left. Custom content may take into account variations among the variables for the three cases: Red Stop, Yellow Caution and Green.

An example of custom content based on two variables is shown below with brief custom content shown in italics below each combination of probability of progressing cancer and length of early warning of progressing cancer:

If Low probability of progressing cancer and Long early warning then content is:

- *Wait patiently as continued testing decreases or increases the probability.* If High probability of progressing cancer and Long early warning then content is:
- *Explore treatments and timing in a deliberate manner because you have time.*
  If Low probability of progressing cancer and Short early warning then content is:
- *Test intensively because time is short in the unlikely event cancer is progressing.*
  If High probability of progressing cancer and Short early warning then content is:
- *Schedule best treatment quickly because you are short of time.*

**VI. Feedback Learning**

Feedback can be a part of improving the accuracy and reliability of one or more of the disclosed systems and methods. Evaluation of the experience of many men using disclosed approaches will provide better estimates of the values and probabilities of many of the variables used in the analysis. The results of each individual evaluation are combined with others and analyzed as a group to create summaries of all screening histories.

It can be less difficult to evaluate individual experience looking backward than it is to predict it looking forward. For example, looking backward allows us to separate individuals into two groups: men who have experienced progressing cancer and men who have not. This knowledge removes an uncertainty from the analysis and allows precise estimation of the contributions of progressing cancer and other factors like enlargement due to BPH.

Improving our ability to predict outcomes and estimate the probability distributions of those outcomes is a central part of the feedback learning process. Multi-dimensional response surfaces will be developed where possible to fine tune the predictions and estimates based on a variety of variables that may include age, race and other demographic variables. Response surfaces will be estimated using standard statistical methods, such as multiple regression analysis. They will be used for two groups of men: Men without Progressing Cancer who may be affected by Infections, Related Changes, and Enlargement from BPH; and Men with Progressing Cancer.

Here are two examples of what we expect to learn. For men without progressing cancer, the stability of the velocity densities is a determinant of our confidence in the predictions of PSA and Free PSA. We expect to learn more about how it behaves through feedback learning. For men with progressing cancer, the joint probability of concurrent changes in the residual Free PSA Velocity % and similar variables improves our confidence in early warning. We expect to learn more about how they are correlated through feedback learning.

## Overall and Detailed Feedback Learning

Two types of feedback learning will improve the method over time, as suggested by the flow chart on **FIG. 62**. Detailed feedback will improve the accuracy of estimates and predictions. Overall feedback will allow us to make sure that estimates of high level outcomes based on detailed estimates and predictions will be unbiased and consistent with overall results. Two examples of high level outcomes are progression probability and Cure Ratio. We will focus on them in much of the following discussion.

Detailed Feedback will be collected for every variable (or important variable) used in the estimation and prediction process. Best estimates and probability distributions will be calculated and used in the estimation and prediction parts of the method. For example, PSA and Free PSA velocity density may be considered important variables used in the prediction process for progression probability, as noted earlier. The probability distributions for predictions depend on how much those variables are likely to vary from year to year for a given man. Less variation for a wide range of men means a tighter probability distribution around the predictions based on those variables.

Overall Feedback calibrates the method so that estimates of high level outcomes using detailed methods are consistent with actual high level outcomes for groups of the population. For example, the average estimated probability for the whole population based on detailed methods should be consistent with the overall probability for the whole population. In addition, this consistency should be maintained for smaller groups of the population.

## Information Gathering

The feedback process depends on gathering information about outcomes, as suggested by **FIG. 63**. Information about outcomes can be fed back to Individual Screening History and to All Screening History for analysis of groups of individuals.

The Biopsy and Treatment module is on **FIG. 63**. For a biopsy, a doctor uses a device to inject thin hollow needles into the prostate to extract tissue. A pathologist exams the tissue and provides a diagnosis of prostate cancer if it exists. Primary treatment is intended to cure prostate cancer. It includes surgery to remove the prostate and various types of radiation to kill the cancer. A pathology report after surgery can provide useful information about the progress of cancer. The results of these pathology reports will provide useful feedback about outcomes that will allow us to improve the effectiveness of the method.

The Follow Up module is on **FIG. 63**. PSA tests and periodic physicals are used to follow patients' progress after treatment or no treatment depending on their choice. PSA tests are used to determine recurrence and the early progress of the disease. Later symptoms, metastasis and eventually death will be followed for many men. Feedback of these outcomes will help us improve the effectiveness of the method, as outlined in the next section.

The Feedback module is on **FIG. 63**. Decisions and results for each man can be analyzed to learn what actually happened. The results can be pooled with others and analyzed for common trends and probability distributions of outcomes. The distributions can be combined with information from a single man to improve predictions and estimates of probabilities, especially for progression.

## High Level Outcomes

There are a range of high level outcomes, including progression probability, Cure Ratio, metastasis and death from prostate cancer and side effects of treatment. We will focus on examples for progression probability, predictions and Cure Ratio.

## Feedback for Progression Probability

We will discuss how we use feedback from two types of primary tests to estimate progression probability: PSA and Free PSA tests and additional confirming tests. The flow chart on **FIG. 64** suggests: How the probability of progressing cancer is calibrated using overall feedback from many men; and How detailed feedback is used to improve predictions and estimates of the probability distributions of the predictions.

## Detailed Feedback for Predictions

Predictions of PSA and Free PSA, and hypotheses about their production by cancer, can be used for estimating the probability of progression, as we have seen. Detailed feedback about predictions of PSA and Free PSA and the associated prediction error are the starting point for improving predictions, as suggested by the flow chart on FIG. 65.

### Detailed Feedback for No Progressing Cancer

Detailed feedback is analyzed for every variable of the prediction process for no progressing cancer, as suggested on **FIG. 66**. We have already mentioned the importance of density velocity and how its stability is likely to allow accurate predictions. Detailed feedback from other variables is also likely to turn out to be helpful in improving the method.

### Detailed Feedback for Infections

Detailed feedback will help improve our ability to identify test results distorted by infections of the prostate. Feedback about PSA, Free PSA and other test results will be analyzed for men who have been diagnosed with infections, possibly using the feedback shown on **FIG. 66**.

### Detailed Feedback for Related Changes

Detailed feedback will help improve our ability to analyze how test results are distorted by related changes in diet, treatment, medication and other factors. Feedback about PSA, Free PSA and other test results will be analyzed for men who have made changes in one or more of these factors with the goal of improving our ability to predict the impact of the changes in other men, possibly using the feedback shown on **FIG. 66**.

### Detailed Feedback for Progressing Cancer

Detailed feedback is analyzed for every variable of the hypothesis generation process for progressing cancer, as suggested on **FIG 67**. Variables include PSA doubling time and variations in the Free PSA velocity %. Analysis of the probability distributions of these and other variables will be used in the higher level process of estimating the probability of progressing cancer.

### Feedback from Confirming Tests

Previous sections have outlined how PSA and Free PSA are predicted, analyzed and combined to predict ratios. Those sections are summarized on the left side of the flow chart on **FIG. 68**. A similar process is carried out for PSA and one or more additional variables, as summarized on the right side of the flow chart using the general term xPSA. The broad goal of this feedback process is to find one or more confirming tests that when combined with PSA and Free PSA will provide strong confirmation of early warning of progressing cancer. The flow chart on **FIG. 68** suggests: How feedback from a range of confirming tests is used to estimate the probability of progressing cancer using overall feedback from many men; and How detailed feedback is used to improve predictions and estimates of the probability distributions of the predictions.

Estimates of Cure Ratio used in the method will also be improved by feedback. The high level flow chart on **FIG. 69** suggests how feedback about a variety of outcomes will be used to improve estimates of the Cure Ratio as a function of primary results. Primary test results will be related to the pathology results after biopsy and treatment (if surgery) and eventually to the probability of recurrence, metastasis and eventually death. For example, the results of late detection are likely to lead less favorable pathology (perhaps Stage T2 and Gleason 7 or more), more frequent recurrence, metastasis and eventually death. In contrast, the results of early detection are likely to lead to favorable pathology (Stage T1, Gleason 6 or more, and small cancer volumes).

### Feedback for Estimating Cure Ratio

Details of how feedback will be used to estimate Cure Ratio are shown on the flow chart on **FIG. 70**. Outcomes for men with surgery, no treatment and progressing cancer will be used to supplement and eventually replace the results from studies reported in medical journals. The Cancer Score will be estimated for men in each group and feedback about the corresponding results will be used to improve each step in the calculation of Cure Ratio.

A computer readable medium is disclosed herein that comprises computer readable instructions, wherein the computer readable instructions when executed cause a processor to execute a method of the invention. The instructions can operate in a software runtime environment. The computer readable instructions can be packaged and marketed as a software product or part of a software package. For example, the instructions can be packaged with an assay kit for PSA.

The invention also provides a data signal that is transmitted using a network, wherein the data signal comprises information provided from a method of the invention. The data signal can comprise packetized data that is transmitted through a carrier wave across the network. For example, data can be sent to a server where a method of the invention is executed on the data and then the data is sent back to a user at a remote location. The data can be sent by any type of network, including a wireless network.

In another aspect of the invention, a medical information system for assessing a disease of a subject is provided that comprises: an input device for receiving subject data corresponding to a biomarker for the disease at least two different times, wherein the data corresponding to the at least two different times form a first trend; a processor that assesses a probability of said trend relating to historical data; a storage unit in communication with the processor having a database for: (i) storing the subject data; (ii) storing historical data related to the disease; and an output device that transmits information relating to the probability of said trend relating to historical data to an end user.

The invention also provides a method for assessing a disease in a subject comprising: collecting data from the subject corresponding to a biomarker for the disease at least two different times, wherein the data corresponding to the at least two different times form a trend; exporting said data for manipulation of said data by executing a method of the invention; and importing the results of said manipulation to an end user. For example, data is collected at a first location, such as a hospital, the data is exported to a second location, such as a remote server in any remote location, where a method of the invention is executed to obtain information regarding the disease in a subject, and then the information is imported from the remote location back to the first location, such as the point-of-care in the hospital, or the information is imported to a third location, such as a database.

With respect to this disclosure, while examples have been used to disclose the invention, including the best mode, and also to enable any person skilled in the art to make and use the invention, the patentable scope of the invention is defined by claims, and may include other examples that occur to those skilled in the art. Accordingly the examples disclosed herein are to be considered non-limiting. As an illustration, it should be understood that for the processing flows described herein, the steps and the order of the steps may be altered, modified, removed and/or augmented and still achieve the desired outcome. A multiprocessing or multitasking environment could allow two or more steps to be executed concurrently.

It is further noted that the systems and methods may be implemented on various types of computer architectures, such as for example on a networked system (e.g.,

**FIG. 71**), or in a client-server configuration, or in an application service provider configuration, on a single general purpose computer or workstation (e.g., **FIG. 72**), etc. The systems and methods may include data signals conveyed via networks (e.g., local area network, wide area network, internet, combinations thereof, etc.), fiber optic medium, carrier waves, wireless networks, etc. for communication with one or more data processing devices. The data signals can carry any or all of the data disclosed herein (e.g., user input data, the results of the analysis to a user, etc.) that is provided to or from a device.

Additionally, the methods and systems described herein may be implemented on many different types of processing devices by program code comprising program instructions that are executable by the device processing subsystem. The software program instructions may include source code, object code, machine code, or any other stored data that is operable to cause a processing system to perform methods described herein.

The systems' and methods' data (e.g., associations, mappings, etc.) may be stored and implemented in one or more different types of computer-implemented ways, such as different types of storage devices and programming constructs (e.g., data stores, RAM, ROM, Flash memory, flat files, databases, programming data structures, programming variables, IF-THEN (or similar type) statement constructs, etc.). It is noted that data structures describe formats for use in organizing and storing data in databases, programs, memory, or other computer-readable media for use by a computer program.

The systems and methods may be provided on many different types of computer-readable media including computer storage mechanisms (e.g., CD-ROM, diskette, RAM, flash memory, computer's hard drive, etc.) that contain instructions (e.g., software) for use in execution by a processor to perform the methods' operations and implement the systems described herein.

The computer components, software modules, functions, data stores and data structures described herein may be connected directly or indirectly to each other in order to allow the flow of data needed for their operations. It is also noted that a module or processor includes but is not limited to a unit of code that performs a software operation, and can be implemented for example as a subroutine unit of code, or as a software function unit of code, or as an object (as in an object-oriented paradigm), or as an applet, or in a computer script language, or as another type of computer code. The software components and/or functionality may be located on a single computer or distributed across multiple computers depending upon the situation at hand.

## Claims

**1.** A method of assessing a disease in a subject comprising:

obtaining data from a subject corresponding to a biomarker for a disease at least two different times, wherein the data corresponding to the at least two different times form a first trend;
removing data from said first trend that has a value outside a tolerance, thereby forming a second trend; and
determining a relationship between said second trend and a historical trend, wherein the historical trend corresponds to the biomarker for the disease.

**2.** A method of assessing a disease in a subject comprising:

obtaining data from a subject corresponding to a biomarker for a disease at least two different times, wherein the data corresponding to the at least two different times form a first trend;
removing data from the first trend that has a value outside a tolerance, thereby forming a second trend; and
estimating future values for the biomarker using the second trend.

**3.** The method of claim 1, wherein the velocity of the second trend is used to determine the relationship between said second trend and said historical trend.

**4.** The method of claim 1, wherein the relationship is determined by a probability calculation.

**5.** The method of claim 4, wherein the probability calculation is a Bayesian method.

**6.** The method of claim 1 or 2, wherein the data comprises a ratio of two biomarkers for the disease.

**7.** The method of claim 1 or 2, wherein the disease is prostate cancer and wherein at least one biomarker is selected from the group consisting of fPSA and PSA.

**8.** The method of claim 1 or 2, wherein the tolerance is determined by a feedback iteration process.

**9.** A system for assessing a disease in a subject comprising a Monte Carlo calculation engine, wherein the engine executes the method of claim 1.

**10.** A method to assess the probability of progressing disease in a subject comprising:

obtaining data from a subject corresponding to a biomarker for a disease at least two different times, wherein the data corresponding to the at least two different times form a trend;
assessing a prior probability of progressing disease at more than one time point; and
determining the probability of said trend relating to the prior probability of progressing disease, thereby assessing the probability of progressing disease.

**11.** A method for screening for progressing cancer comprising:

estimating two trends over at least two time points corresponding to data from two biomarkers and the ratio between the two biomarkers;
determining prior probabilities of progressing cancer at different times; and
estimating the probability of progressing cancer by performing a Bayesian method with said trend and said prior probabilities, thereby screening for progressing cancer.

**12.** A method of assessing disease progression comprising:

determining prior probabilities of cancer occurrence at more than one time;
obtaining data from a subject corresponding to a biomarker for a disease at least two different times, wherein the data corresponding to the at least two different times form a trend;
determining a probability of an observation of the trend conditional on no progressing disease;
determining a probability of an observation of the trend conditional on progressing disease; and
assessing disease progression by performing a Bayesian method with the prior probabilities, the probability of an observation conditional on no progressing disease, and the probability of an observation conditional on progressing disease.

**13.** The method of claim 10, 11, or 12, wherein the disease is prostate cancer and wherein a biomarker is selected from the group consisting of fPSA and PSA.

**14.** The method of claim 10 or 12 further comprising removing data that has a value outside a tolerance from the trend.

**15.** The method of claim 11 further comprising removing data that has a value outside a tolerance from the two trends.

**16.** The method of claim 12, wherein the probability of an observation of the trend conditional on no progressing disease is calculated using Monte Carlo methods.

**17.** The method of claim 12, wherein the probability of an observation of the trend conditional on progressing disease is calculated using Monte Carlo methods.

**18.** A method of delivering medical treatment to a subject with a disease comprising:

obtaining information provided from executing a method of claim 1, 2, 10, 11, or 12;
determining a course of medical action based on said information; and
delivering medical treatment by following the course of medical action.

**19.** The method of claim 18, wherein the course of medical action comprises administration of medical tests.

**20.** The method of claim 18, wherein the course of medical action comprises a specific time for delivering medical treatment.

**21.** The method of claim 18, wherein the course of medical action comprises calculation of a Life Score.

**22.** A method of diagnosing a disease in a subject comprising:

obtaining information provided from executing a method of claim 1, 2, 10, 11, or 12; and
diagnosing the disease in the subject using said information.

**23.** A computer readable medium comprising computer readable instructions, wherein the computer readable instructions when executed cause a processor to execute a method of claim 1, 2, 10, 11, or 12.

**24.** The computer readable medium of claim 23, wherein the instructions operate in a software runtime environment.

**25.** A data signal that is transmitted using a network, wherein the data signal comprises information provided from a method of claim 1, 2, 10, 11, or 12.

**26.** The data signal of claim 25 further comprising packetized data that is transmitted through a carrier wave across the network.

**27.** A medical information system for assessing a disease of a subject comprising:

an input device for receiving subject data corresponding to a biomarker for the disease at least two different times, wherein the data corresponding to the at least two different times form a first trend;
a processor that assesses a probability of said trend relating to historical data;
a storage unit in communication with the processor having a database for: (i) storing the subject data; (ii) storing historical data related to the disease; and
an output device that transmits information relating to the probability of said trend relating to historical data to an end user.

**28.** A method of assessing a disease in a subject comprising
collecting data from the subject corresponding to a biomarker for the disease at least two different times, wherein the data corresponding to the at least two different times form a trend;
exporting said data for manipulation of said data by executing a method of claim 1, 2, 10, 11, or 12; and
importing the results of said manipulation to an end user.

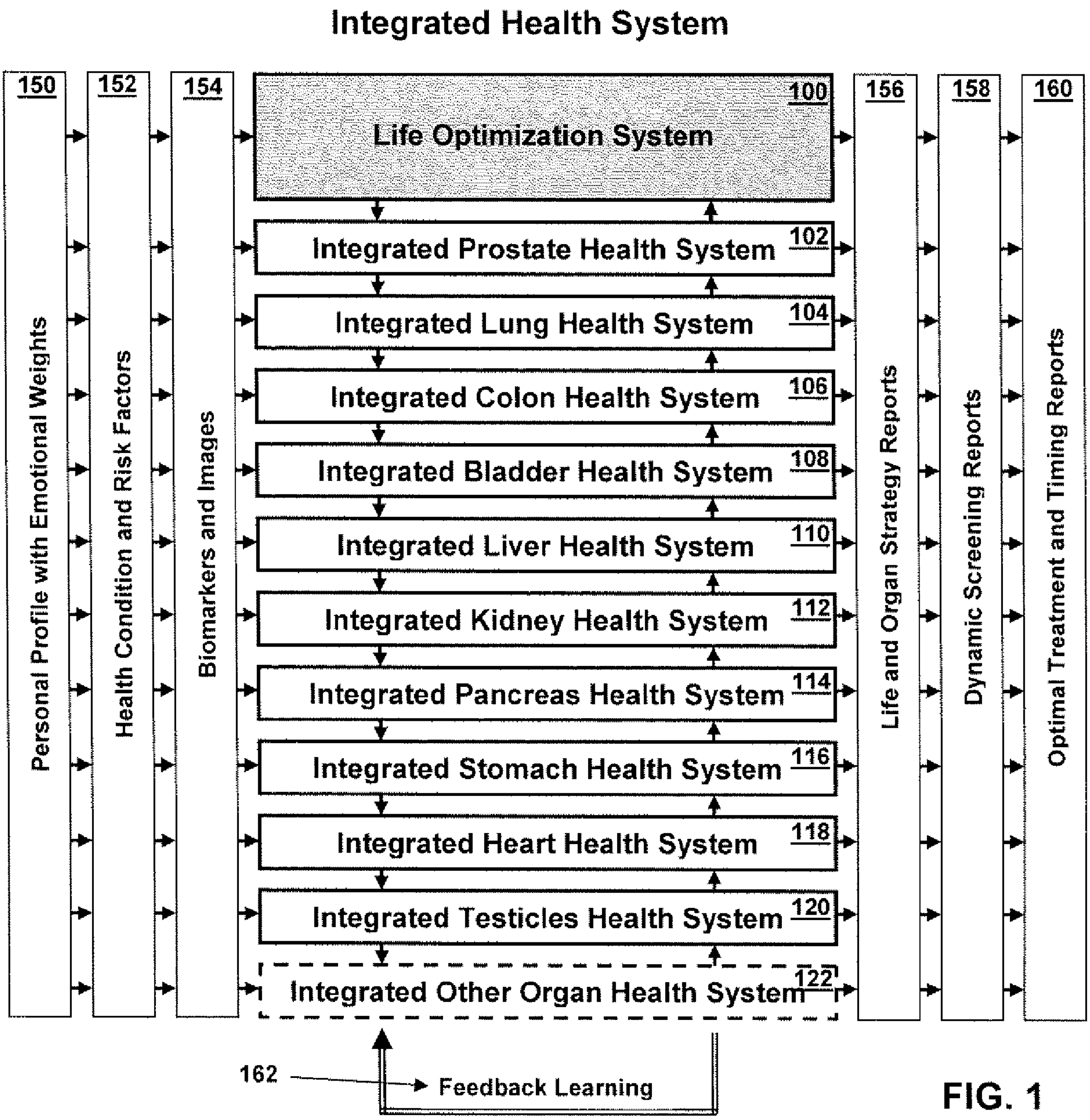
Integrated Health System
150
Personal Profile with Emotional Weights
152
Health Condition and Risk Factors
154
Biomarkers and Images
100
Life Optimization System
Integrated Prostate Health System 102
Integrated Lung Health System 104
Integrated Colon Health System 106
Integrated Bladder Health System 108
Integrated Liver Health System 110
Integrated Kidney Health System 112
Integrated Pancreas Health System 114
Integrated Stomach Health System 116
Integrated Heart Health System 118
Integrated Testicles Health System 120
Integrated Other Organ Health System 122
156
Life and Organ Strategy Reports
158
Dynamic Screening Reports
160
Optimal Treatment and Timing Reports
162
Feedback Learning

FIG. 1

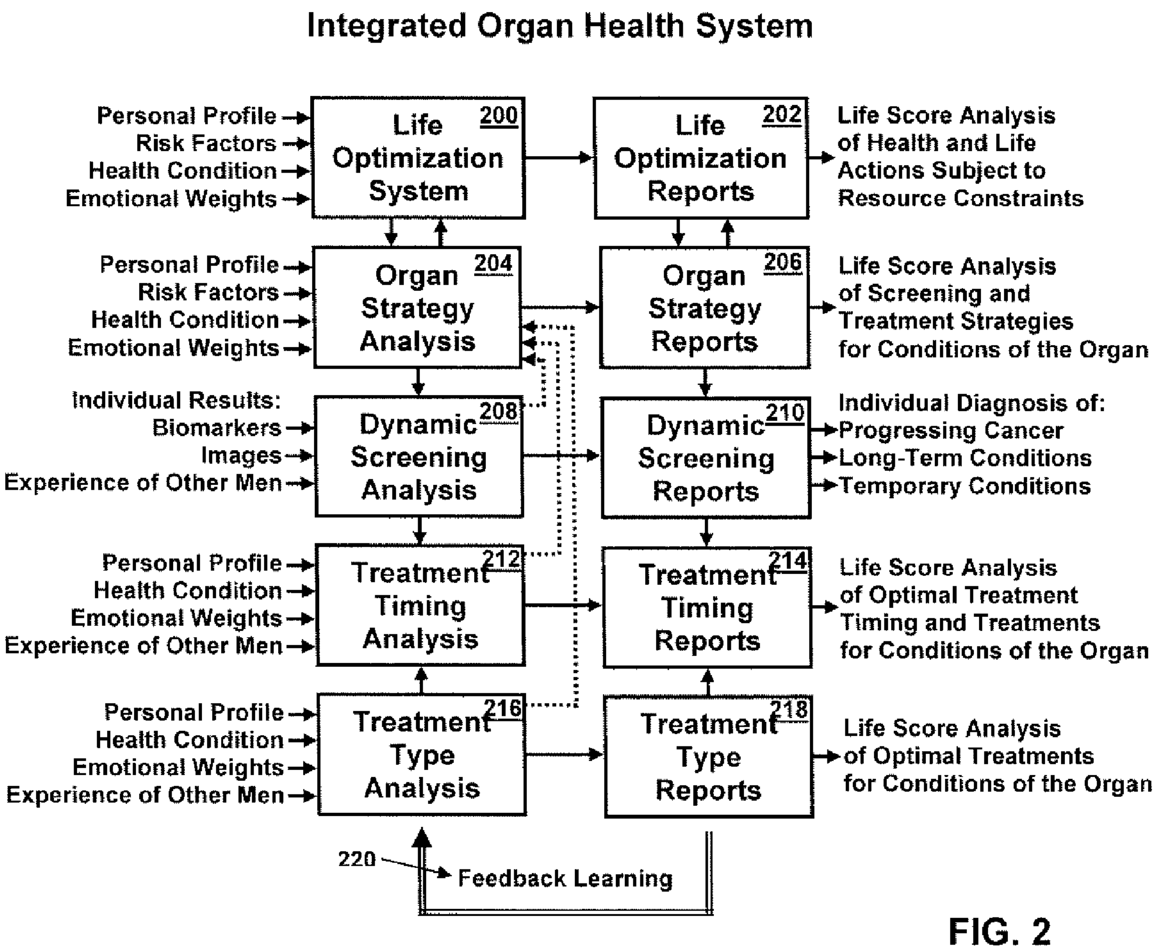
Integrated Organ Health System
Personal Profile
Risk Factors
Health Condition
Emotional Weights
Life Optimization System 200
Life Optimization Reports 202
Life Score Analysis of Health and Life Actions Subject to Resource Constraints
Personal Profile
Risk Factors
Health Condition
Emotional Weights
Organ Strategy Analysis 204
Organ Strategy Reports 206
Life Score Analysis of Screening and Treatment Strategies for Conditions of the Organ
Individual Results:
Biomarkers
Images
Experience of Other Men
Dynamic Screening Analysis 208
Dynamic Screening Reports 210
Individual Diagnosis of:
Progressing Cancer
Long-Term Conditions
Temporary Conditions
Personal Profile
Health Condition
Emotional Weights
Experience of Other Men
Treatment Timing Analysis 212
Treatment Timing Reports 214
Life Score Analysis of Optimal Treatment Timing and Treatments for Conditions of the Organ
Personal Profile
Health Condition
Emotional Weights
Experience of Other Men
Treatment Type Analysis 216
Treatment Type Reports 218
Life Score Analysis of Optimal Treatments for Conditions of the Organ
220
Feedback Learning

FIG. 2

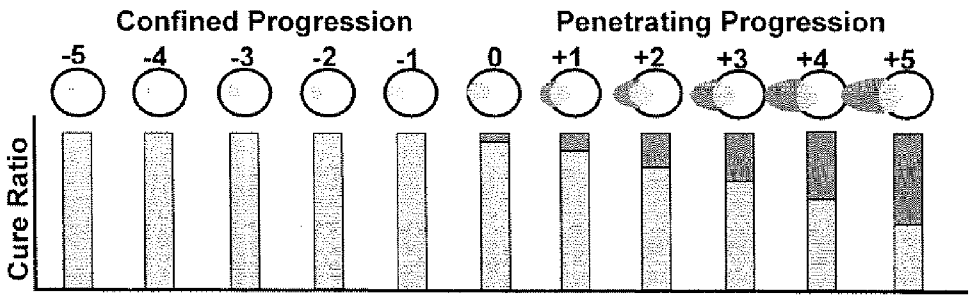
Confined Progression
Penetrating Progression
-5
-4
-3
-2
-1
0
+1
+2
+3
+4
+5
Cure Ratio

FIG. 3

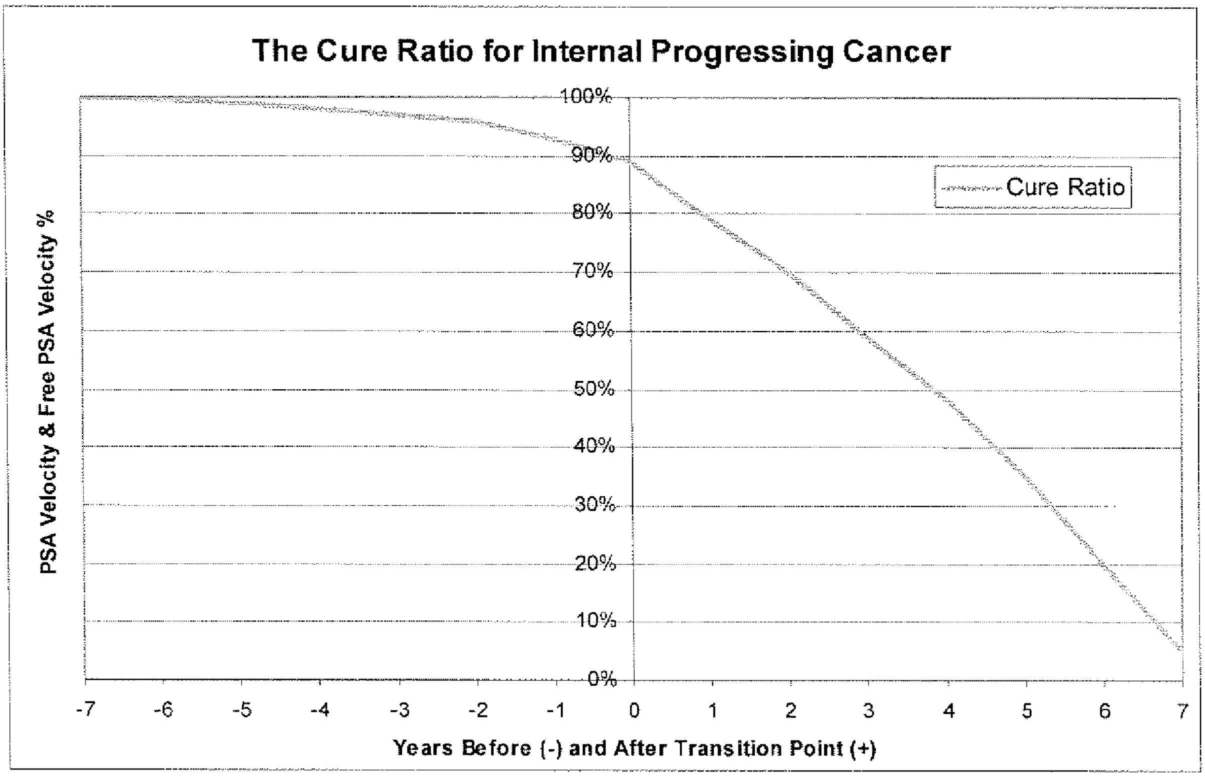
The Cure Ratio for Internal Progressing Cancer
100%
90%
80%
70%
60%
50%
40%
30%
20%
10%
0%
Cure Ratio
PSA Velocity & Free PSA Velocity %
-7
-6
-5
-4
-3
-2
-1
0
1
2
3
4
5
6
7
Years Before (-) and After Transition Point (+)

FIG. 4

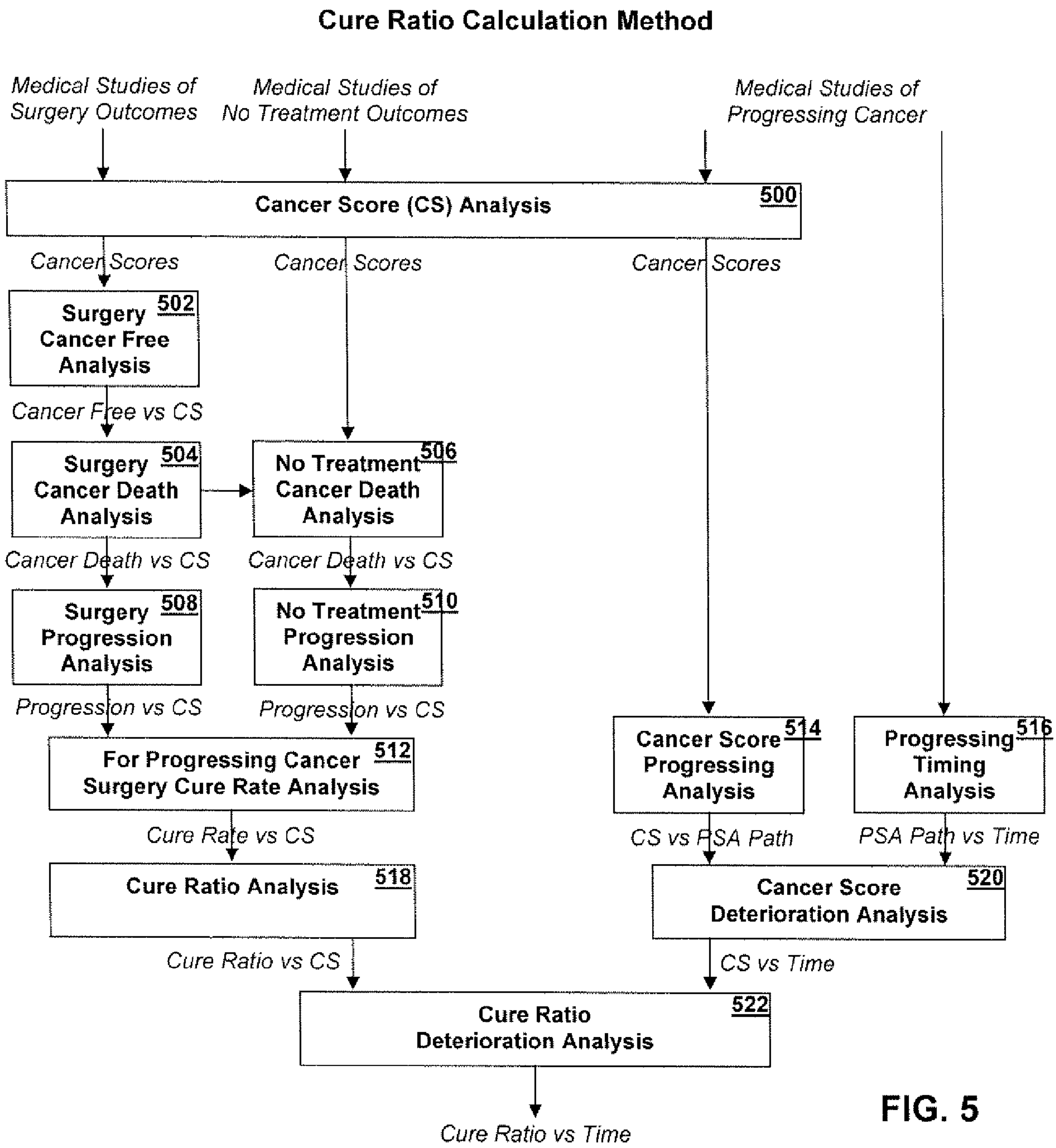
Cure Ratio Calculation Method
Medical Studies of Surgery Outcomes
Medical Studies of No Treatment Outcomes
Medical Studies of Progressing Cancer
Cancer Score (CS) Analysis 500
Cancer Scores
Cancer Scores
Cancer Scores
Surgery Cancer Free Analysis 502
Cancer Free vs CS
Surgery Cancer Death Analysis 504
No Treatment Cancer Death Analysis 506
Cancer Death vs CS
Cancer Death vs CS
Surgery Progression Analysis 508
No Treatment Progression Analysis 510
Progression vs CS
Progression vs CS
For Progressing Cancer Surgery Cure Rate Analysis 512
Cancer Score Progressing Analysis 514
Progressing Timing Analysis 516
Cure Rate vs CS
CS vs PSA Path
PSA Path vs Time
Cure Ratio Analysis 518
Cancer Score Deterioration Analysis 520
Cure Ratio vs CS
CS vs Time
Cure Ratio Deterioration Analysis 522
Cure Ratio vs Time

FIG. 5

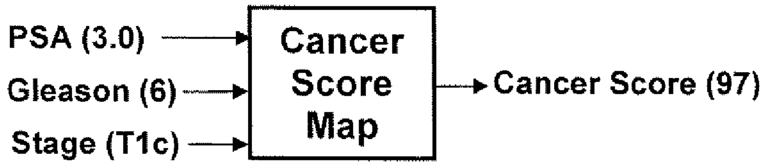


FIG. 6

| PSA | Gleason | Stage | Cancer Score |
|---|---|---|---|
| 3 | 6 | T1c | 97 |
| 3 | 3+4 | T1c | 95 |
| 8 | 6 | T1c | 95 |
| 8 | 3+4 | T1c | 91 |
| 8 | 4+3 | T1c | 83 |
| 8 | 4+3 | T2a | 78 |
| 14 | 4+3 | T2a | 67 |
| 18 | 8 | T2a | 52 |

FIG. 7

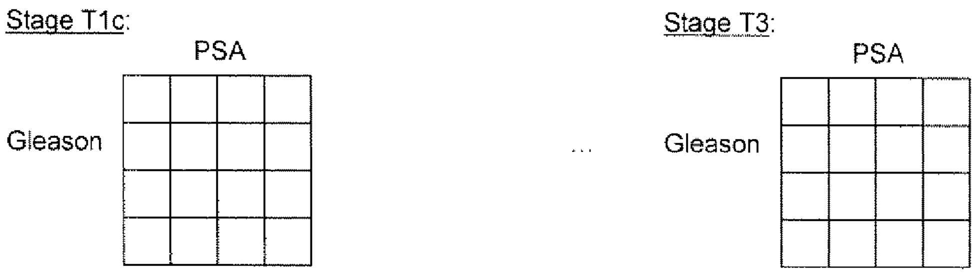


FIG. 8

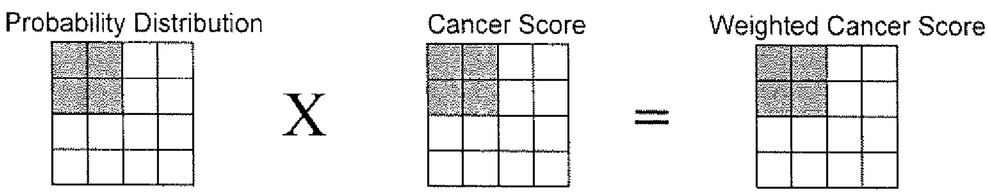


FIG. 9

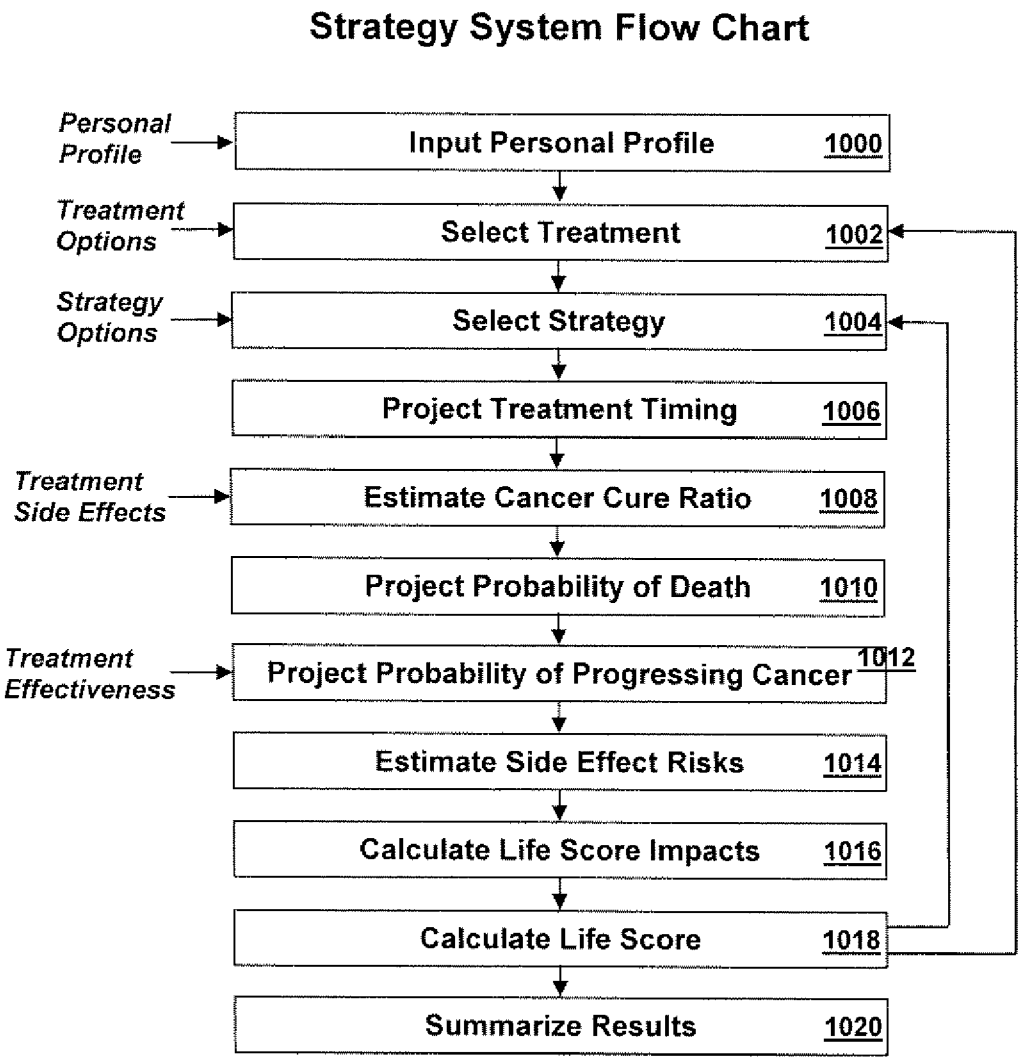
Strategy System Flow Chart
Personal Profile
Input Personal Profile 1000
Treatment Options
Select Treatment 1002
Strategy Options
Select Strategy 1004
Project Treatment Timing 1006
Treatment Side Effects
Estimate Cancer Cure Ratio 1008
Project Probability of Death 1010
Treatment Effectiveness
Project Probability of Progressing Cancer 1012
Estimate Side Effect Risks 1014
Calculate Life Score Impacts 1016
Calculate Life Score 1018
Summarize Results 1020

FIG. 10

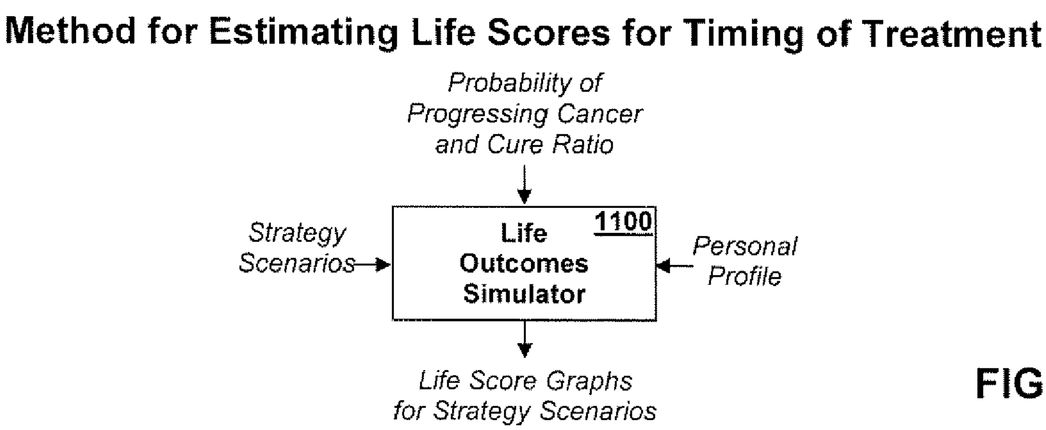
Method for Estimating Life Scores for Timing of Treatment
Probability of Progressing Cancer and Cure Ratio
Strategy Scenarios
Life Outcomes Simulator 1100
Personal Profile
Life Score Graphs for Strategy Scenarios

FIG. 11

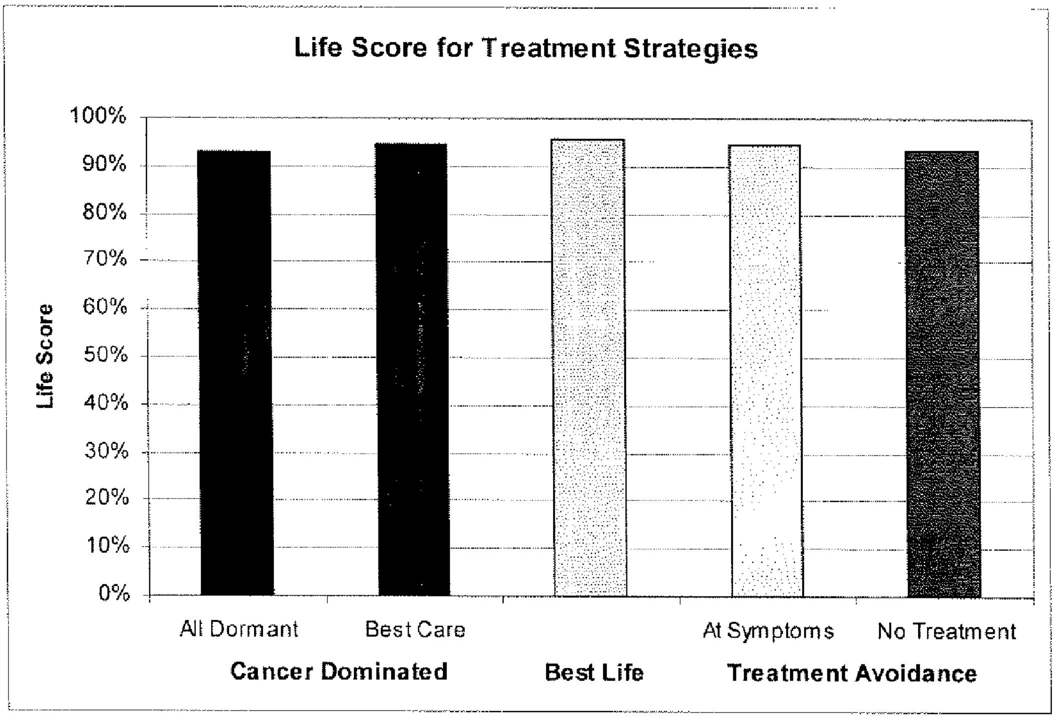
Life Score for Treatment Strategies
Life Score
100%
90%
80%
70%
60%
50%
40%
30%
20%
10%
0%
All Dormant
Best Care
At Symptoms
No Treatment
Cancer Dominated
Best Life
Treatment Avoidance

FIG. 12

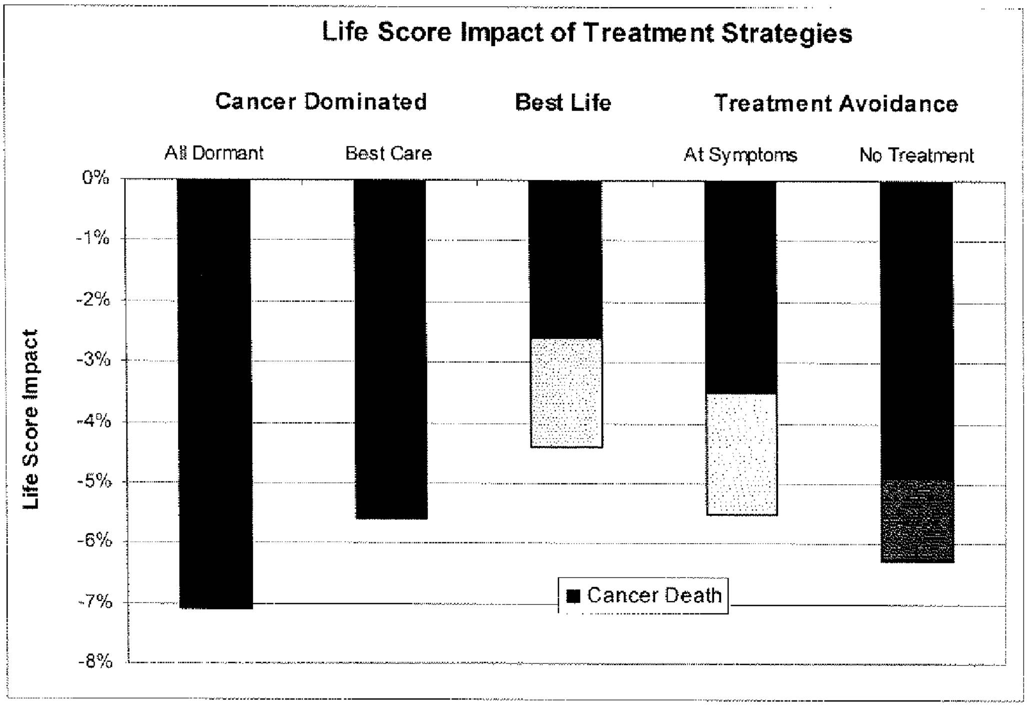
Life Score Impact of Treatment Strategies
Cancer Dominated
Best Life
Treatment Avoidance
All Dormant
Best Care
At Symptoms
No Treatment
Life Score Impact
0%
-1%
-2%
-3%
-4%
-5%
-6%
-7%
-8%
Cancer Death

FIG. 13

Treatment Timing System Flow Chart

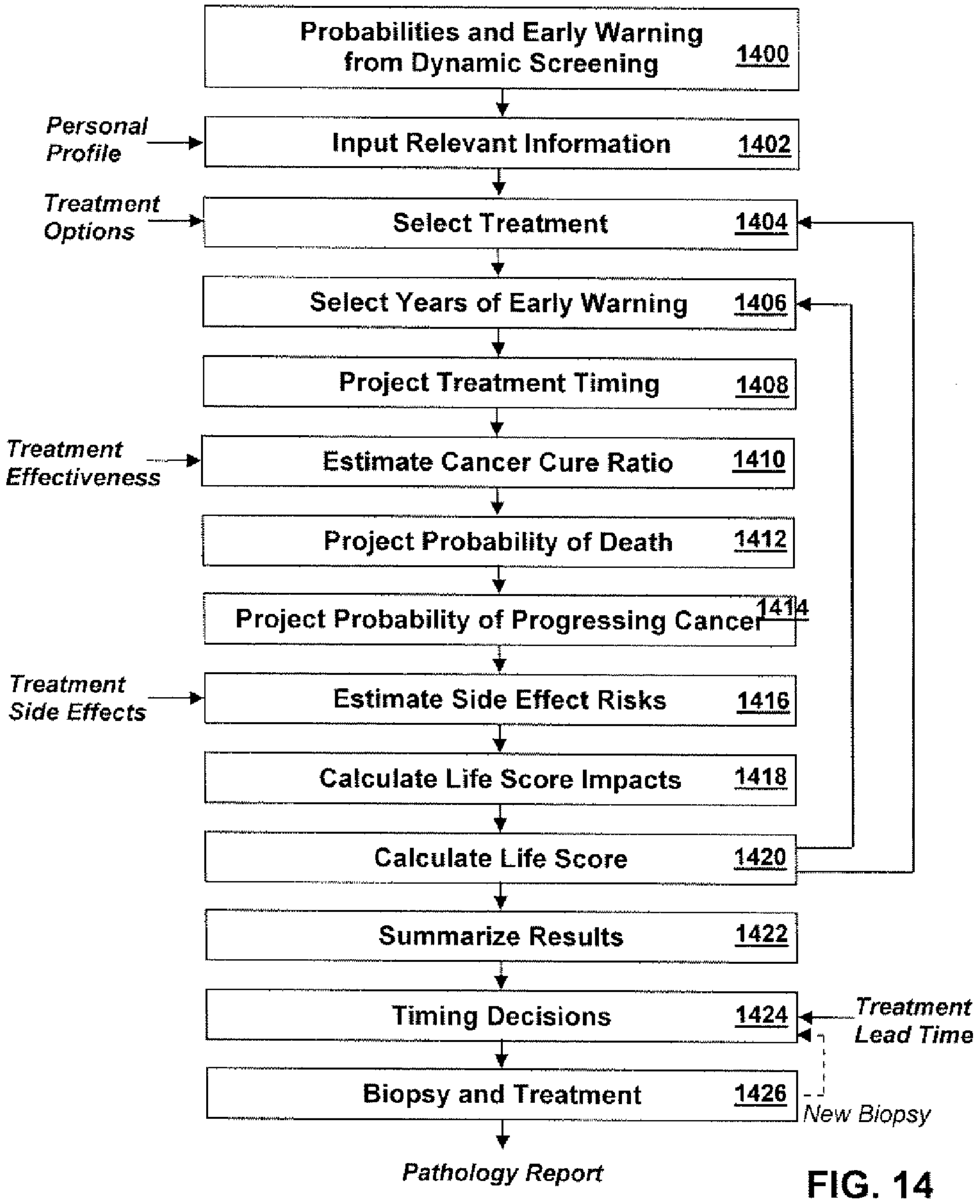


FIG. 14

Method for Estimating Life Scores for Timing of Treatment

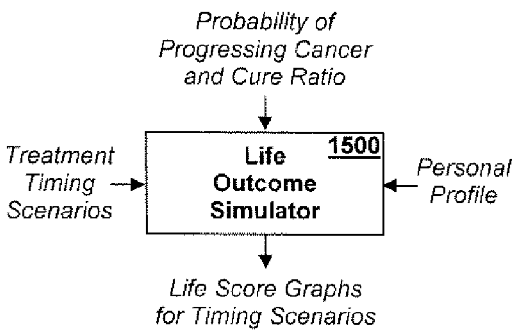


FIG. 15

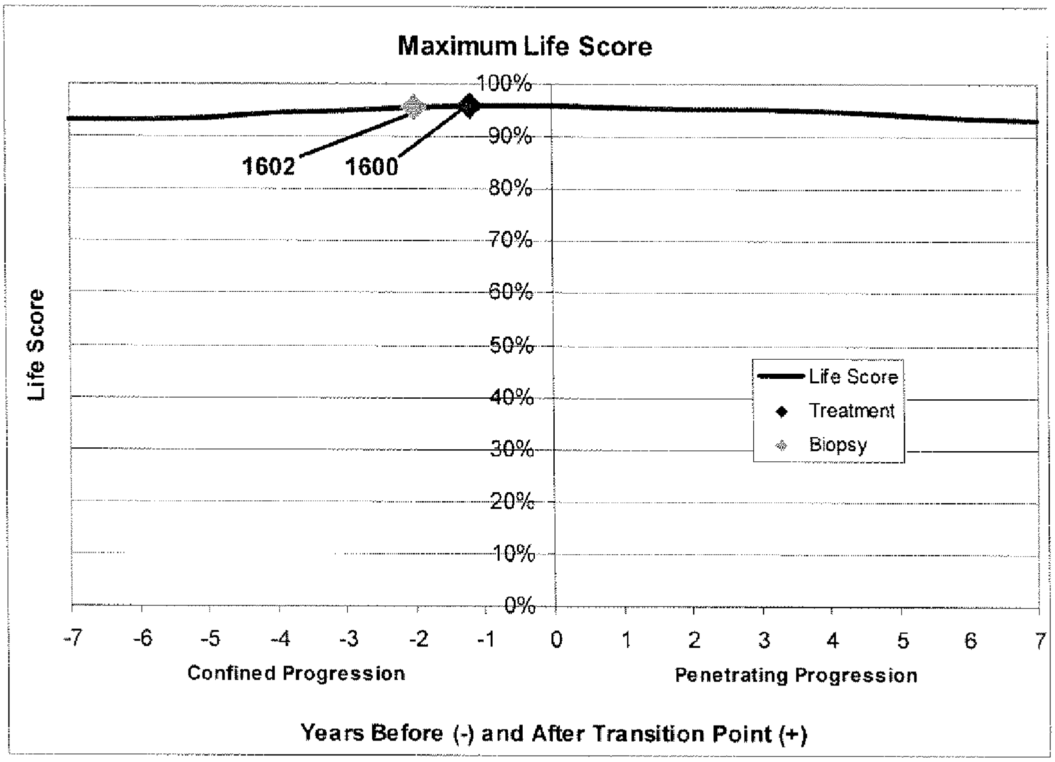
Maximum Life Score
Life Score
100%
90%
80%
70%
60%
50%
40%
30%
20%
10%
0%
1602
1600
Life Score
Treatment
Biopsy
-7 -6 -5 -4 -3 -2 -1 0 1 2 3 4 5 6 7
Confined Progression
Penetrating Progression
Years Before (-) and After Transition Point (+)

FIG. 16

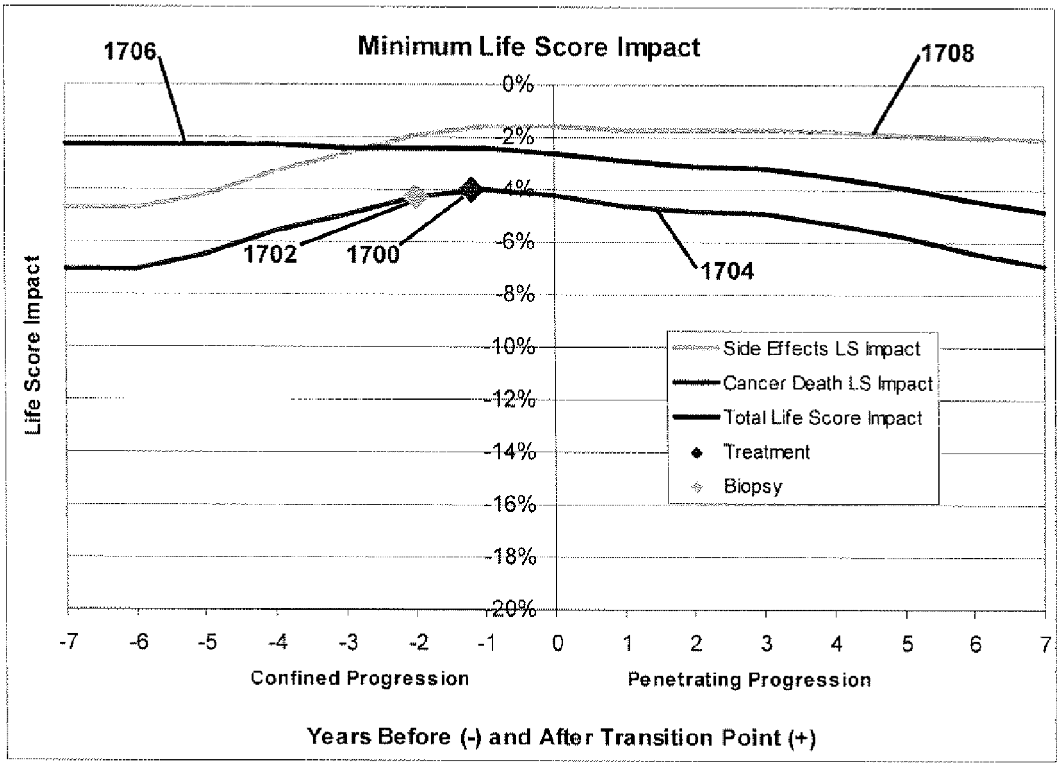
Minimum Life Score Impact
1706
1708
Life Score Impact
0%
-2%
-4%
-6%
-8%
-10%
-12%
-14%
-16%
-18%
-20%
1702
1700
1704
Side Effects LS Impact
Cancer Death LS Impact
Total Life Score Impact
Treatment
Biopsy
-7 -6 -5 -4 -3 -2 -1 0 1 2 3 4 5 6 7
Confined Progression
Penetrating Progression
Years Before (-) and After Transition Point (+)

FIG. 17

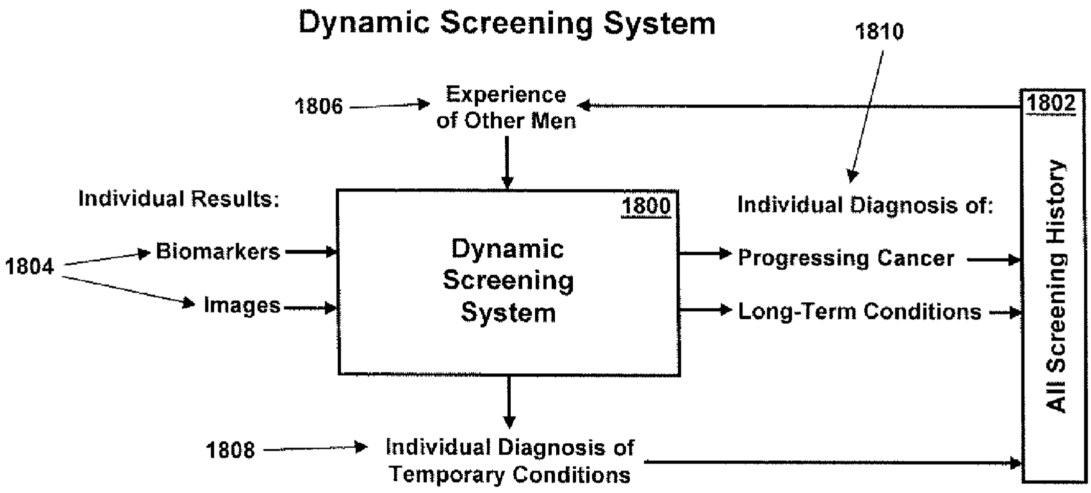
Dynamic Screening System
1810
1806
Experience of Other Men
1802
Individual Results:
1800
Dynamic Screening System
Individual Diagnosis of:
1804
Biomarkers
Images
Progressing Cancer
Long-Term Conditions
All Screening History
1808
Individual Diagnosis of Temporary Conditions

FIG. 18

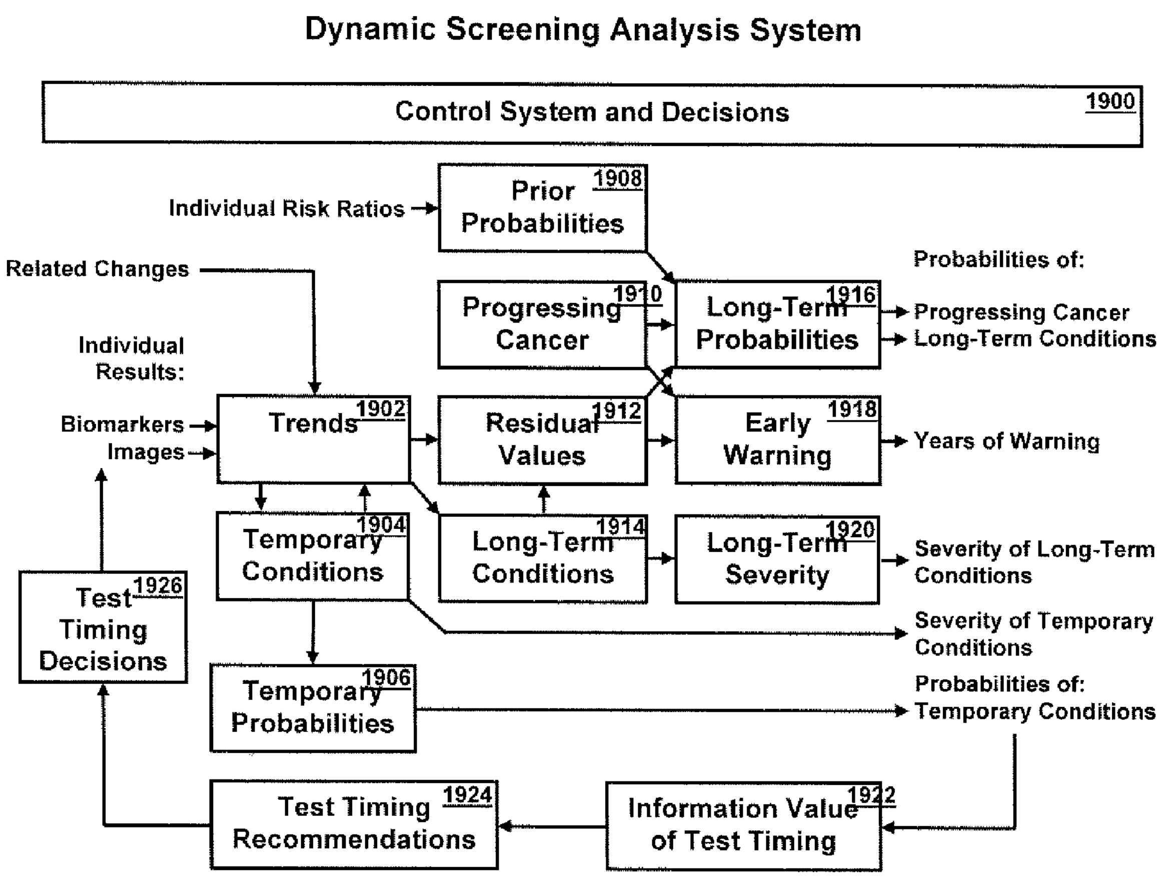
Dynamic Screening Analysis System
Control System and Decisions
1900
Individual Risk Ratios
Prior Probabilities
1908
Related Changes
Probabilities of:
Progressing Cancer
1910
Long-Term Probabilities
1916
Progressing Cancer
Long-Term Conditions
Individual Results:
Biomarkers
Images
Trends
1902
Residual Values
1912
Early Warning
1918
Years of Warning
Temporary Conditions
1904
Long-Term Conditions
1914
Long-Term Severity
1920
Severity of Long-Term Conditions
Test Timing Decisions
1926
Severity of Temporary Conditions
Temporary Probabilities
1906
Probabilities of:
Temporary Conditions
Test Timing Recommendations
1924
Information Value of Test Timing
1922

FIG. 19

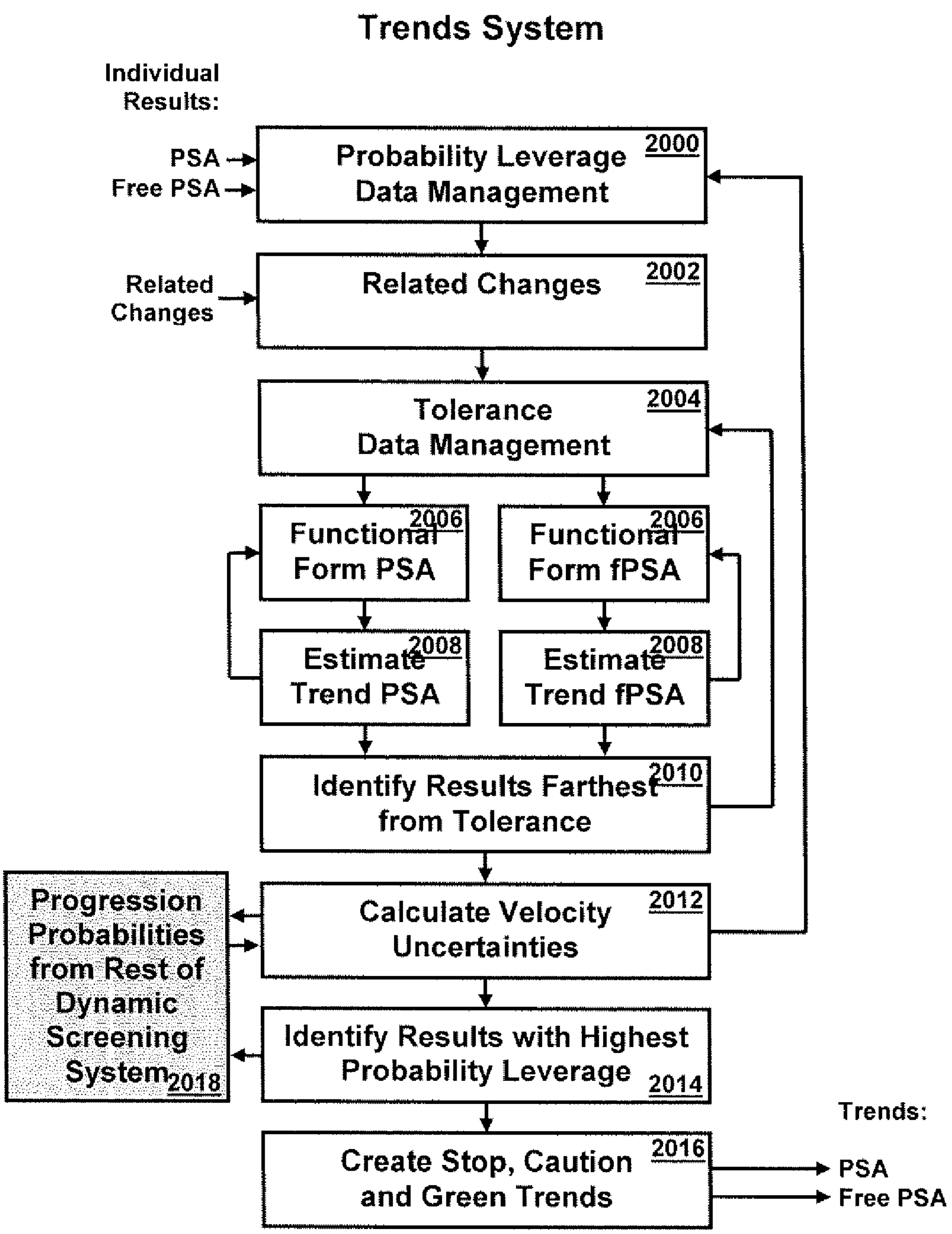
Trends System
Individual Results:
PSA
Free PSA
Probability Leverage Data Management 2000
Related Changes
Related Changes 2002
Tolerance Data Management 2004
Functional Form PSA 2006
Functional Form fPSA 2006
Estimate Trend PSA 2008
Estimate Trend fPSA 2008
Identify Results Farthest from Tolerance 2010
Progression Probabilities from Rest of Dynamic Screening System 2018
Calculate Velocity Uncertainties 2012
Identify Results with Highest Probability Leverage 2014
Trends:
Create Stop, Caution and Green Trends 2016
PSA
Free PSA

FIG. 20

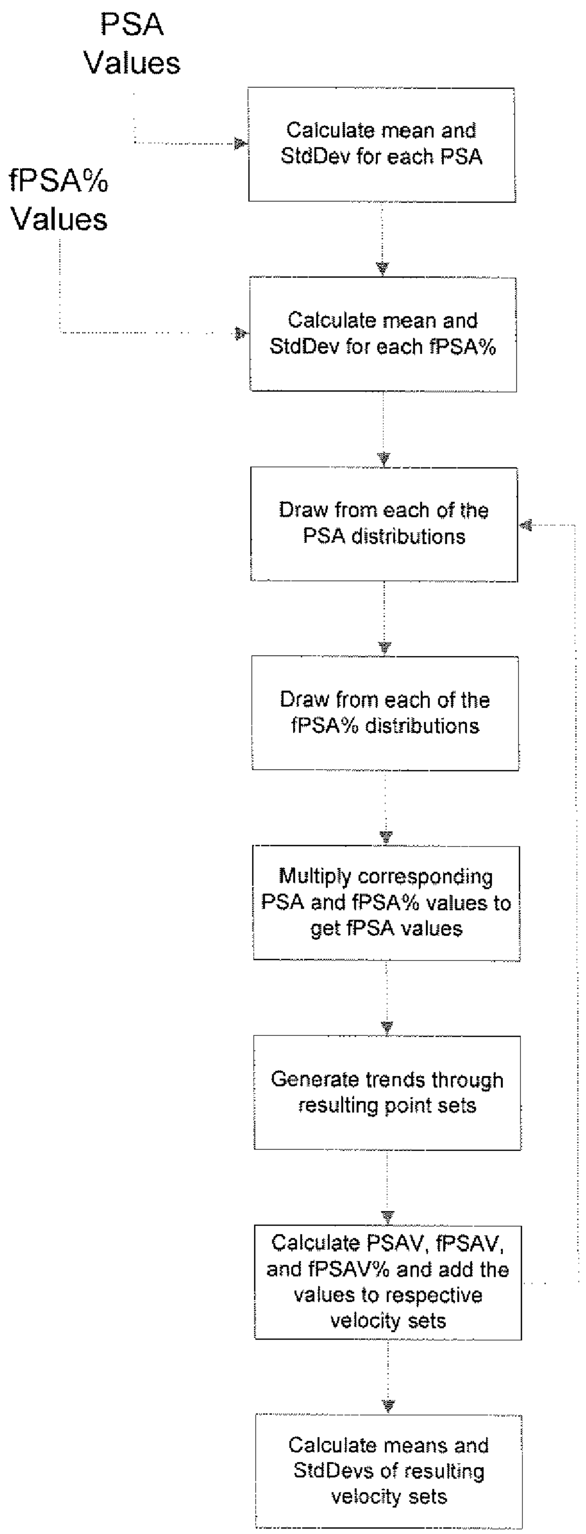
PSA Values
fPSA% Values
Calculate mean and StdDev for each PSA
Calculate mean and StdDev for each fPSA%
Draw from each of the PSA distributions
Draw from each of the fPSA% distributions
Multiply corresponding PSA and fPSA% values to get fPSA values
Generate trends through resulting point sets
Calculate PSAV, fPSAV, and fPSAV% and add the values to respective velocity sets
Calculate means and StdDevs of resulting velocity sets

FIG. 21

## Volume Estimation System for Multiple Volume Measurements

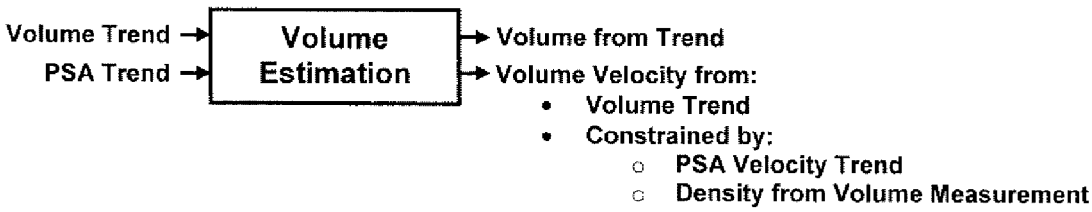


FIG. 22

## Volume Estimation System for One Volume Measurement

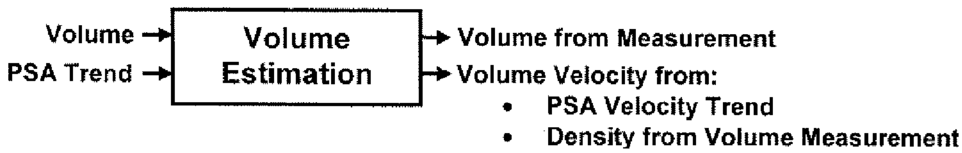


FIG. 23

## Volume Estimation System from PSA Trend – No Volume Measuremen

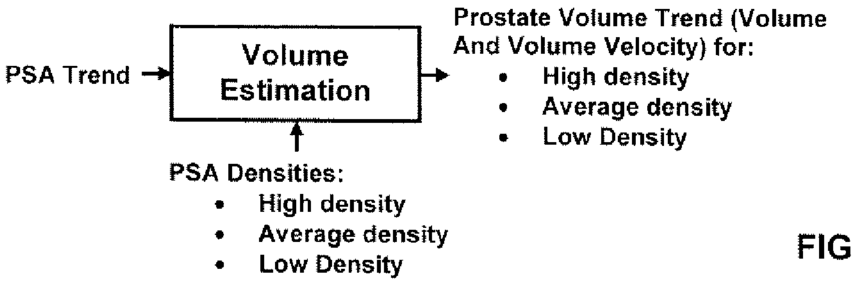


FIG. 24

## Prediction Generators for No Progressing Cancer

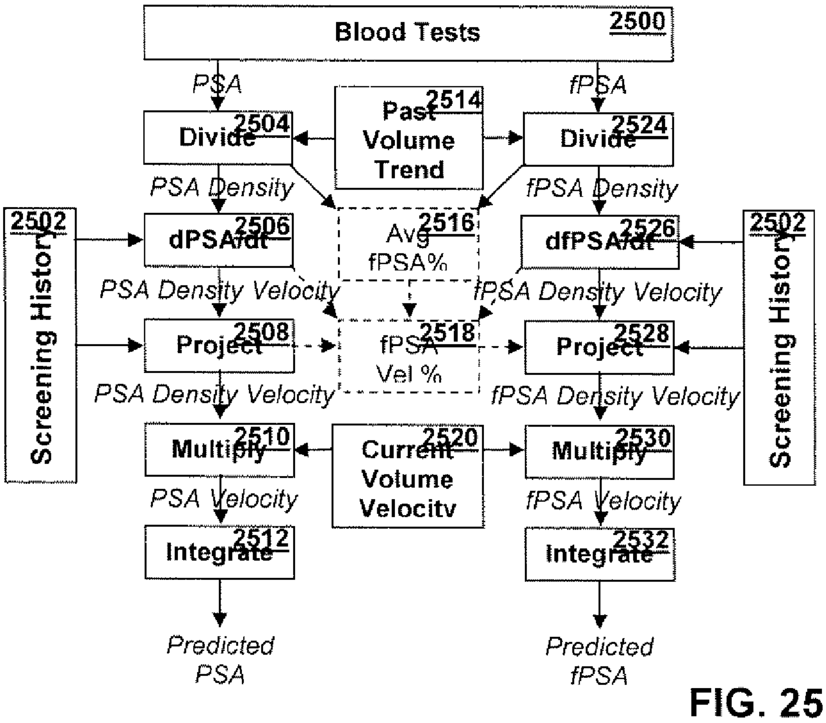


FIG. 25

## Prediction Generators for No Progressing Cancer

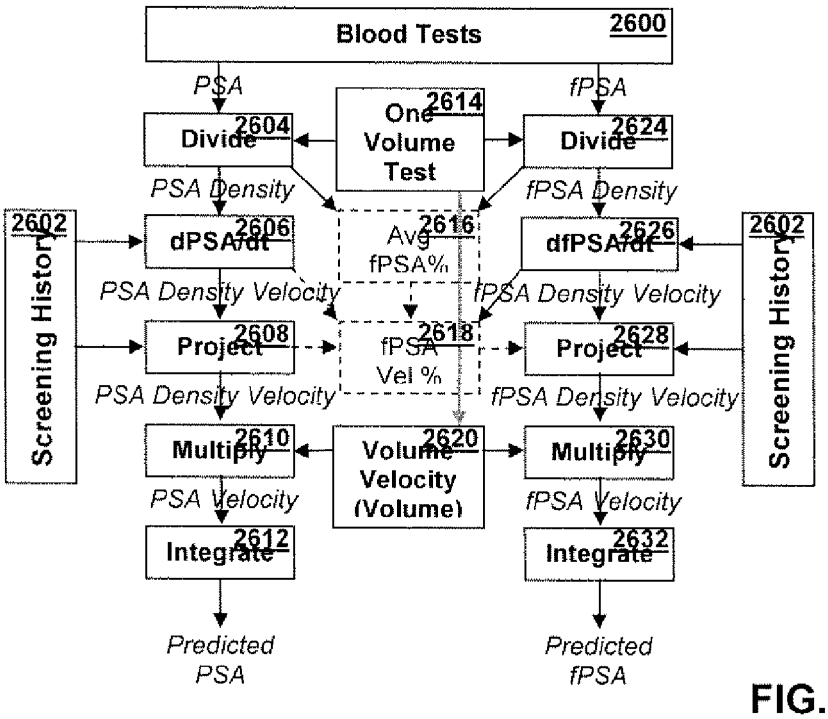


FIG. 26

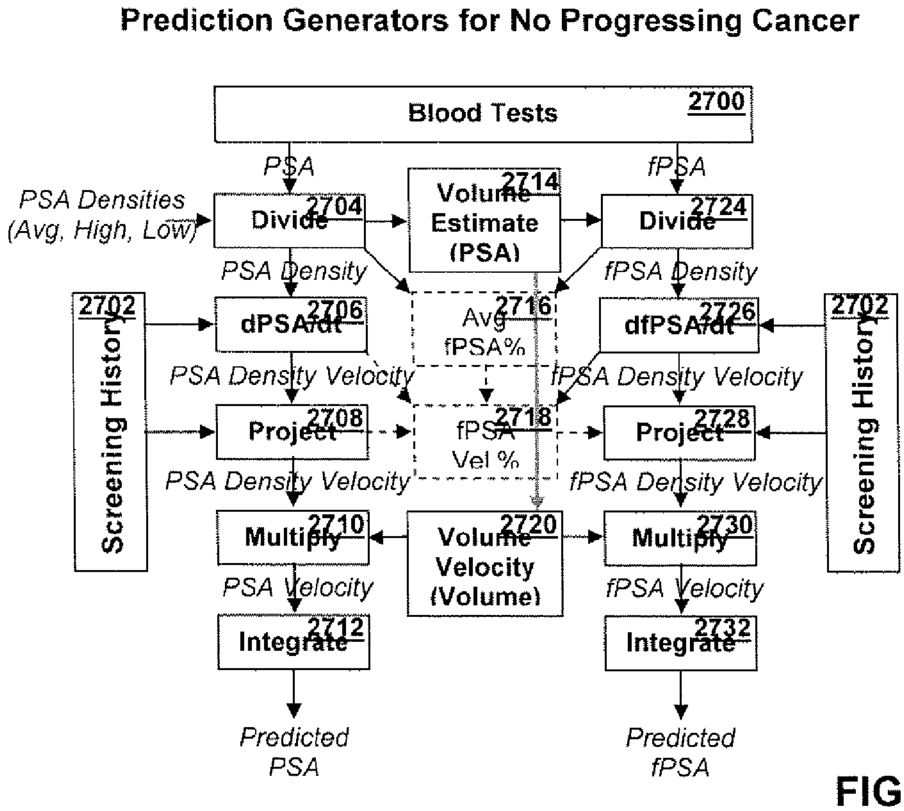
Prediction Generators for No Progressing Cancer
Blood Tests 2700
PSA
fPSA
PSA Densities (Avg, High, Low)
Divide 2704
Volume Estimate (PSA) 2714
Divide 2724
PSA Density
fPSA Density
2702
Screening History
dPSA/dt 2706
Avg fPSA% 2716
dfPSA/dt 2726
PSA Density Velocity
fPSA Density Velocity
Project 2708
fPSA Vel % 2718
Project 2728
Multiply 2710
Volume Velocity (Volume) 2720
Multiply 2730
PSA Velocity
fPSA Velocity
Integrate 2712
Integrate 2732
Predicted PSA
Predicted fPSA

FIG. 27

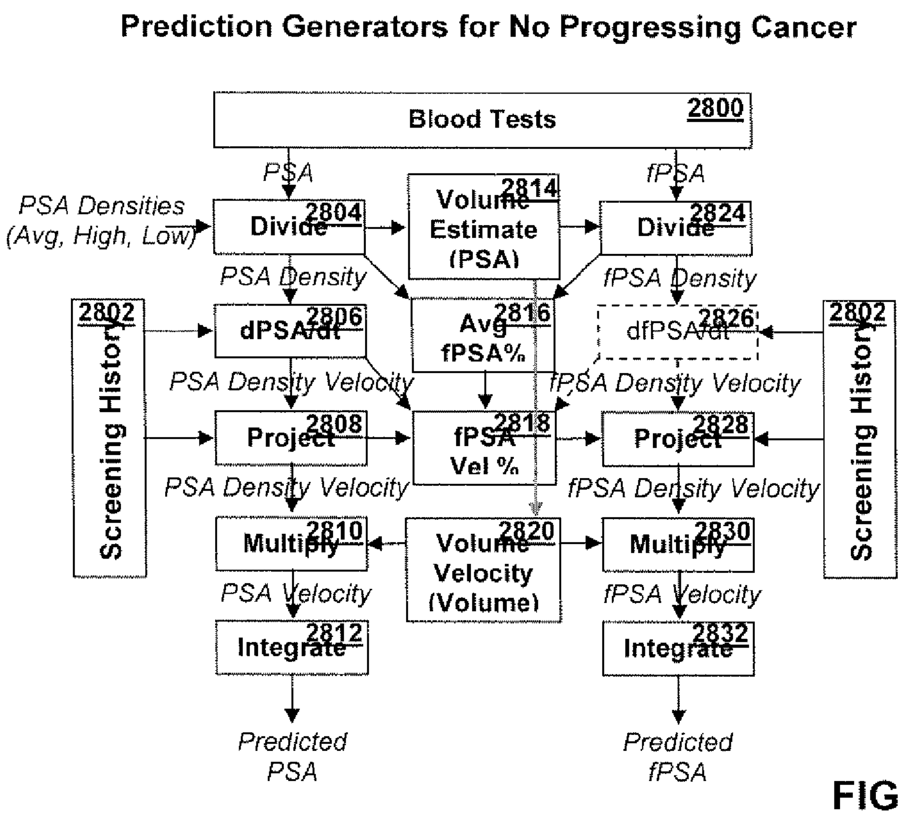
Prediction Generators for No Progressing Cancer
Blood Tests 2800
PSA
fPSA
PSA Densities (Avg, High, Low)
Divide 2804
Volume Estimate (PSA) 2814
Divide 2824
PSA Density
fPSA Density
2802
Screening History
dPSA/dt 2806
Avg fPSA% 2816
dfPSA/dt 2826
PSA Density Velocity
fPSA Density Velocity
Project 2808
fPSA Vel % 2818
Project 2828
Multiply 2810
Volume Velocity (Volume) 2820
Multiply 2830
PSA Velocity
fPSA Velocity
Integrate 2812
Integrate 2832
Predicted PSA
Predicted fPSA

FIG. 28

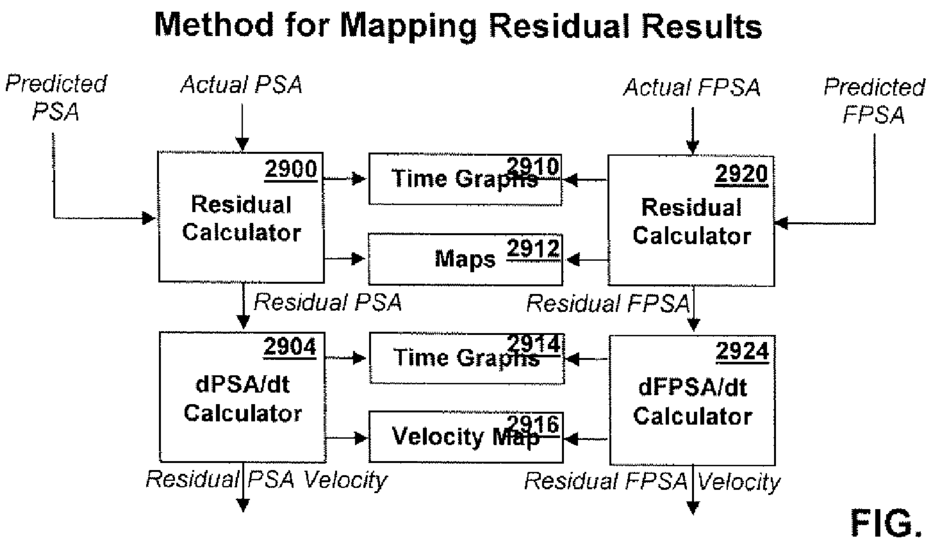
Method for Mapping Residual Results
Predicted PSA
Actual PSA
Actual FPSA
Predicted FPSA
2900
Residual Calculator
Time Graphs 2910
2920
Residual Calculator
Maps 2912
Residual PSA
Residual FPSA
2904
dPSA/dt Calculator
Time Graphs 2914
2924
dFPSA/dt Calculator
Velocity Map 2916
Residual PSA Velocity
Residual FPSA Velocity

FIG. 29

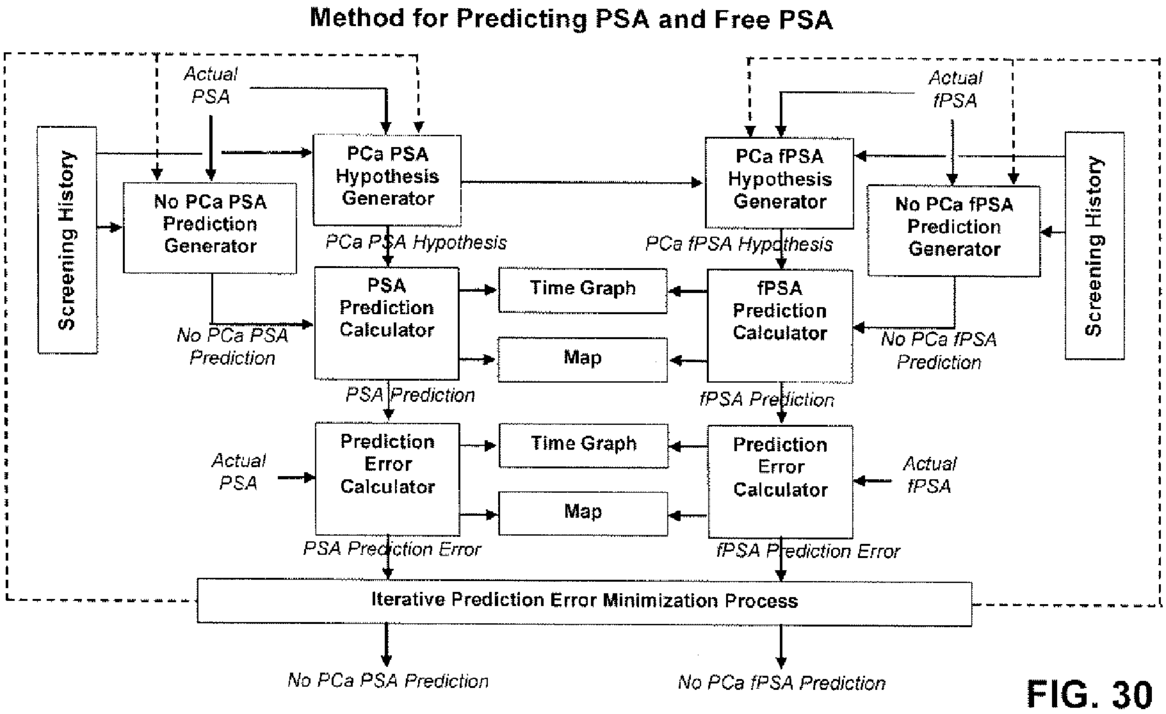
Method for Predicting PSA and Free PSA
Actual PSA
Screening History
No PCa PSA Prediction Generator
PCa PSA Hypothesis Generator
PCa fPSA Hypothesis Generator
Actual fPSA
No PCa fPSA Prediction Generator
Screening History
PCa PSA Hypothesis
PCa fPSA Hypothesis
PSA Prediction Calculator
Time Graph
fPSA Prediction Calculator
No PCa PSA Prediction
Map
No PCa fPSA Prediction
PSA Prediction
fPSA Prediction
Actual PSA
Prediction Error Calculator
Time Graph
Map
Prediction Error Calculator
Actual fPSA
PSA Prediction Error
fPSA Prediction Error
Iterative Prediction Error Minimization Process
No PCa PSA Prediction
No PCa fPSA Prediction

FIG. 30

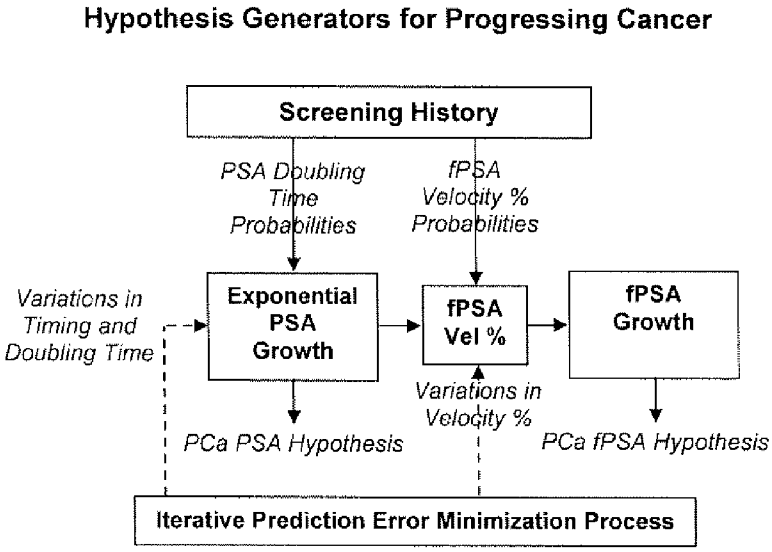


FIG. 31

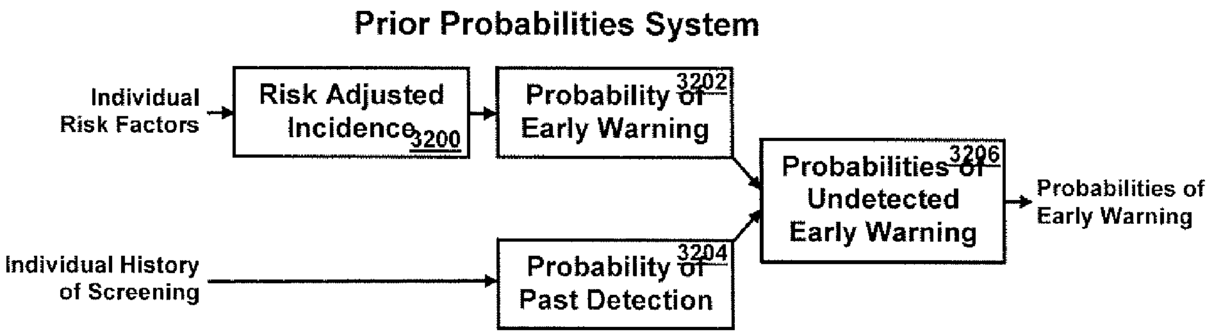


FIG. 32

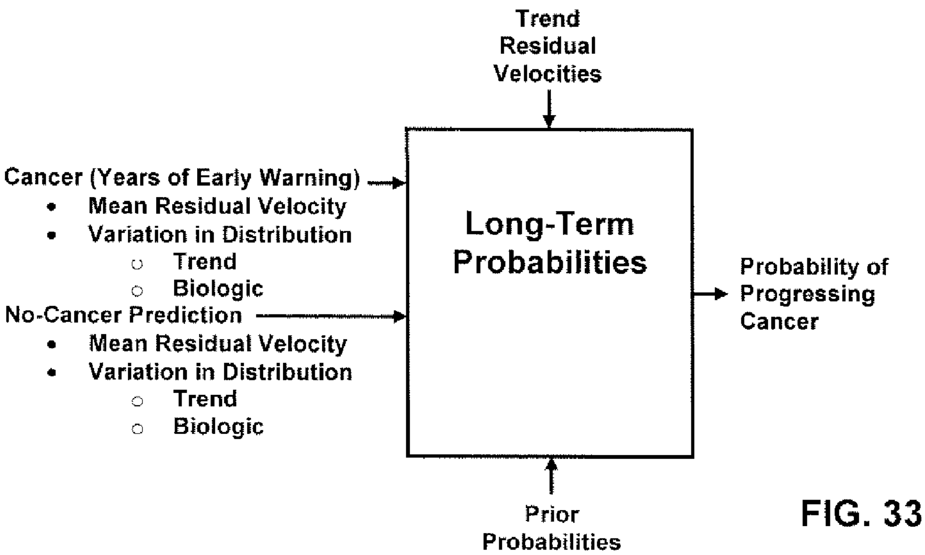


FIG. 33

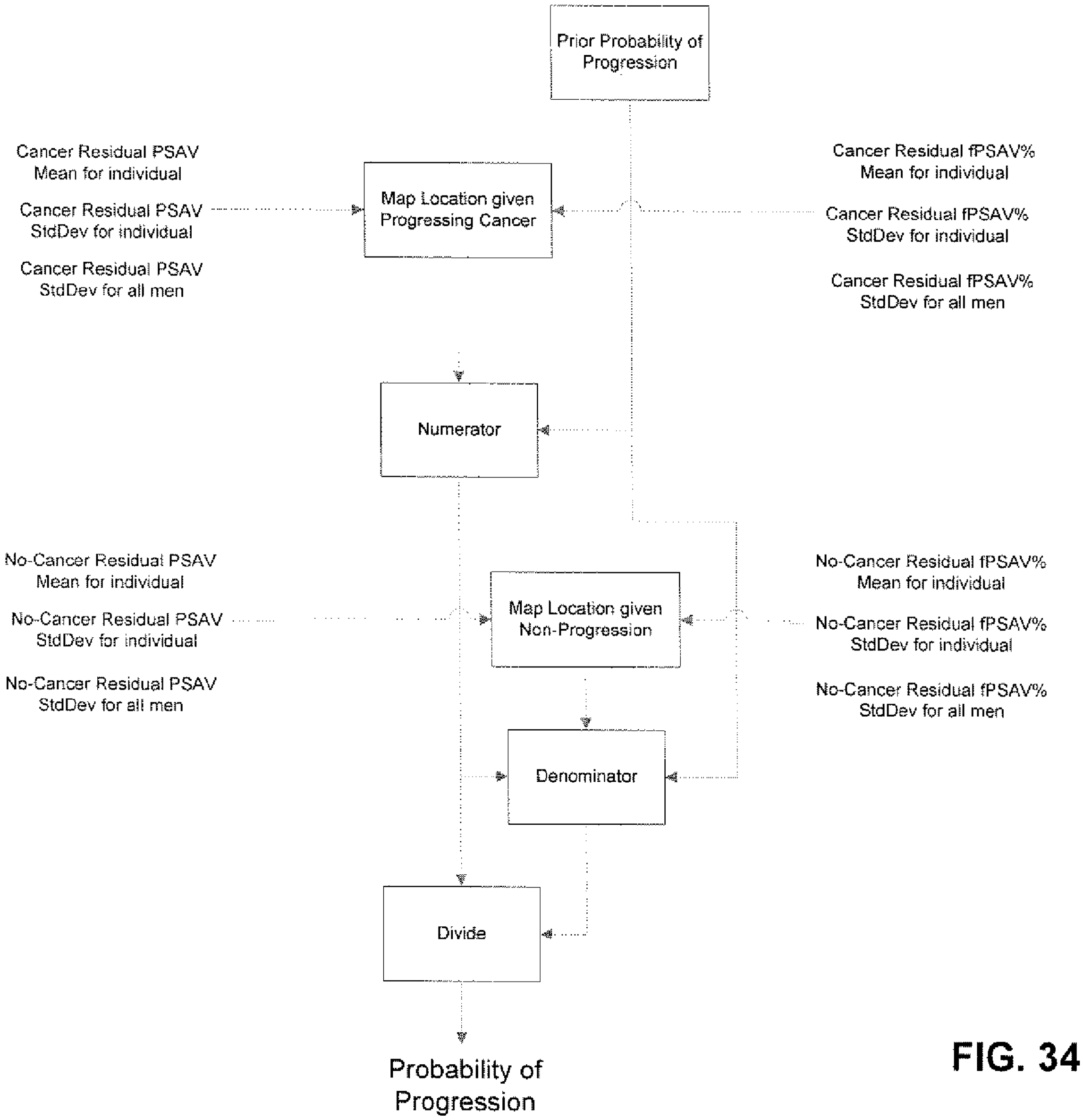
Prior Probability of Progression
Cancer Residual PSAV Mean for individual
Cancer Residual PSAV StdDev for individual
Cancer Residual PSAV StdDev for all men
Map Location given Progressing Cancer
Cancer Residual fPSAV% Mean for individual
Cancer Residual fPSAV% StdDev for individual
Cancer Residual fPSAV% StdDev for all men
Numerator
No-Cancer Residual PSAV Mean for individual
No-Cancer Residual PSAV StdDev for individual
No-Cancer Residual PSAV StdDev for all men
Map Location given Non-Progression
No-Cancer Residual fPSAV% Mean for individual
No-Cancer Residual fPSAV% StdDev for individual
No-Cancer Residual fPSAV% StdDev for all men
Denominator
Divide
Probability of Progression

FIG. 34

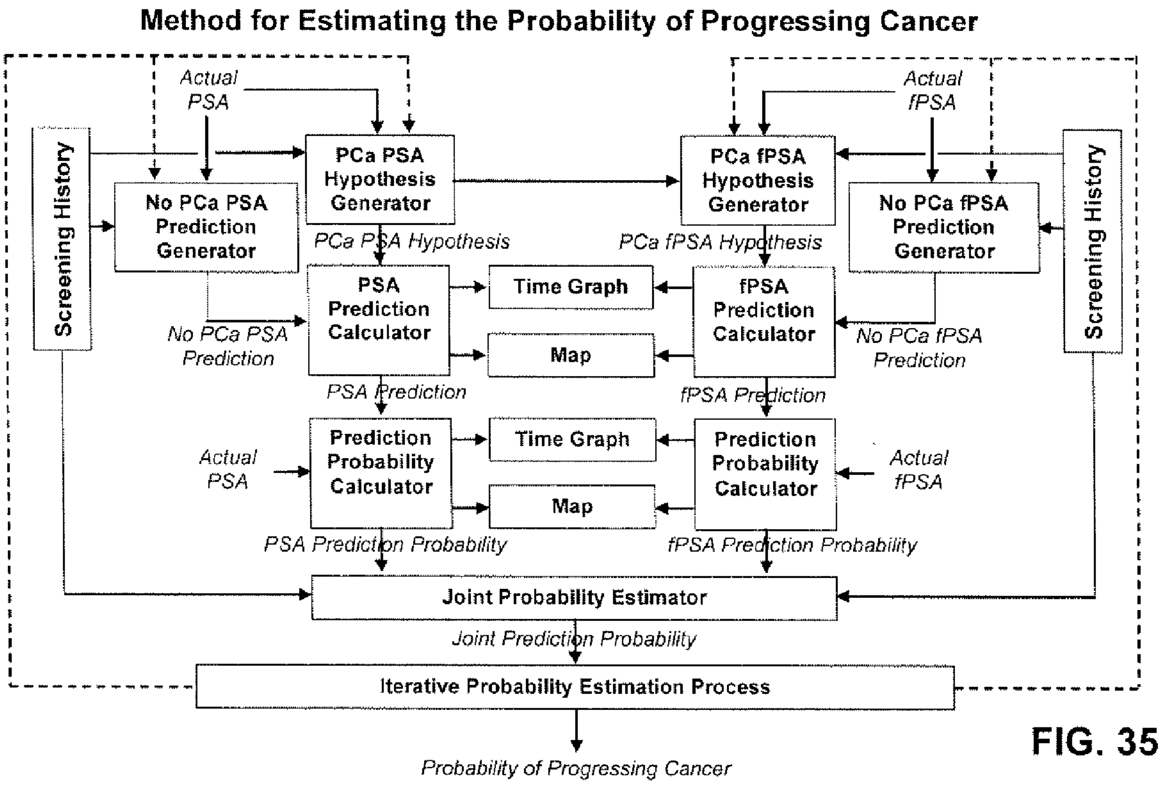
Method for Estimating the Probability of Progressing Cancer
Actual PSA
Screening History
PCa PSA Hypothesis Generator
No PCa PSA Prediction Generator
PCa PSA Hypothesis
PSA Prediction Calculator
No PCa PSA Prediction
Time Graph
Map
PSA Prediction
Actual PSA
Prediction Probability Calculator
Time Graph
Map
PSA Prediction Probability
Actual fPSA
PCa fPSA Hypothesis Generator
No PCa fPSA Prediction Generator
Screening History
PCa fPSA Hypothesis
fPSA Prediction Calculator
No PCa fPSA Prediction
fPSA Prediction
Prediction Probability Calculator
Actual fPSA
fPSA Prediction Probability
Joint Probability Estimator
Joint Prediction Probability
Iterative Probability Estimation Process
Probability of Progressing Cancer

FIG. 35

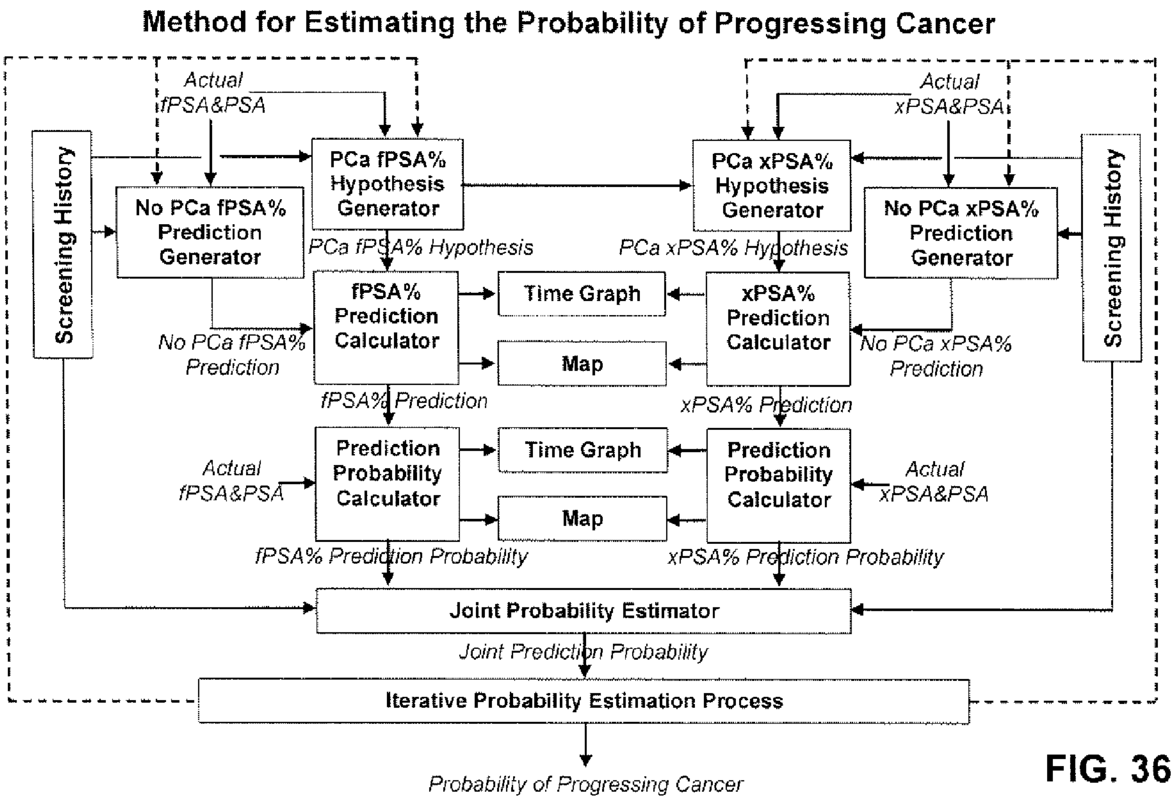
Method for Estimating the Probability of Progressing Cancer
Actual fPSA&PSA
Screening History
PCa fPSA% Hypothesis Generator
No PCa fPSA% Prediction Generator
PCa fPSA% Hypothesis
fPSA% Prediction Calculator
No PCa fPSA% Prediction
Time Graph
Map
fPSA% Prediction
Actual fPSA&PSA
Prediction Probability Calculator
Time Graph
Map
fPSA% Prediction Probability
Actual xPSA&PSA
PCa xPSA% Hypothesis Generator
No PCa xPSA% Prediction Generator
Screening History
PCa xPSA% Hypothesis
xPSA% Prediction Calculator
No PCa xPSA% Prediction
xPSA% Prediction
Prediction Probability Calculator
Actual xPSA&PSA
xPSA% Prediction Probability
Joint Probability Estimator
Joint Prediction Probability
Iterative Probability Estimation Process
Probability of Progressing Cancer

FIG. 36

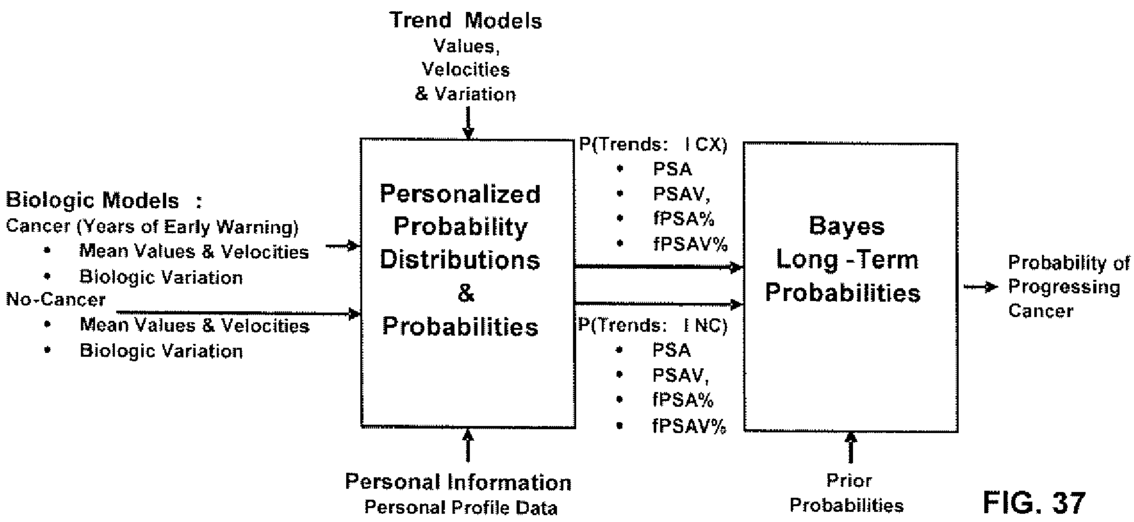
Trend Models
Values,
Velocities
& Variation
Biologic Models :
Cancer (Years of Early Warning)
• Mean Values & Velocities
• Biologic Variation
No-Cancer
• Mean Values & Velocities
• Biologic Variation
Personalized
Probability
Distributions
&
Probabilities
P(Trends: I CX)
• PSA
• PSAV,
• fPSA%
• fPSAV%
P(Trends: I NC)
• PSA
• PSAV,
• fPSA%
• fPSAV%
Bayes
Long -Term
Probabilities
Probability of
Progressing
Cancer
Personal Information
Personal Profile Data
Prior
Probabilities

FIG. 37

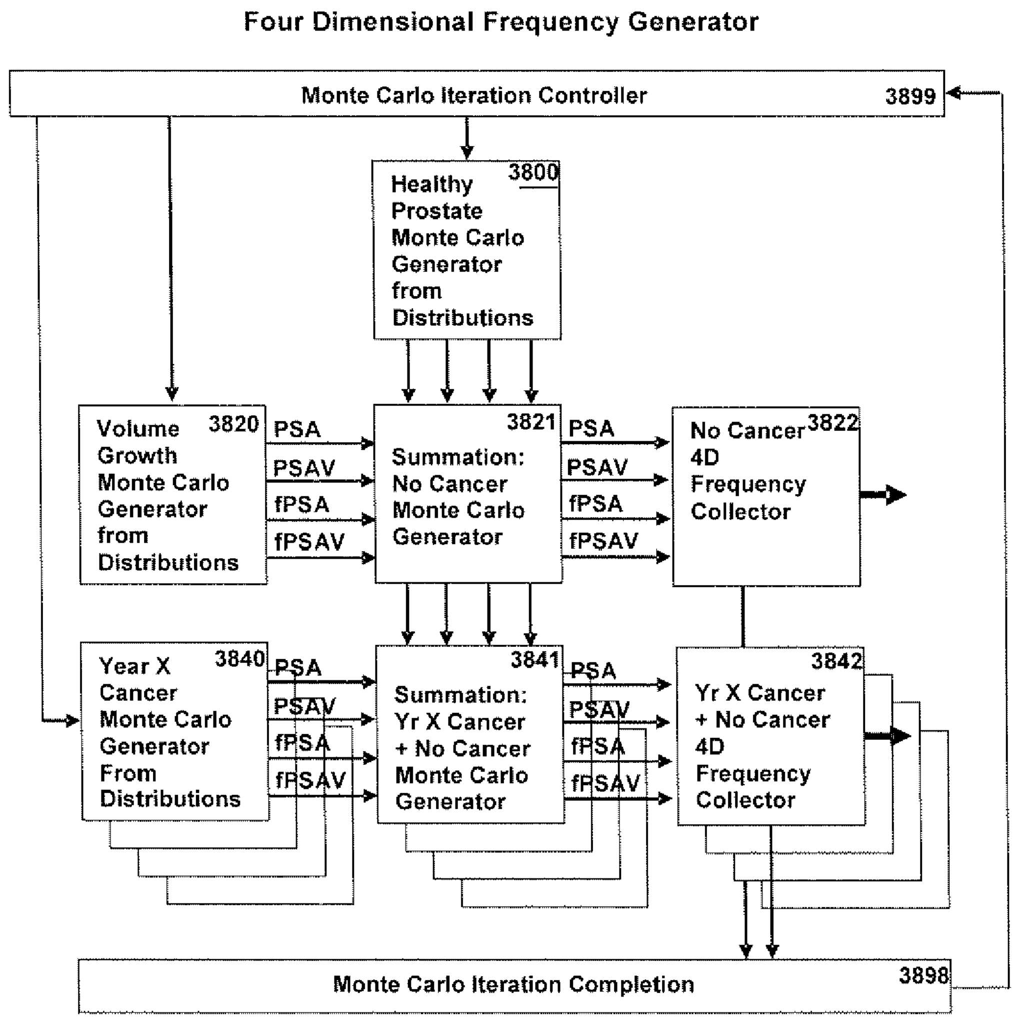

Four Dimensional Frequency Generator
Monte Carlo Iteration Controller
3899
Healthy Prostate Monte Carlo Generator from Distributions
3800
Volume Growth Monte Carlo Generator from Distributions
3820
PSA
PSAV
fPSA
fPSAV
Summation: No Cancer Monte Carlo Generator
3821
No Cancer 4D Frequency Collector
3822
Year X Cancer Monte Carlo Generator From Distributions
3840
Summation: Yr X Cancer + No Cancer Monte Carlo Generator
3841
Yr X Cancer + No Cancer 4D Frequency Collector
3842
Monte Carlo Iteration Completion
3898


FIG. 38

No Cancer Four Dimensional Frequency Generator

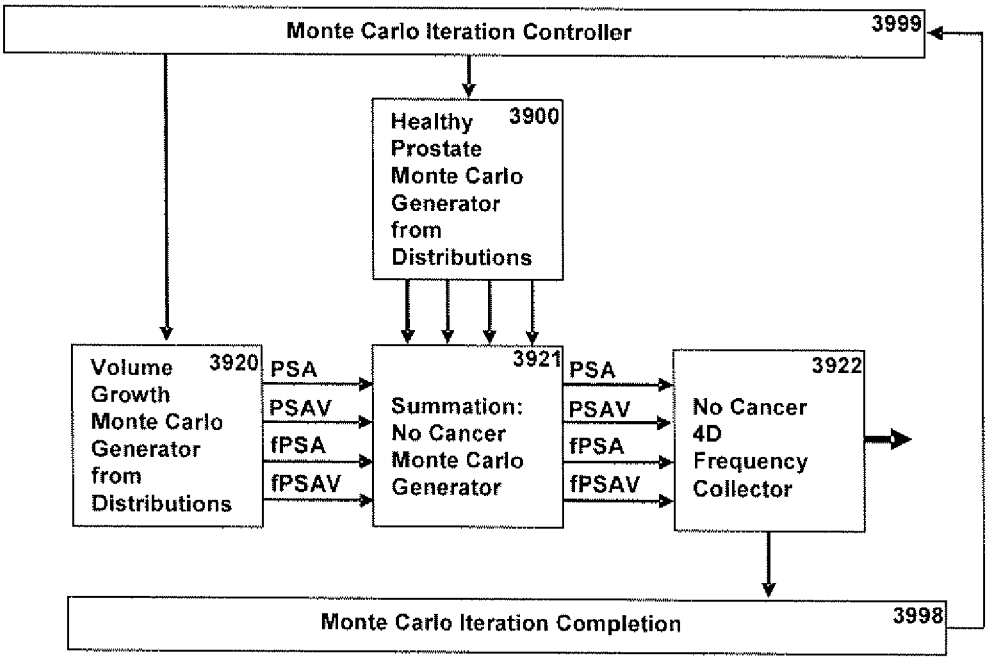


FIG. 39

Cancer Plus No Cancer Four Dimensional Frequency Generator

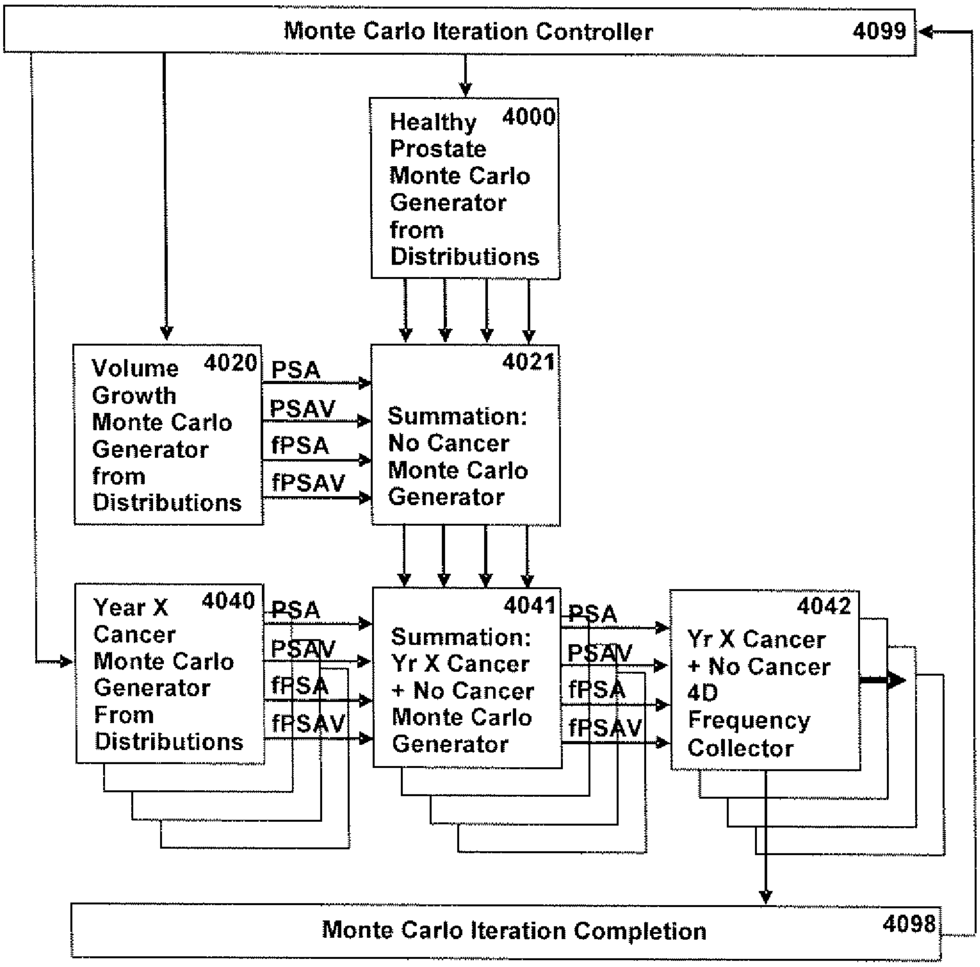


FIG. 40

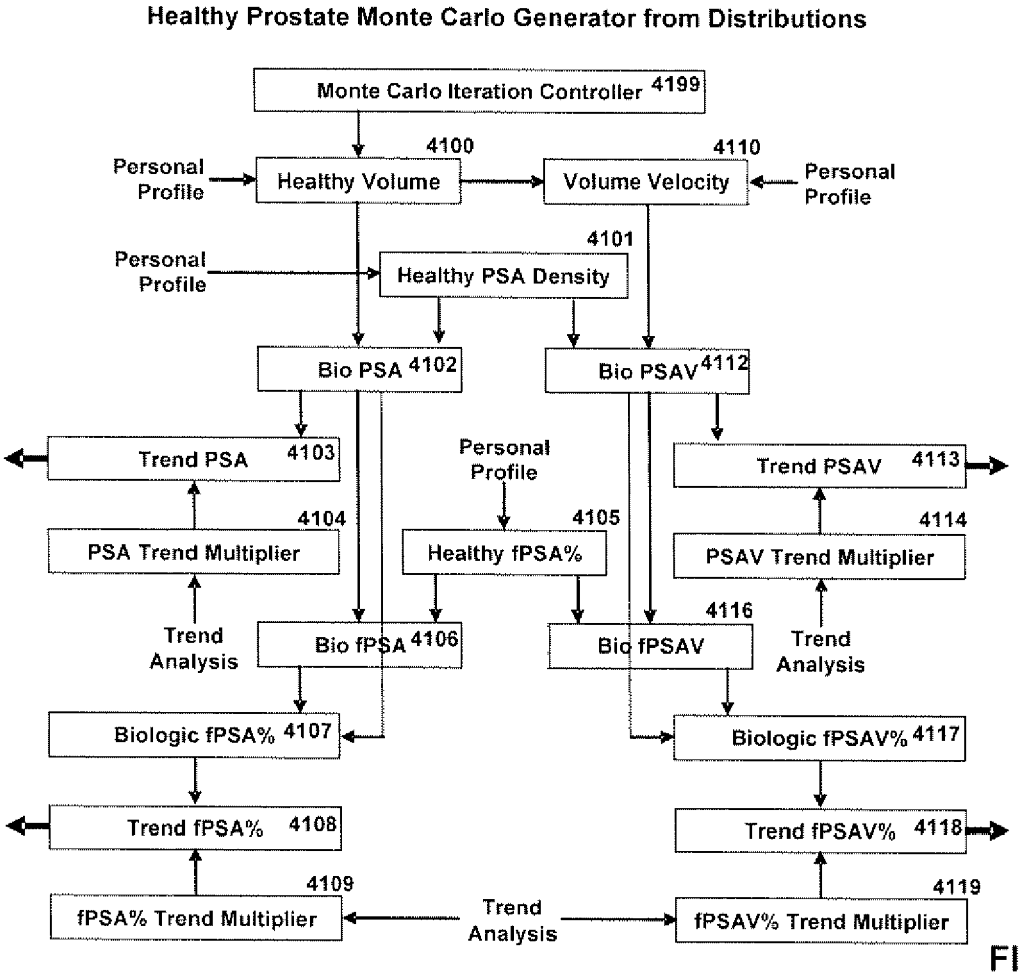
Healthy Prostate Monte Carlo Generator from Distributions
Monte Carlo Iteration Controller 4199
4100
Personal Profile
Healthy Volume
4110
Volume Velocity
Personal Profile
4101
Personal Profile
Healthy PSA Density
Bio PSA 4102
Bio PSAV 4112
Trend PSA 4103
Personal Profile
Trend PSAV 4113
4104
PSA Trend Multiplier
4105
Healthy fPSA%
4114
PSAV Trend Multiplier
Trend Analysis
Bio fPSA 4106
4116
Bio fPSAV
Trend Analysis
Biologic fPSA% 4107
Biologic fPSAV% 4117
Trend fPSA% 4108
Trend fPSAV% 4118
4109
fPSA% Trend Multiplier
Trend Analysis
4119
fPSAV% Trend Multiplier

FIG. 41

Volume Growth Monte Carlo Generator from Distributions

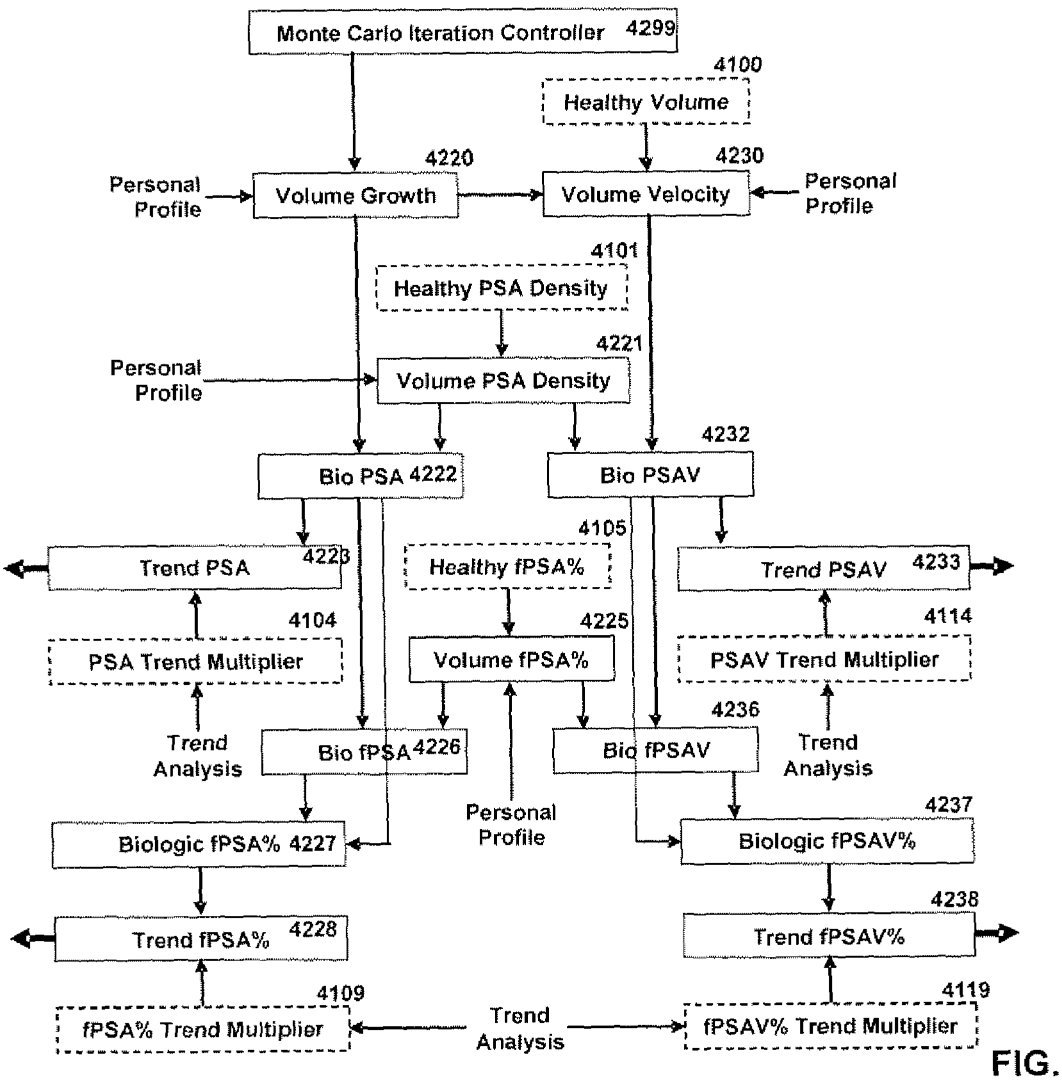


FIG. 42

Year X Cancer Monte Carlo Generator from Distributions

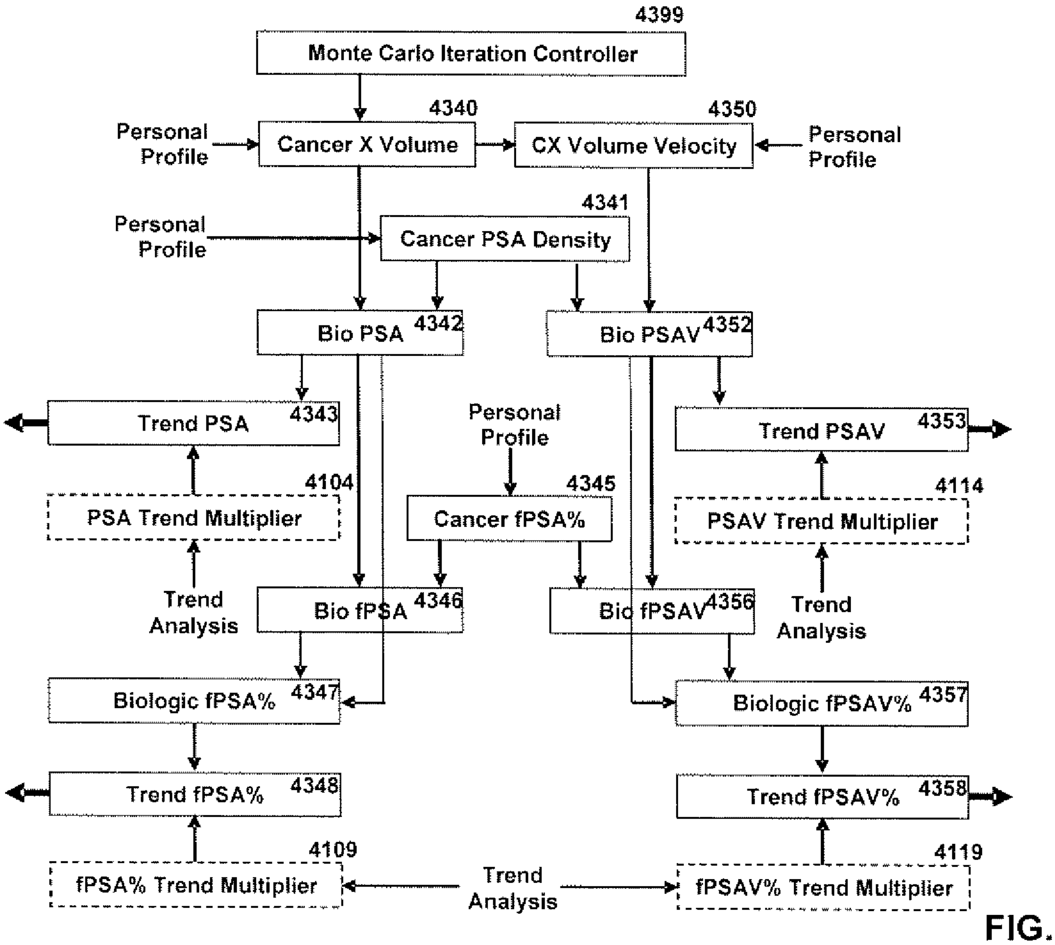


FIG. 43

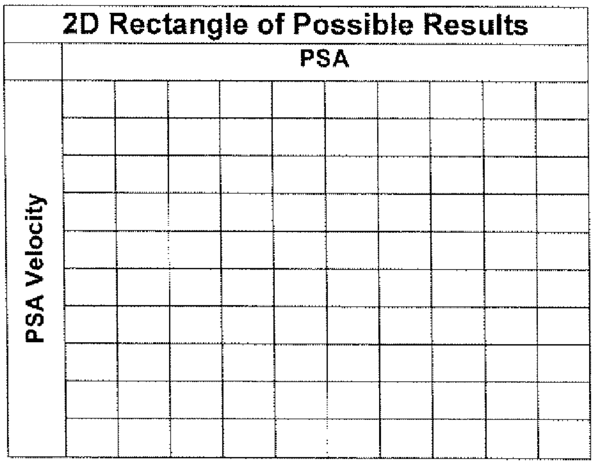


FIG. 44

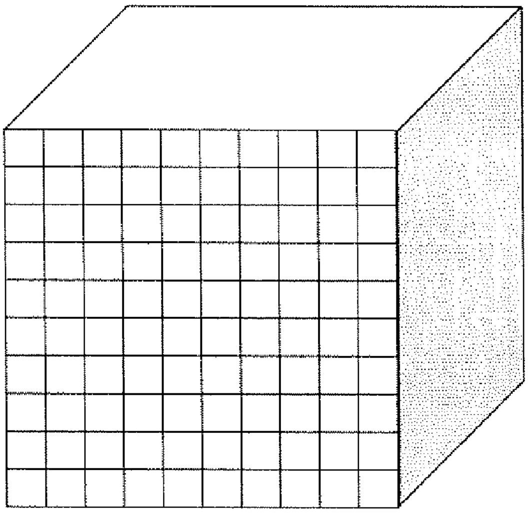

FIG. 45

| Required Monte Carlo Iterations | | | | | |
|---|---|---|---|---|---|
| Tests | Dimensions | Segments | Buckets | Avg Freq | Iterations |
| 1 | 2 | 10 | 100 | 100 | 10,000 |
| 2 | 4 | 10 | 10,000 | 100 | 1,000,000 |
| 3 | 6 | 10 | 1,000,000 | 100 | 100,000,000 |
| 4 | 8 | 10 | 100,000,000 | 100 | 10,000,000,000 |
| 5 | 10 | 10 | 10,000,000,000 | 100 | 1,000,000,000,000 |

FIG. 46

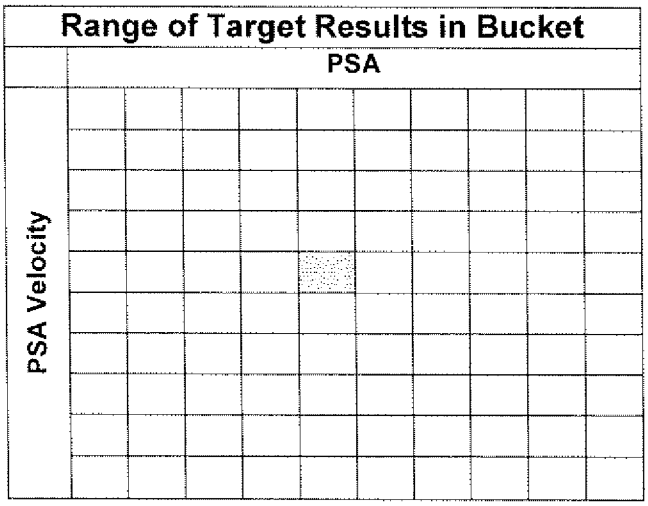


FIG. 47

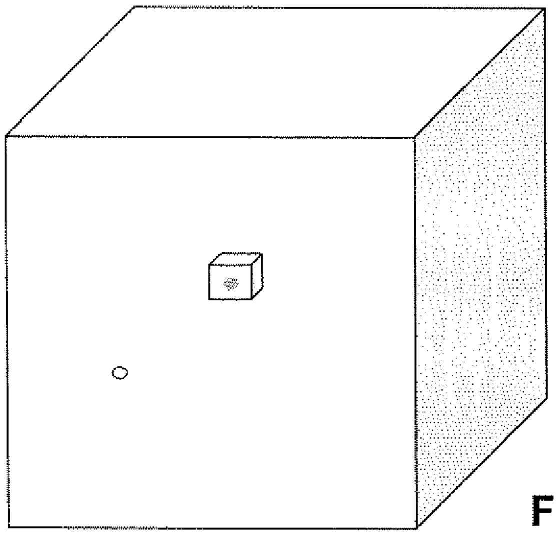

FIG. 48

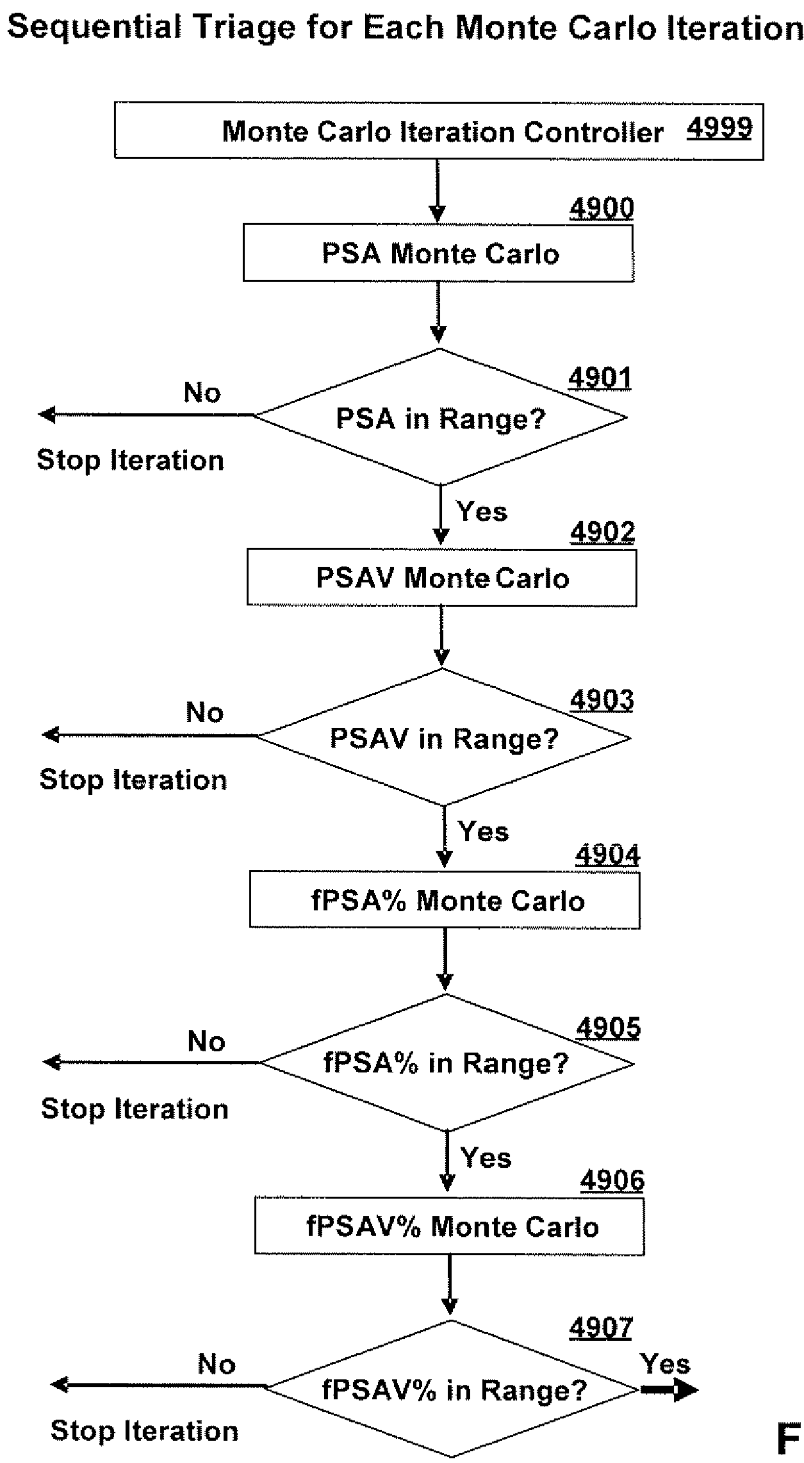
Sequential Triage for Each Monte Carlo Iteration
Monte Carlo Iteration Controller 4999
4900
PSA Monte Carlo
4901
PSA in Range?
No
Stop Iteration
Yes
4902
PSAV Monte Carlo
4903
PSAV in Range?
No
Stop Iteration
Yes
4904
fPSA% Monte Carlo
4905
fPSA% in Range?
No
Stop Iteration
Yes
4906
fPSAV% Monte Carlo
4907
fPSAV% in Range?
No
Stop Iteration
Yes

FIG. 49

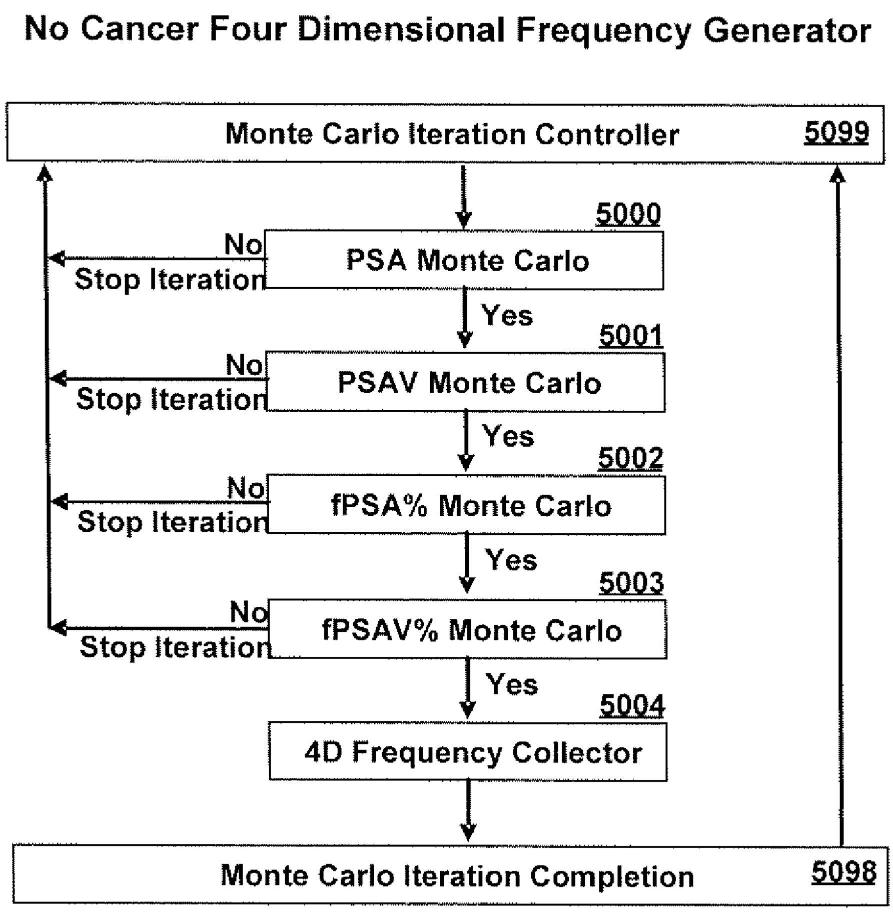
No Cancer Four Dimensional Frequency Generator
Monte Carlo Iteration Controller
5099
5000
PSA Monte Carlo
No
Stop Iteration
Yes
5001
PSAV Monte Carlo
No
Stop Iteration
Yes
5002
fPSA% Monte Carlo
No
Stop Iteration
Yes
5003
fPSAV% Monte Carlo
No
Stop Iteration
Yes
5004
4D Frequency Collector
Monte Carlo Iteration Completion
5098

FIG. 50

No Cancer PSA Monte Carlo Generator from Distributions

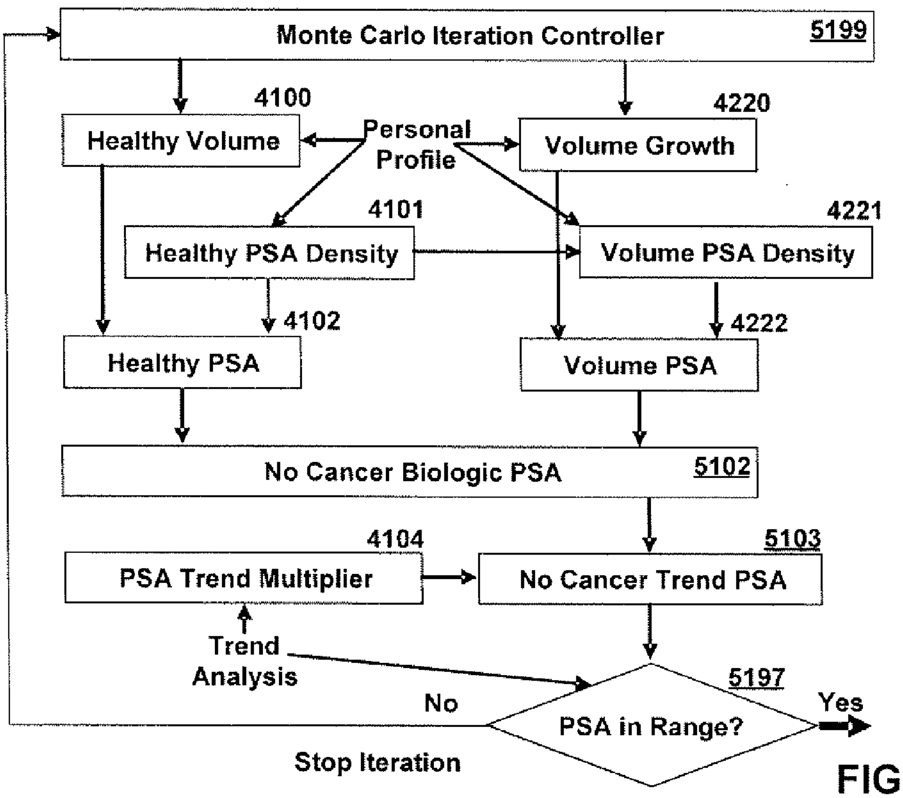


FIG. 51

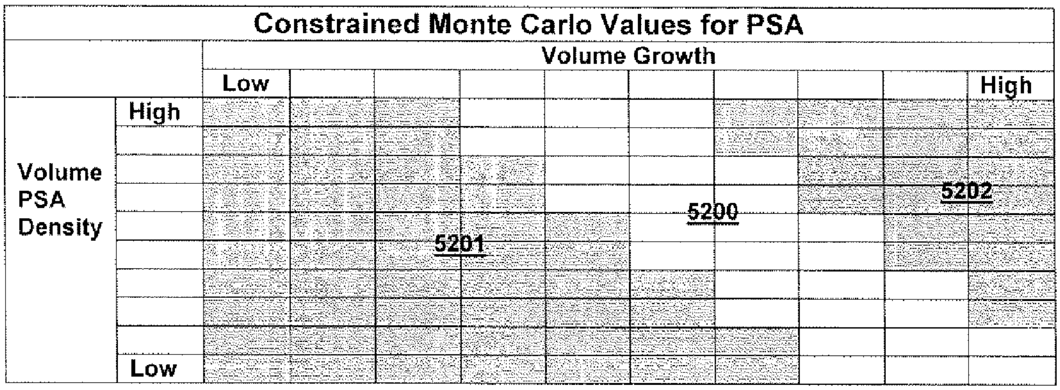


FIG. 52

No Cancer PSAV Monte Carlo Generator from Distributions

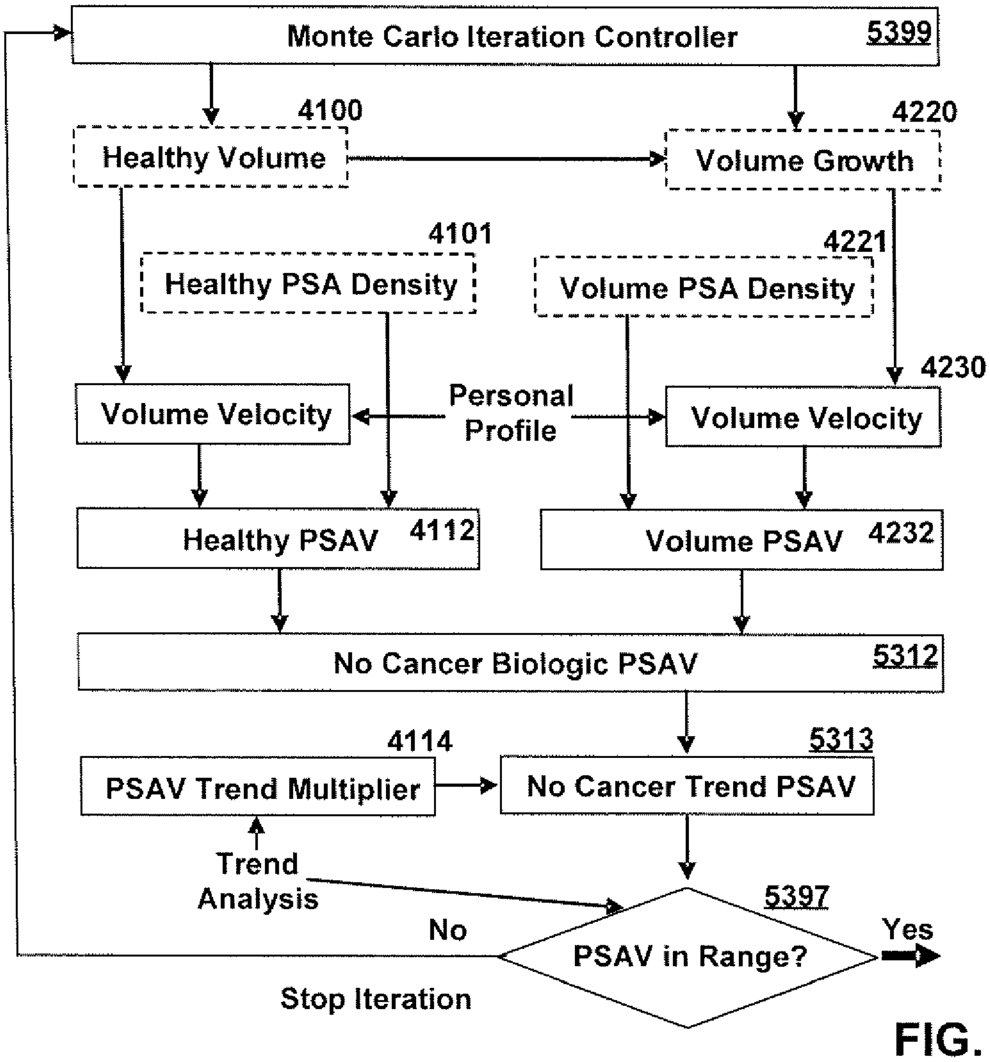


FIG. 53

No Cancer fPSA Monte Carlo Generator from Distributions

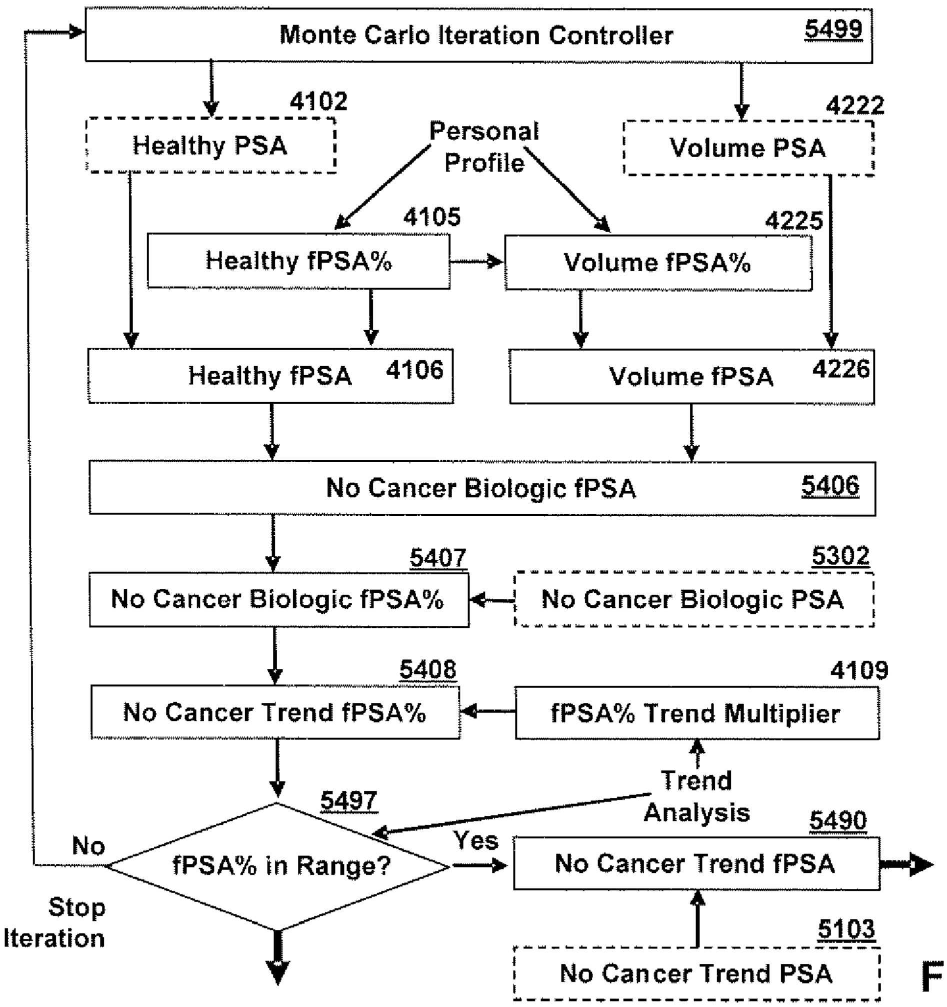


FIG. 54

No Cancer fPSAV Monte Carlo Generator from Distributions

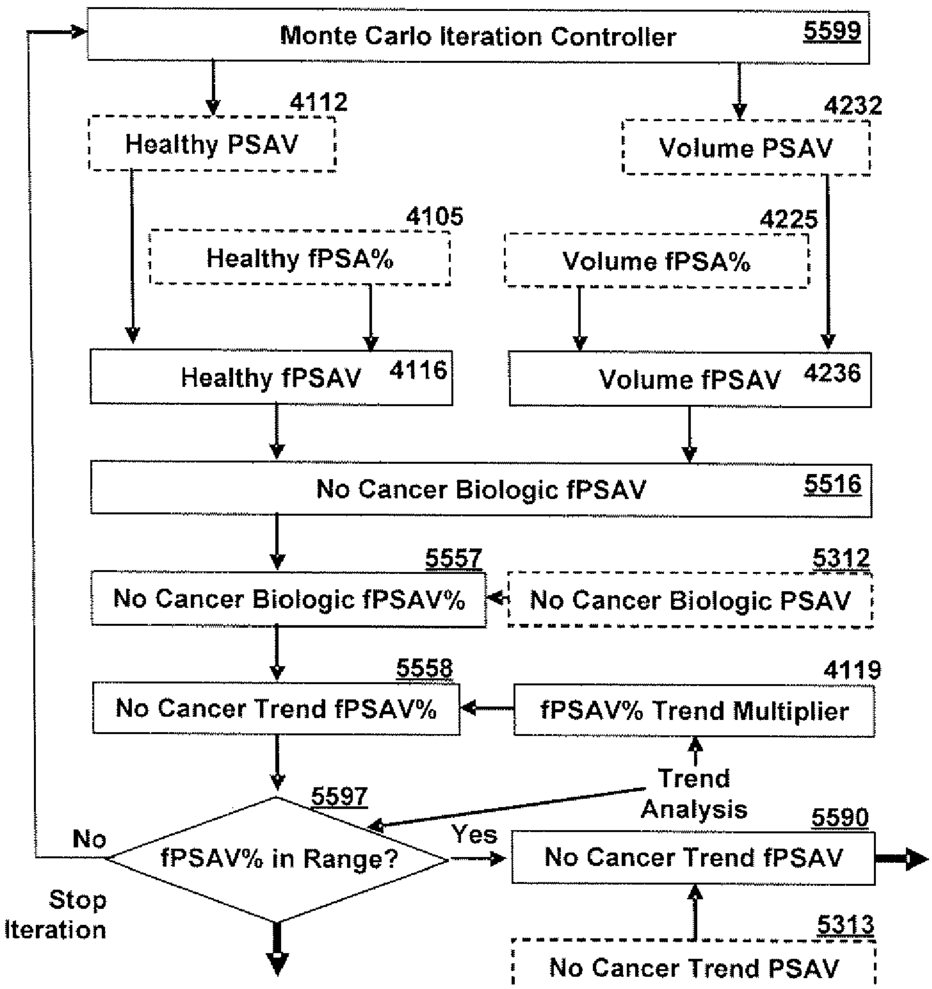


FIG. 55

Cancer Plus No Cancer Four Dimensional Frequency Generator

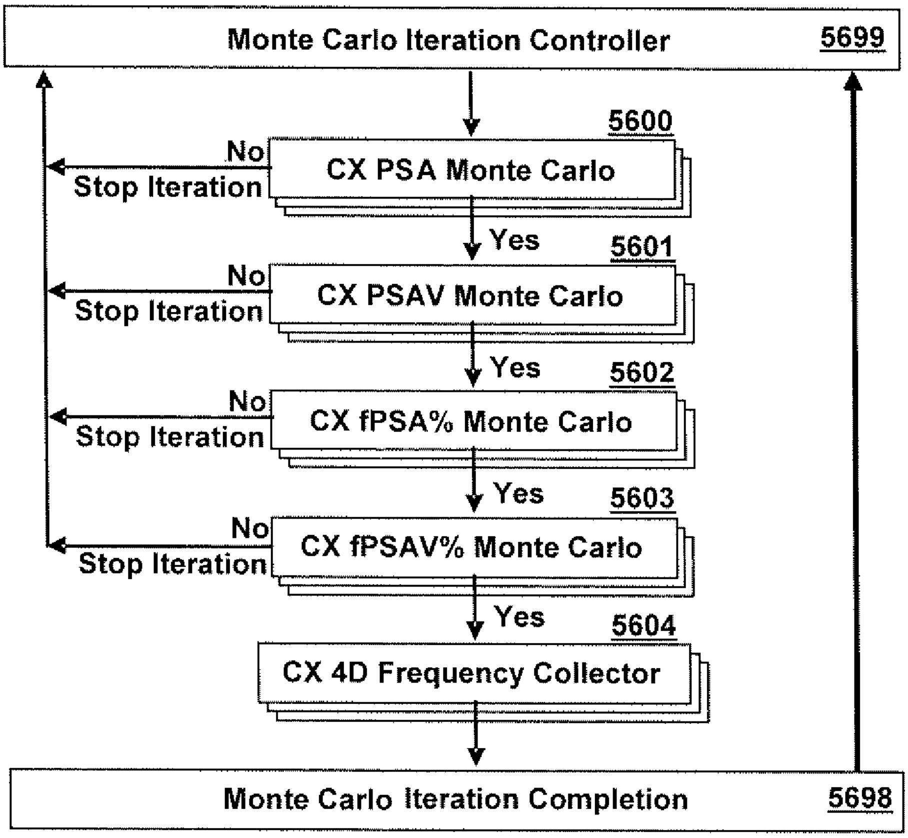


FIG. 56

Year X Cancer Plus No Cancer PSA Monte Carlo Generator

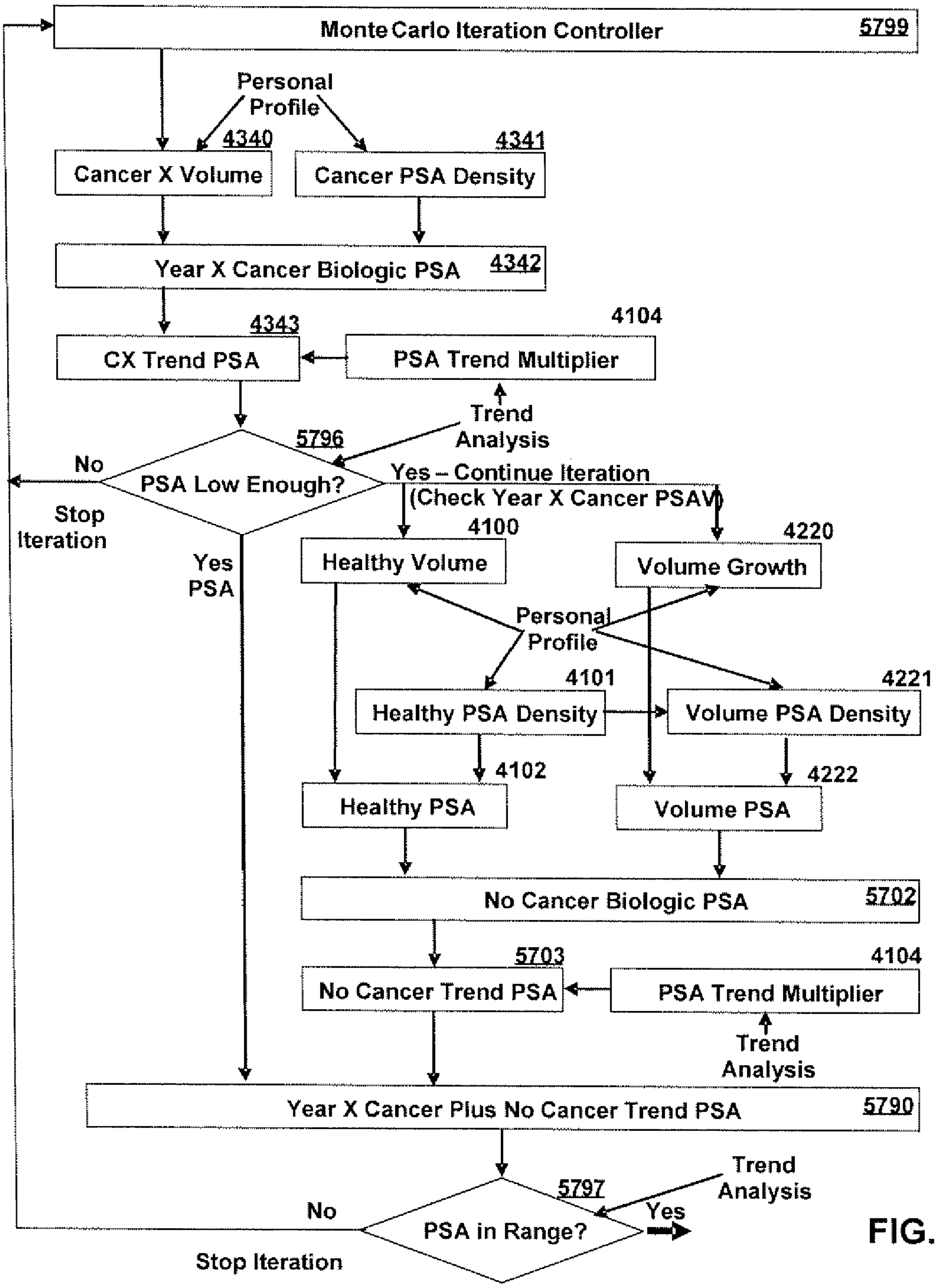


FIG. 57

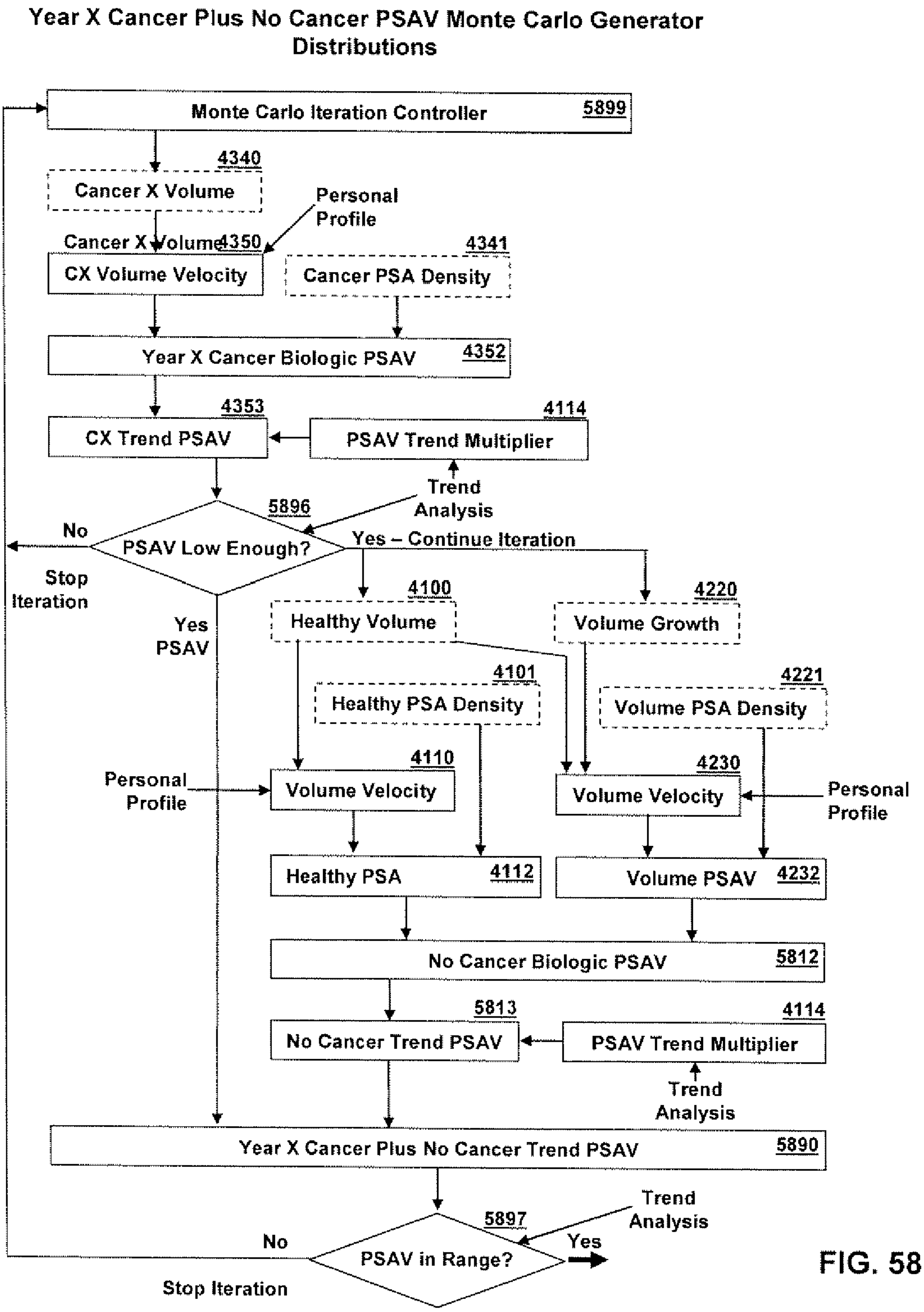
Year X Cancer Plus No Cancer PSAV Monte Carlo Generator Distributions
Monte Carlo Iteration Controller 5899
4340
Cancer X Volume
Personal Profile
Cancer X Volume
4350
4341
CX Volume Velocity
Cancer PSA Density
Year X Cancer Biologic PSAV 4352
4353
4114
CX Trend PSAV
PSAV Trend Multiplier
Trend Analysis
5896
No
PSAV Low Enough?
Yes – Continue Iteration
Stop Iteration
Yes PSAV
4100
Healthy Volume
4220
Volume Growth
4101
Healthy PSA Density
4221
Volume PSA Density
4110
Personal Profile
Volume Velocity
4230
Volume Velocity
Personal Profile
Healthy PSA 4112
Volume PSAV 4232
No Cancer Biologic PSAV 5812
5813
4114
No Cancer Trend PSAV
PSAV Trend Multiplier
Trend Analysis
Year X Cancer Plus No Cancer Trend PSAV 5890
Trend Analysis
5897
No
PSAV in Range?
Yes
Stop Iteration

FIG. 58

Year X Cancer Plus No Cancer fPSA Monte Carlo Generator

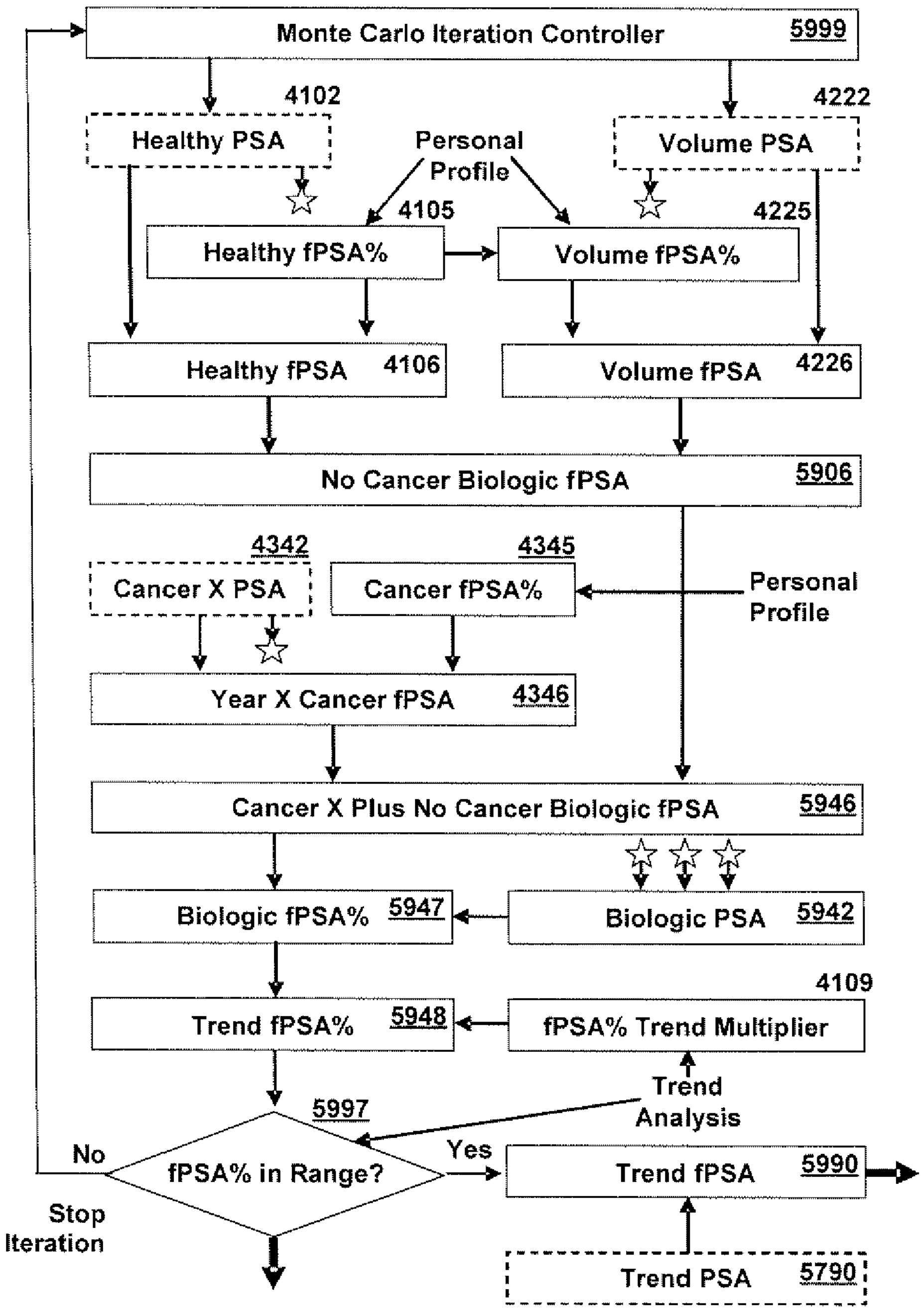


FIG. 59

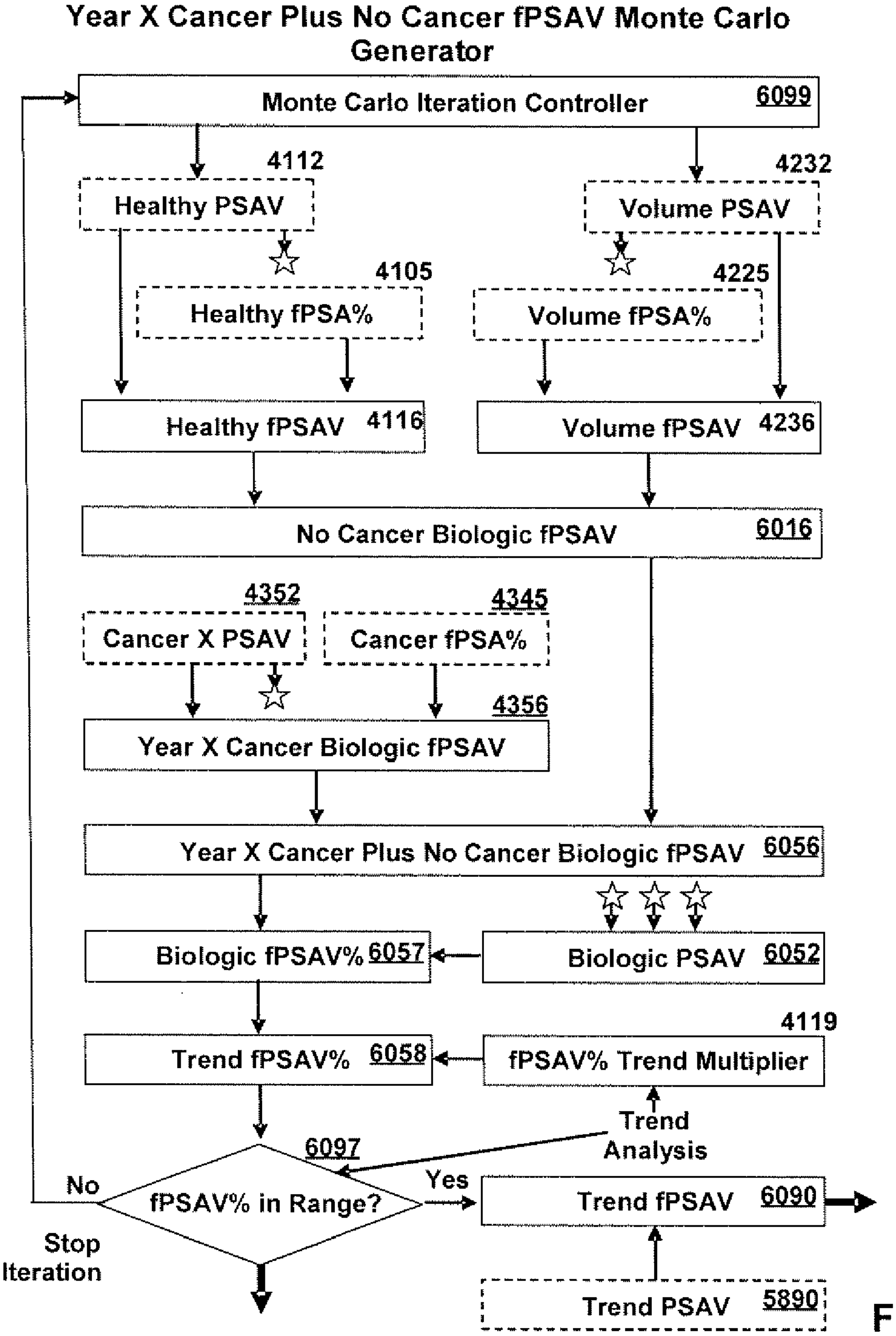
Year X Cancer Plus No Cancer fPSAV Monte Carlo Generator
Monte Carlo Iteration Controller 6099
4112
Healthy PSAV
4232
Volume PSAV
4105
Healthy fPSA%
4225
Volume fPSA%
Healthy fPSAV 4116
Volume fPSAV 4236
No Cancer Biologic fPSAV 6016
4352
Cancer X PSAV
4345
Cancer fPSA%
4356
Year X Cancer Biologic fPSAV
Year X Cancer Plus No Cancer Biologic fPSAV 6056
Biologic fPSAV% 6057
Biologic PSAV 6052
4119
Trend fPSAV% 6058
fPSAV% Trend Multiplier
Trend Analysis
6097
No
fPSAV% in Range?
Yes
Trend fPSAV 6090
Stop Iteration
Trend PSAV 5890

FIG. 60

## Dynamic Screening Custom Content System

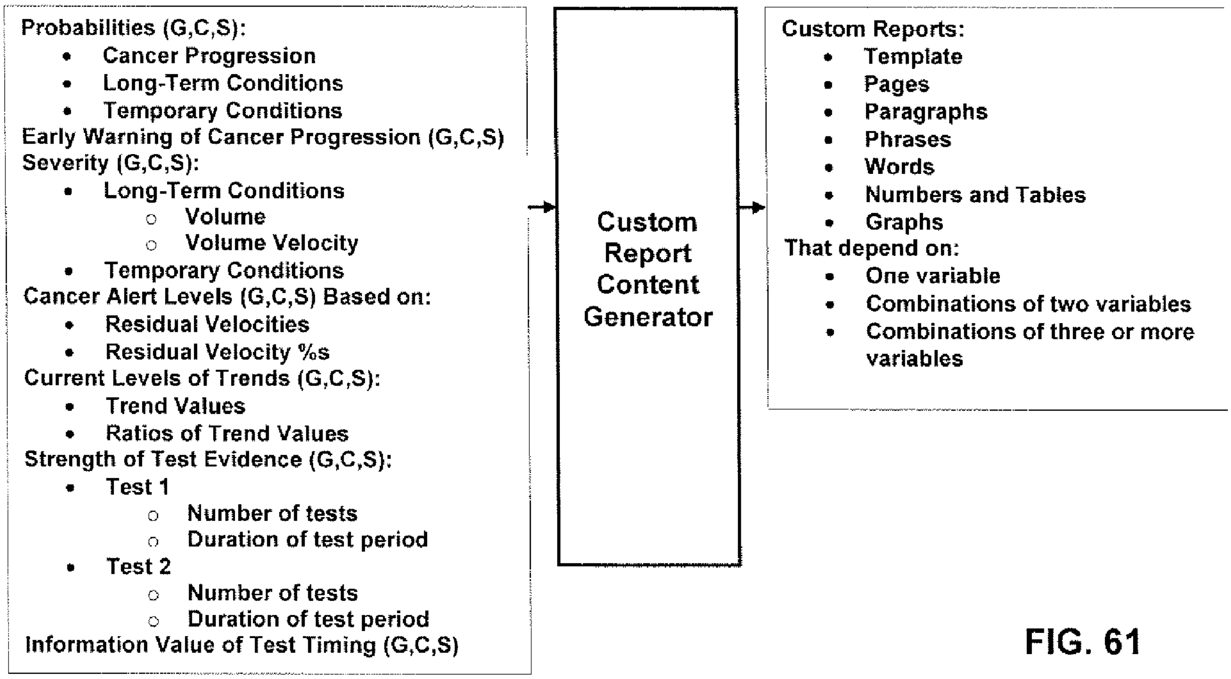


FIG. 61

NOTE: Three cases (G,C,S) are evaluated to provide more robust analysis and custom content:

- Stop Case (S) Shown in Red – Based on all tests that are within tolerance of predicted values. Earliest warning with highest chance of false warning.
- Caution Case (C) Shown in Yellow – Drops most anomalous test pair from trend estimation. Less early warning with less chance of false warning.
- Green Case (C) – Drops two most anomalous test pairs from trend estimation. Least early warning with least chance of false warning.

Overall and Detailed Feedback Learning

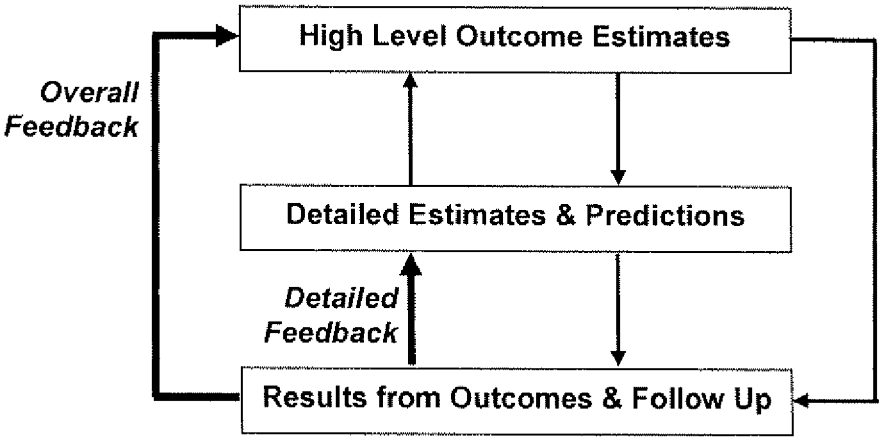


FIG. 62

Feedback Learning Flow Chart

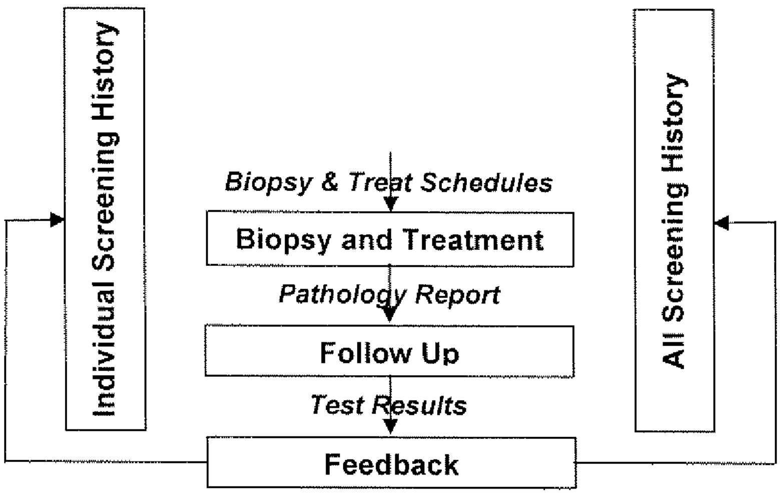


FIG. 63

Feedback for Estimating the Probability of Progressing Cancer

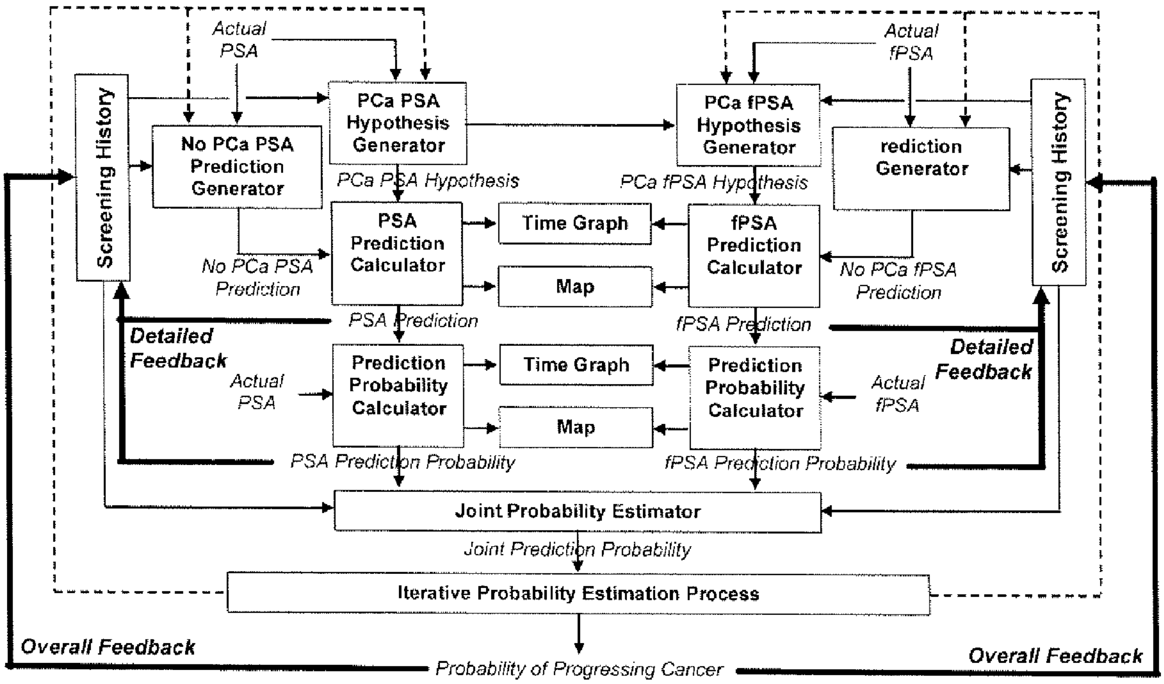


FIG. 64

Detailed Feedback for Predictions

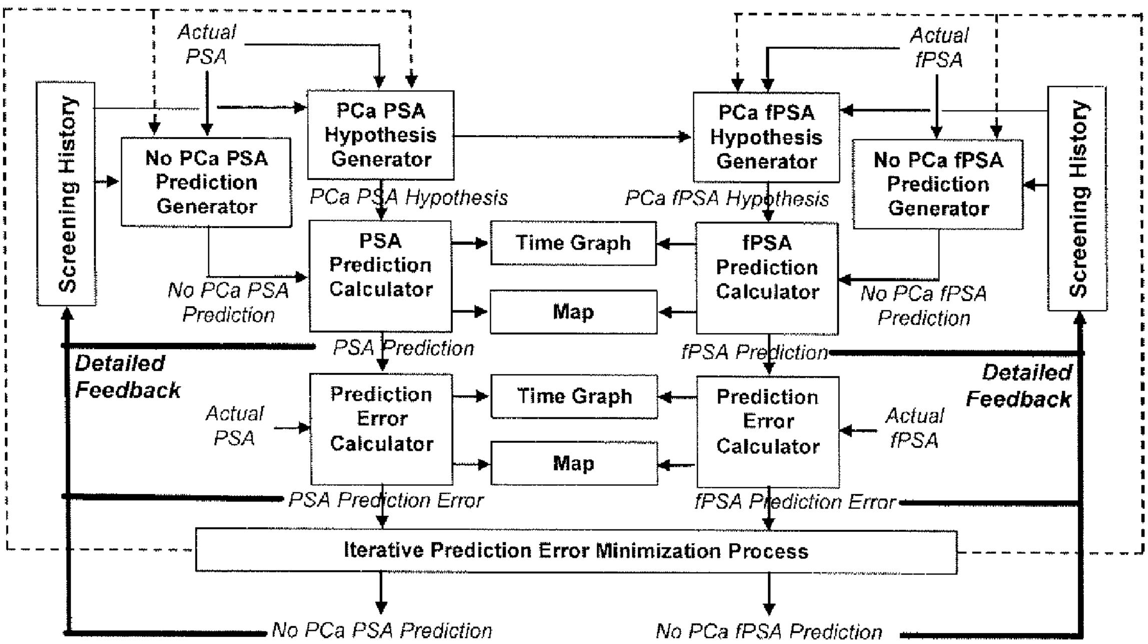


FIG. 65

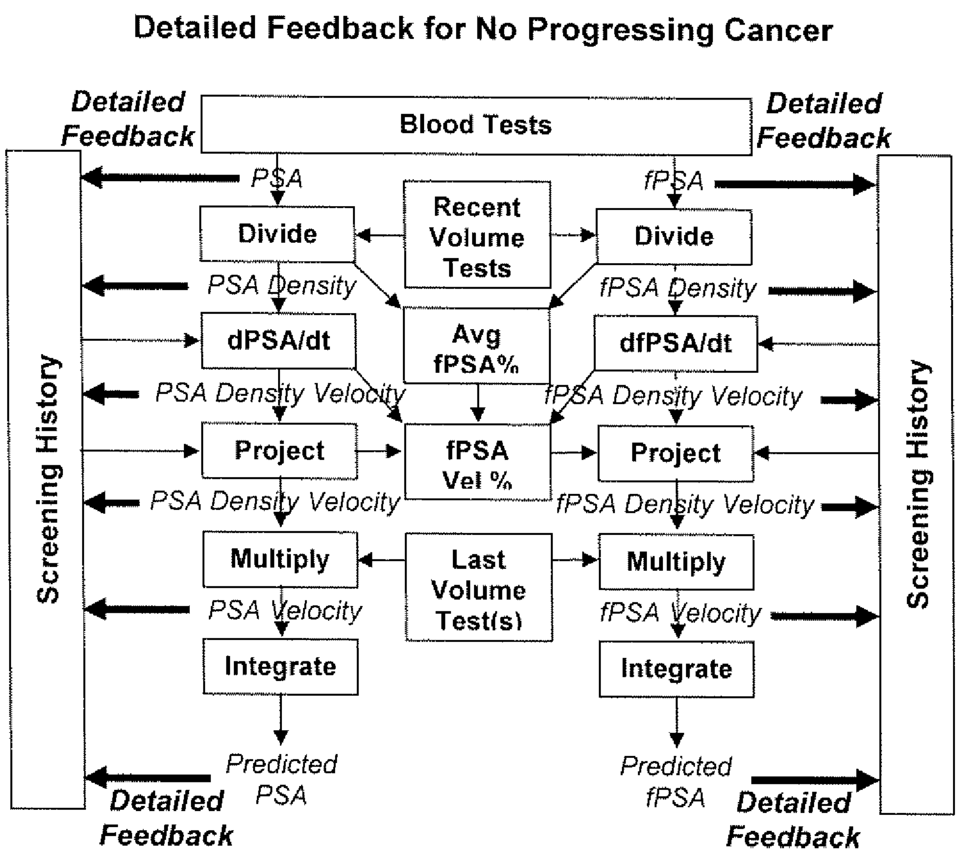
Detailed Feedback for No Progressing Cancer
Detailed Feedback
Blood Tests
Detailed Feedback
PSA
fPSA
Recent Volume Tests
Divide
Divide
PSA Density
fPSA Density
dPSA/dt
Avg fPSA%
dfPSA/dt
Screening History
PSA Density Velocity
fPSA Density Velocity
Screening History
Project
fPSA Vel %
Project
PSA Density Velocity
fPSA Density Velocity
Multiply
Last Volume Test(s)
Multiply
PSA Velocity
fPSA Velocity
Integrate
Integrate
Predicted PSA
Predicted fPSA
Detailed Feedback
Detailed Feedback

FIG. 66

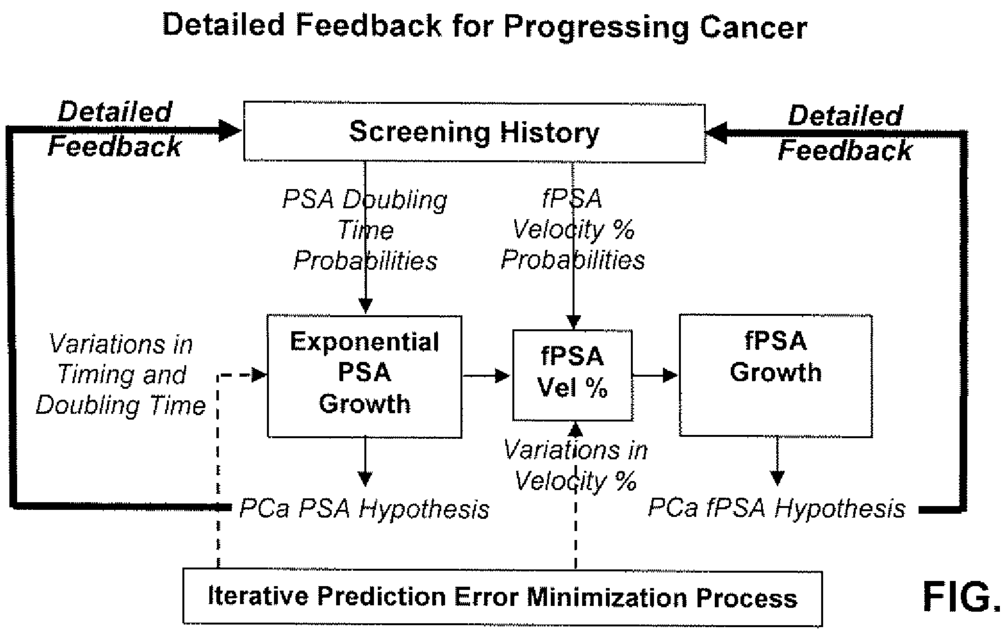
Detailed Feedback for Progressing Cancer
Detailed Feedback
Screening History
Detailed Feedback
PSA Doubling Time Probabilities
fPSA Velocity % Probabilities
Variations in Timing and Doubling Time
Exponential PSA Growth
fPSA Vel %
fPSA Growth
Variations in Velocity %
PCa PSA Hypothesis
PCa fPSA Hypothesis
Iterative Prediction Error Minimization Process

FIG. 67

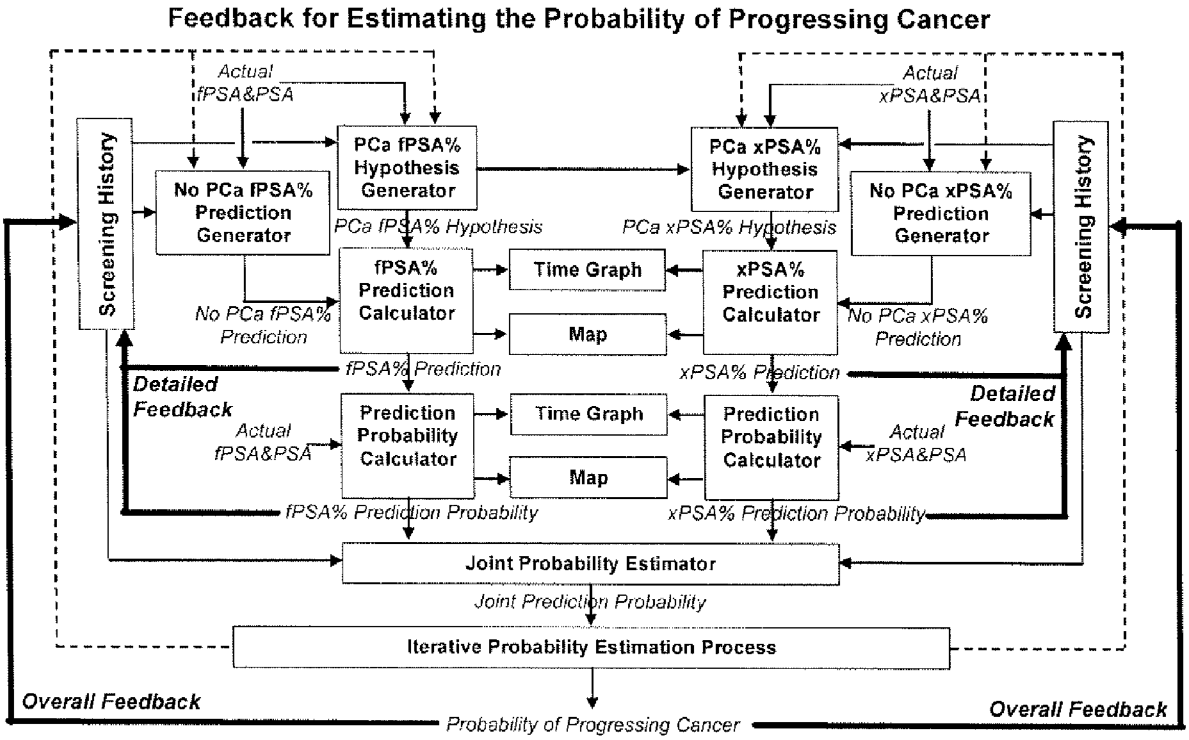
Feedback for Estimating the Probability of Progressing Cancer
Actual fPSA&PSA
Actual xPSA&PSA
Screening History
No PCa fPSA% Prediction Generator
PCa fPSA% Hypothesis Generator
PCa xPSA% Hypothesis Generator
No PCa xPSA% Prediction Generator
Screening History
PCa fPSA% Hypothesis
PCa xPSA% Hypothesis
fPSA% Prediction Calculator
Time Graph
Map
xPSA% Prediction Calculator
No PCa fPSA% Prediction
No PCa xPSA% Prediction
fPSA% Prediction
xPSA% Prediction
Detailed Feedback
Actual fPSA&PSA
Prediction Probability Calculator
Time Graph
Map
Prediction Probability Calculator
Actual xPSA&PSA
Detailed Feedback
fPSA% Prediction Probability
xPSA% Prediction Probability
Joint Probability Estimator
Joint Prediction Probability
Iterative Probability Estimation Process
Overall Feedback
Probability of Progressing Cancer
Overall Feedback

FIG. 68

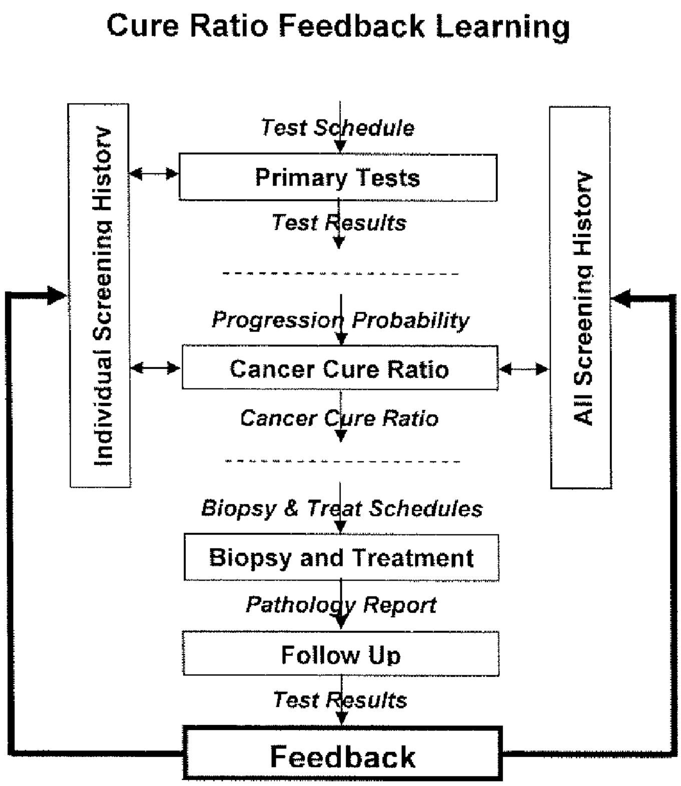
Cure Ratio Feedback Learning
Individual Screening History
Test Schedule
Primary Tests
Test Results
Progression Probability
Cancer Cure Ratio
Cancer Cure Ratio
Biopsy & Treat Schedules
Biopsy and Treatment
Pathology Report
Follow Up
Test Results
Feedback
All Screening History

FIG. 69

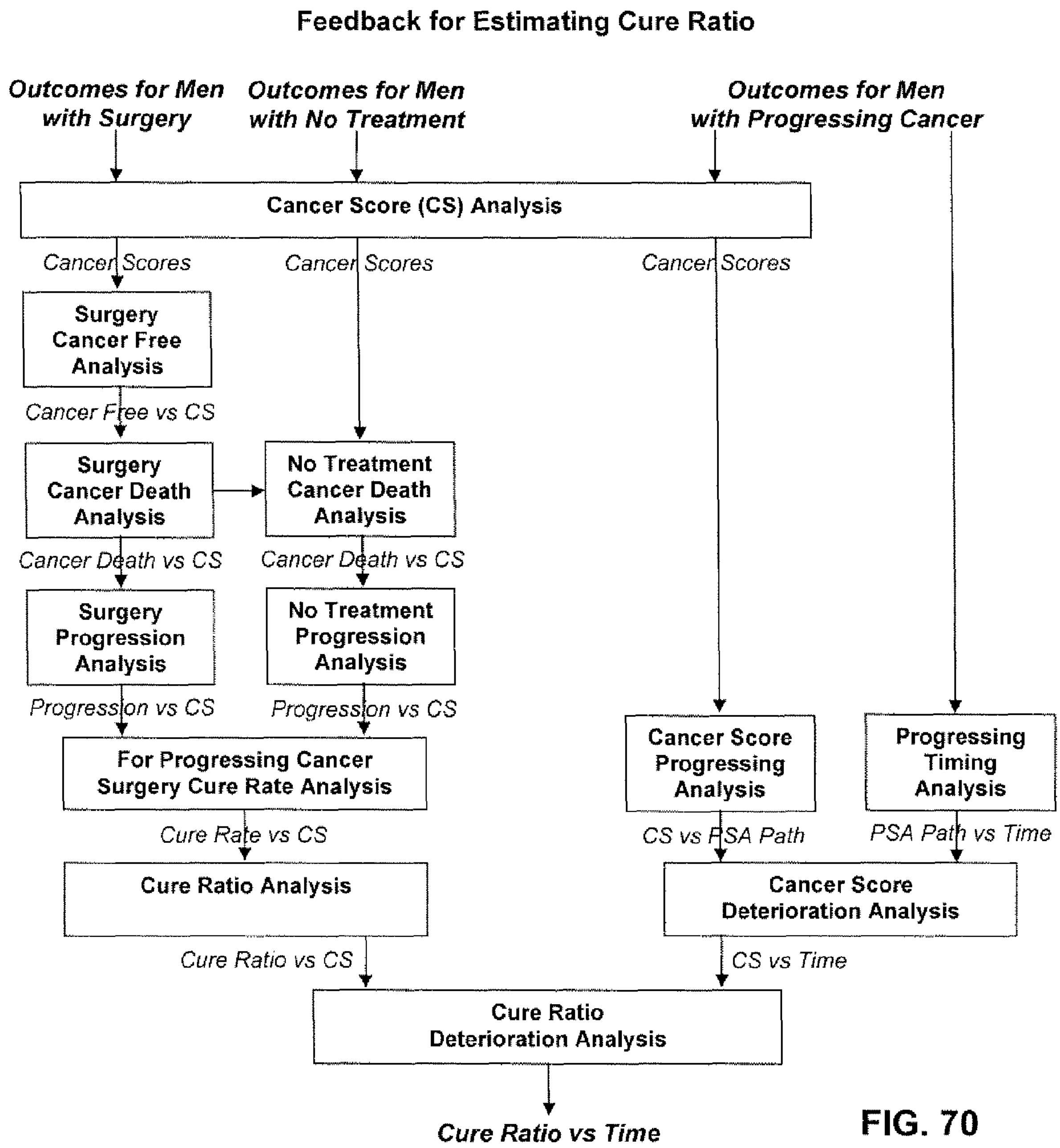
Feedback for Estimating Cure Ratio
Outcomes for Men with Surgery
Outcomes for Men with No Treatment
Outcomes for Men with Progressing Cancer
Cancer Score (CS) Analysis
Cancer Scores
Cancer Scores
Cancer Scores
Surgery Cancer Free Analysis
Cancer Free vs CS
Surgery Cancer Death Analysis
No Treatment Cancer Death Analysis
Cancer Death vs CS
Cancer Death vs CS
Surgery Progression Analysis
No Treatment Progression Analysis
Progression vs CS
Progression vs CS
For Progressing Cancer Surgery Cure Rate Analysis
Cancer Score Progressing Analysis
Progressing Timing Analysis
Cure Rate vs CS
CS vs PSA Path
PSA Path vs Time
Cure Ratio Analysis
Cancer Score Deterioration Analysis
Cure Ratio vs CS
CS vs Time
Cure Ratio Deterioration Analysis
Cure Ratio vs Time

FIG. 70

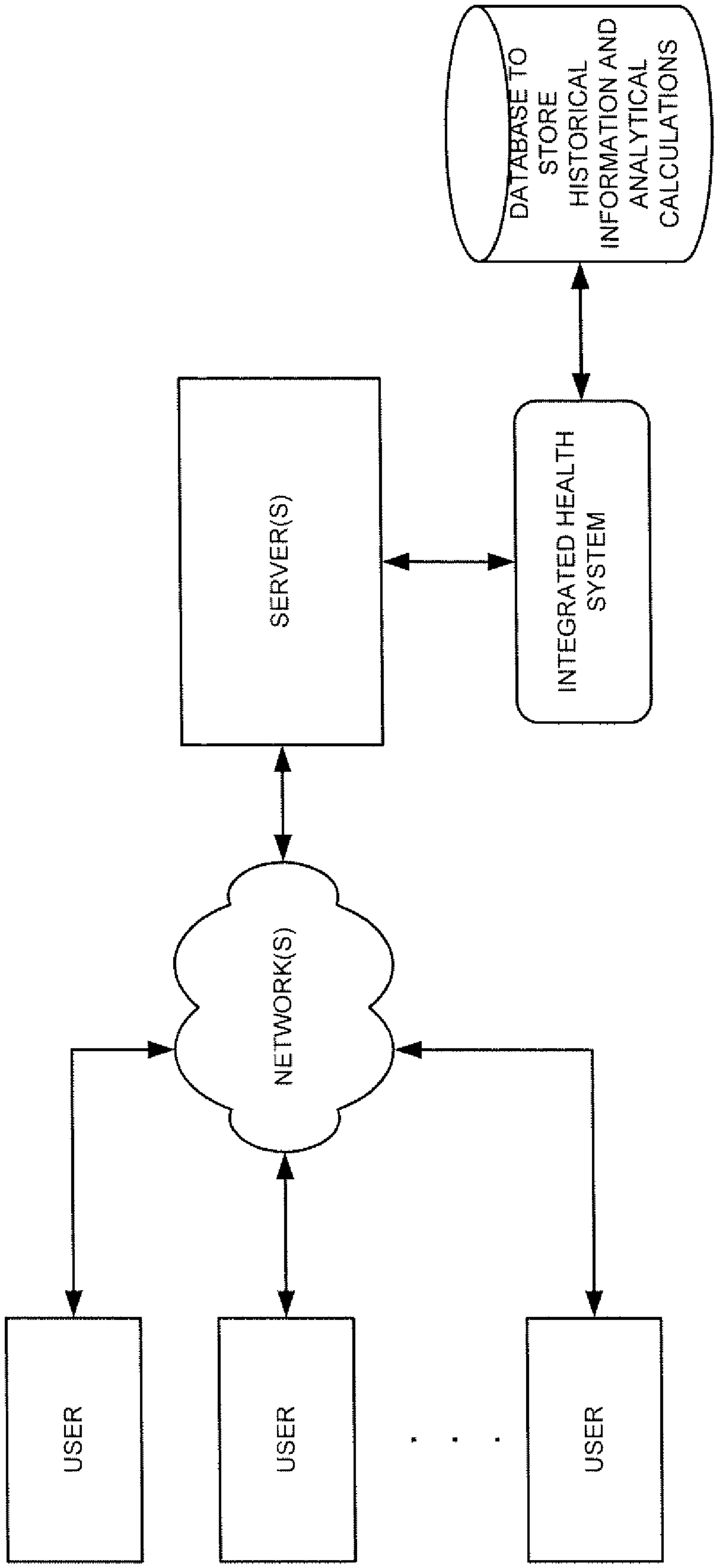
DATABASE TO STORE HISTORICAL INFORMATION AND ANALYTICAL CALCULATIONS
SERVER(S)
INTEGRATED HEALTH SYSTEM
NETWORK(S)
USER
USER
USER

FIG. 71

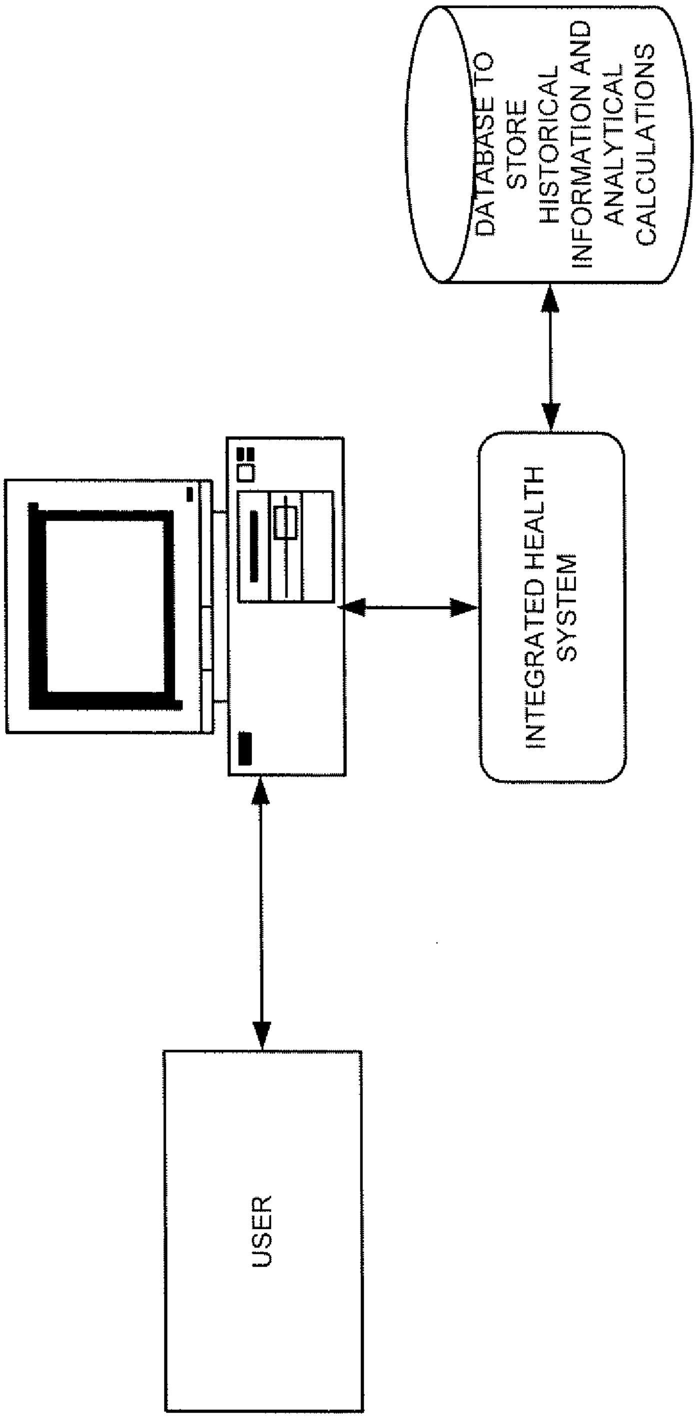
DATABASE TO STORE HISTORICAL INFORMATION AND ANALYTICAL CALCULATIONS
INTEGRATED HEALTH SYSTEM
USER

FIG. 72